# EUROPEAN PATENT SPECIFICATION

(11) **EP 0 847 319 B1**
(45) Date of publication and mention of the grant of the patent: **07.04.2004**
(21) Application number: 96931439.2
(22) Date of filing: 30.08.1996
(51) Int. Cl.: B23K 26/14, A61C 1/00

(54) **USER PROGRAMMABLE COMBINATION OF ATOMIZED PARTICLES FOR ELECTROMAGNETICALLY INDUCED CUTTING**
BENUTZERPROGRAMMIERBARE KOMBINATION VON ZERSTÄUBTEN TEILCHEN FÜR ELEKTROMAGNETISCH INDUZIERTES SCHNEIDEN
COMBINAISON PROGRAMMABLE PAR L'UTILISATEUR DE PARTICULES ATOMISEES POUR DECOUPAGE PAR PROCEDE ELECTROMAGNETIQUE

(30) Priority: 31.08.1995 US 522503; 20.12.1995 US 575775; 14.02.1996 US 599984; 18.06.1996 US 666666
(43) Date of publication of application: 17.06.1998
(73) Proprietor: Biolase Technology, Inc., San Clemente, CA 92673 (US)
(72) Inventor: RIZOIU, Ioana, M., Capistrano Beach, CA 92624 (US); KIMMEL, Andrew, I., San Clemente, CA 92672 (US)
(74) Representative: Baker, Colin John
(86) International application number: PCT/US1996/013960
(87) International publication number: WO 1997/007928

(56) References cited:
- EP-A- 0 181 199
- EP-A- 0 454 312
- DE-A- 4 138 468
- US-A- 3 679 863
- US-A- 3 991 296
- US-A- 4 724 299
- US-A- 5 092 864
- US-A- 5 199 870
- PATENT ABSTRACTS OF JAPAN vol. 8, no. 148 & JP 59 045092 A (FUJA DENPA KOUKI K.K.)

## Description

### Background of the Invention

The present invention relates generally to devices adapted for operating on hard and soft materials and, more particularly, to devices for combining electromagnetic and hydro energies for cutting and removing both hard and soft tissues and to systems for introducing conditioned fluids into cutting, irrigating, evacuating, cleaning, and drilling systems.

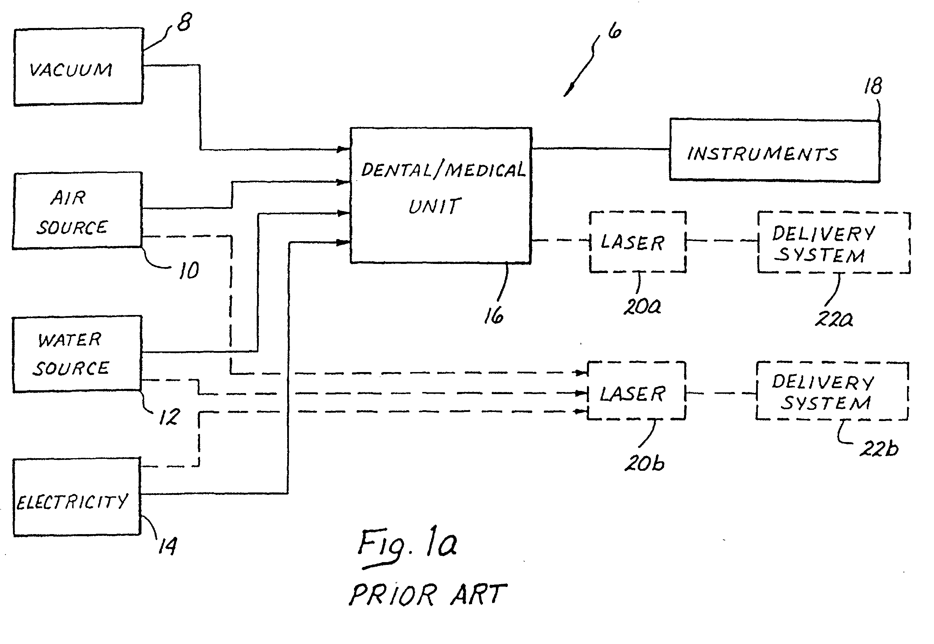

A prior art dental/medical work station 6 is shown in Figure 1a. A vacuum line 8 and an air supply line 10 supply negative and positive pressures, respectively. A water supply line 12 and an electrical outlet 14 supply water and power, respectively. The vacuum line 8, the air supply line 10, the water supply line 12, and the power source 14 are all connected to the dental/medical unit 16.

The dental/medical unit 16 may comprise a dental seat or an operating table, a sink, an overhead light, and other conventional equipment used in dental and medical procedures. The dental/medical unit 16 provides water, air, vacuum and/or power to the instruments 18. These instruments may include an electrocauterizer, an electromagnetic energy source, a mechanical drill, a mechanical saw, a canal finder, a syringe, and/or an evacuator.

The electromagnetic energy source is typically a laser coupled with a delivery system. The laser 20a and delivery system 22a, both shown in phantom, as well as any of the above-mentioned instruments, may be connected directly to the dental/ medical unit 16. Alternatively, the laser 20b and delivery system 22b, both shown in phantom, may be connected directly to the water supply 12, the air supply 10, and the electric outlet 14. Other instruments 18 may be connected directly to any of the vacuum line 8, the air supply line 10, the water supply line 12, and/or the electrical outlet 14.

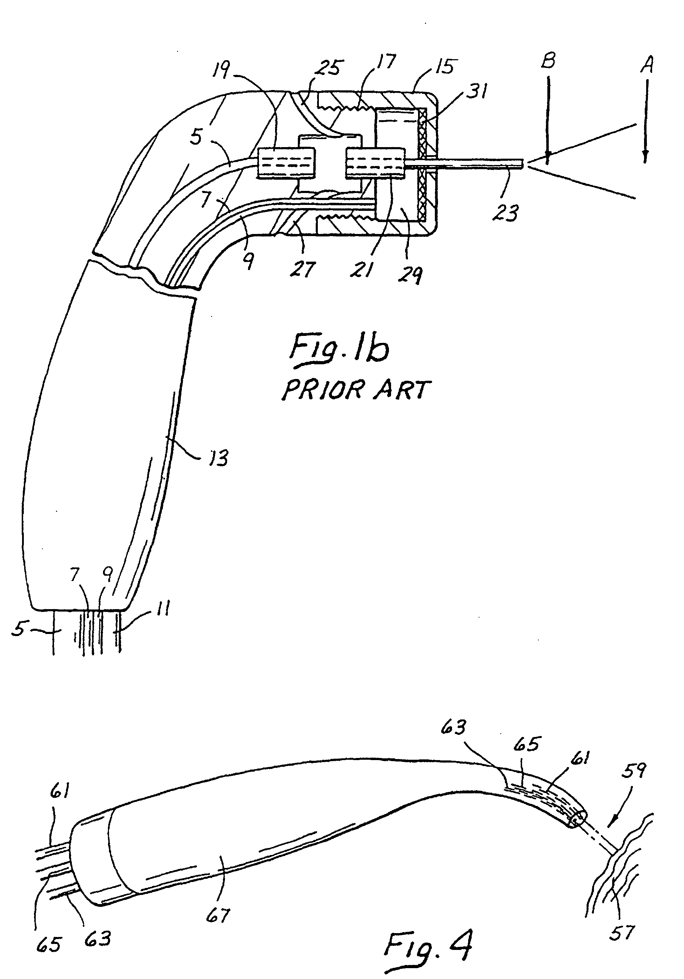

The laser 20 and delivery system 22 may typically comprise an electromagnetic cutter for dental use. A conventional prior art electromagnetic cutter is shown in Figure 1b. According to this prior art apparatus, a fiber guide tube 5, a water line 7, an air line 9, and an air knife line 11 (which supplies pressurized air) are fed into the hand-held apparatus 13. A cap 15 fits onto the hand-held apparatus 13 and is secured via threads 17. The fiber guide tube 5 abuts within a cylindrical metal piece 19. Another cylindrical metal piece 21 is a part of the cap 15.

When the cap 15 is threaded onto the hand-held device 13, the two cylindrical metal tubes 19 and 21 are moved into very close proximity of one another. A gap of air, however, remains between these two cylindrical metal tubes 19 and 21. Thus, the laser within the fiber guide tube 5 must jump this air gap before it can travel and exit through another fiber guide tube 23. Heat is dissipated as the laser jumps this air gap.

The pressurized air from the air knife line 11 surrounds and cools the laser as the laser bridges the gap between the two metal cylindrical objects 19 and 21. Thus, a first problem in this prior art apparatus is that the interface between the two metal cylindrical objects 19 and 21 has a dissipation of heat which must be cooled by pressurized air from the air knife line 11. (Air from the air knife line 11 flows out of the two exhausts 25 and 27 after cooling the interface between elements 19 and 21.) This inefficient interface between elements 19 and 21 results from the removability of the cap 15, since a perfect interface between elements 19 and 21 is not achieved.

The laser energy exits from the fiber guide tube 23 and is applied to a target surface within the patient's mouth, according to a predetermined surgical plan. Water from the water line 7 and pressurized air from the air line 9 are forced into the mixing chamber 29. The air and water mixture is very turbulent in the mixing chamber 29, and exits this chamber through a mesh screen with small holes 31. The air and water mixture travels along the outside of the fiber guide tube 23, and then leaves the tube and contacts the area of surgery. This air and water spray coming from the tip of the fiber guide tube 23 helps to cool the target surface being cut and to remove cut materials by the laser. The need for cooling the patient surgical area being cut is another problem with the prior art.

Water is generally used in a variety of laser cutting operations in order to cool the target surface. Additionally, water is used in mechanical drilling operations for cooling the target surface and removing cut or drilled materials therefrom. Many prior art cutting or drilling systems use a combination of air and water, commonly combined to form a light mist, for cooling a target surface and/or removing cut materials from the target surface.

The use of water in these prior art systems has been somewhat successful for the limited purposes of cooling a target surface or removing debris therefrom. These prior art uses of water in cutting and drilling operations, however, have not allowed for versatility, outside of the two functions of cooling and removing debris. In particular, during cutting or drilling operations, medication treatments, preventative measure applications, and aesthetically pleasing substances, such as flavors or aromas, have not been possible or used. A conventional drilling operation may benefit from the use of an anesthetic near the drilling operation, for example, but during this drilling operation only water and/or air has so far been used. In the case of a laser cutting operation, a disinfectant, such as iodine, could be applied to the target surface during drilling to guard against infection, but this additional disinfectant has not been applied during such laser cutting operations. In the case of an oral drilling or cutting operation, unpleasant tastes or odors may be generated, which may be unpleasing to the patient. The conventional use of only water during this oral procedure does not mask the undesirable taste or odor. A need has thus existed in the prior art for versatility of applications and of treatments during drilling and cutting procedures.

Compressed gases, pressurized air, and electrical motors are commonly used to provide the driving force for mechanical cutting instruments, such as drills, in dentistry and medicine. The compressed gases and pressurized water are subsequently ejected into the atmosphere in close proximity to or inside of the patient's mouth and/or nose. The same holds true for electrically driven turbines when a cooling spray (air and water) is typically ejected into the patient's mouth, as well. These ejected fluids commonly contain vaporous elements of burnt flesh or drilled tissue structure. This odor can be quite uncomfortable for the patient, and can increase trauma experienced by the patient during the drilling or cutting procedure. In a such a drilling or cutting procedure, a mechanism for masking the smell and the odor generated from the cutting or drilling may be advantageous.

Another problem exists in the prior art with bacteria growth on surfaces within a dental operating room. The interior surfaces of air, vacuum, and water lines of the dental unit, for example, are subject to bacteria growth. Additionally, the air and water used to cool the tissue being cut or drilled within the patient's mouth is often vaporized into the air to some degree. This vaporized air and water condensates on surfaces of the dental equipment within the dental operating room. These moist surfaces can also promote bacteria growth, which is undesirable. A system for reducing the bacteria growth within air, vacuum, and water lines, and for reducing the bacteria growth resulting from condensation on exterior surfaces, is needed to reduce sources of contamination within a dental operating room.

In addition to prior art systems which utilize laser light from a fiber guide tube 23, for example, to cut tissue and use water to cool this cut tissue, other prior art systems have been proposed. U.S. Patent No. 5,199,870 to Steiner et al., which issued on April 6, 1993, discloses an optical cutting system which utilizes the expansion of water to destroy and remove tooth material. This prior art approach requires a film of liquid having a thickness of between 10 and 200 mm. Another prior art system is disclosed in U.S. Patent No. 5,267,856 to Wolbarsht et al., which issued on December 7, 1993. This cutting apparatus is similar to the Steiner et al. patent, since it relies on the absorption of laser radiation into water to thereby achieve cutting.

Similarly to the Steiner et al. patent, the Wolbarsht et al. patent requires water to be deposited onto the tooth before laser light is irradiated thereon. Specifically, the Wolbarsht et al. patent requires water to be inserted into pores of the material to be cut. Since many materials, such as tooth enamel, are not very porous, and since a high level of difficulty is associated with inserting water into the "pores" of many materials, this cutting method is somewhat less than optimal. Even the Steiner et al. patent has met with limited success, since the precision and accuracy of the cut is highly dependent upon the precision and accuracy of the water film on the material to be cut. In many cases, a controllable water film cannot be consistently maintained on the surface to be cut. For example, when the targeted tissue to be cut resides on the upper pallet, a controllable water film cannot be maintained.

The above-mentioned prior art systems have all sought in vain to obtain "cleanness" of cutting. In several dental applications, for example, a need to excise small amounts of soft tissues and/or hard tissues with a great degree of precision has existed. These soft tissues may include gingiva, frenum, and lesions and, additionally, the hard tissues may include dentin, enamel, bone, and cartilage. The term "cleanness" of cutting refers to extremely fine, smooth incisions which provide ideal bonding surfaces for various biomaterials. Such biomaterials include cements, glass ionomers and other composites used in dentistry or other sciences to fill holes in structures such as teeth or bone where tooth decay or some other defect has been removed. Even when an extremely fine incision has been achieved, the incision is often covered with a rough surface instead of the desired smooth surface required for ideal bonding.

One specific dental application, for example, which requires smooth and accurate cutting through both hard and soft tissues is implantology. According to the dental specialty of implantology, a dental implant can be installed in a person's mouth when that person has lost his or her teeth. The conventional implant installation technique is to cut through the soft tissue above the bone where the tooth is missing, and then to drill a hole into the bone. The hole in the bone is then threaded with a low-speed motorized tap, and a titanium implant is then screwed into the person's jaw. A synthetic tooth, for example, can be easily attached to the portion of the implant residing above the gum surface. One problem associated with the conventional technique occurs when the clinician drills into the patient's jaw to prepare the site for the implant. This drilling procedure generates a great deal of heat, corresponding to friction from the drilling instrument. If the bone is heated too much, it will die. Additionally, since the drilling instrument is not very precise, severe trauma tot the jaw occurs after the drilling operation. The drilling operation creates large mechanical internal stress on the bone structure.

JP-A-59045092, on which the preamble of claim 1 is based, discloses a laser working device intended for working a surface having high reflectivity and heat conductivity. Water is supplied to the surface of the work-piece and energy from a carbon dioxide laser is applied which is absorbed to some degree by the water on the surface. Some of the water explodes providing the force to work the surface.

### Summary of the Invention

The present invention provides an apparatus comprising:
a fluid output comprising a fluid during use and being constructed to direct the fluid to a first vicinity relative to the fluid output; and
an electromagnetic energy source for supplying electromagnetic energy to a second vicinity;
the first vicinity and the second vicinity being disposed at respective locations relative to the fluid output;
characterised in that said first vicinity and said second vicinity intersect in a volume relative to the fluid output, said fluid output further comprises an atomizer for generating a combination of atomized fluid particles, and for placing the combination of atomized fluid particles into said volume; said electromagnetic energy source comprising a specifically configured electromagnetic energy source that is arranged, when said apparatus is in use, to supply electromagnetic energy of a wavelength which is substantially absorbed by said combination of atomized fluid particles and to direct a peak concentration of said electromagnetic energy into said volume; said combination of atomized fluid particles being sized and distributed in such a way that, when placed into said volume and irradiated with said focused electromagnetic energy, said electromagnetic energy is substantially absorbed by at least a portion of said combination of atomized fluid particles wherein disruptive mechanical forces are imparted to a target surface.

The present invention also provides a non-therapeutic method comprising:
supplying fluid to a first vicinity of the target surface; and
supplying electromagnetic energy to a second vicinity of the target surface;
the first vicinity and the second vicinity intersecting in a volume adjacent to the target surface;
said step of supplying fluid comprising generating a combination of atomized fluid particles and placing said combination of atomized fluid particles into said volume adjacent to the target surface;
said step of supplying electromagnetic energy comprising directing a peak concentration of electromagnetic energy, which has a wavelength that is substantially absorbed by said fluid, into said volume so as to be absorbed by at least a portion of said fluid particles to cause the portion of atomized fluid particles to impart disruptive mechanical forces to said target surface.

In context of this invention the phrase "above the target surface" is not limiting to any particular target orientation, but refer to a location spaced away from the surface.

The invention may be incorporated into various different contents which are outlined below as various aspects of the invention.

The present invention therefore discloses an electromagnetically induced cutting mechanism, which can provide accurate cutting operations on hard and soft tissues, and other materials, as well. The electromagnetically induced cutter is capable of providing extremely fine and smooth incisions, irrespective of the cutting surface. Additionally, a user programmable combination of atomized particles allows for user control of various cutting parameters. The various cutting parameters may also be controlled by changing spray nozzles and electromagnetic energy source parameters. Applications for the present invention include medical, dental, industrial (etching, engraving, cutting and cleaning) and any other environments where an objective is to precisely remove surface materials without inducing thermal damage, uncontrolled cutting parameters, and/or rough surfaces inappropriate for ideal bonding. The present invention further does not require any films of water or any particularly porous surfaces to obtain very accurate and controllable cutting.

Drills, saws and osteotomes are standard mechanical instruments used in a variety of dental and medical applications. The limitations associated with these instruments include: temperature induced necrosis (bone death), aerosolized solid-particle release, limited access, lack of precision in cutting depth and large mechanical stress created on the tissue structure. The electromagnetically induced mechanical cutter according to one embodiment of the present invention is uniquely suited for these dental and medical applications, such as, for example, implantology. In an implantology procedure the electromagnetically induced mechanical cutter is capable of accurately and efficiently cutting through both oral soft tissues overlaying the bone and also through portions of the jawbone itself. The electromagnetically induced mechanical cutter according to one embodiment of the present invention does not induce thermal damage and does not create high internal structural stress on the patient's jaw, for example. After the patient's jaw is prepared with the electromagnetically induced mechanical cutter, traditional methods can be employed for threading the hole in the patient's jaw and inserting the dental implant. Similar techniques can be used for preparing hard tissue structures for insertion of other types of medical implants, such as pins, screws, wires, etc.

The electromagnetically induced mechanical cutter according to one embodiment of the present invention includes an electromagnetic energy source, which focuses electromagnetic energy into a volume of air adjacent to a target surface. The target surface may be a tooth, for example. A user input device specifies whether either a high resolution or a low resolution cut is needed, and further specifies whether a deep penetration cut or a shallow penetration cut is needed. An atomizer generates a combination of atomized fluid particles, according to information from the user input device. The atomizer places the combination of atomized fluid particles into the volume of air adjacent to the target surface. The electromagnetic energy, which is focused into the volume of air adjacent to the target surface, is selected to have a wavelength.suitable for the fluid particles. In particular, the wavelength of the electromagnetic energy should be substantially absorbed by the atomized fluid particles in the volume of air adjacent to the target surface to thereby explode the atomized fluid particles. Explosion of the atomized fluid particles imparts mechanical cutting forces onto the target surface.

The user input device according to one embodiment may incorporate only a single dial for controlling the cutting efficiency, or may include a number of dials for controlling the fluid particle size, fluid particle velocity, spray cone angle, average laser power, laser repetition rate, fiberoptic diameter, etc. According to one feature of the present invention, the atomizer generates relatively small fluid particles when the user input specifies a high resolution cut, and generates relatively large fluid particles when the user input specifies a low resolution cut. The atomizer generates a relatively low density distribution of fluid particles when the user input specifies a deep penetration cut, and generates a relatively high density distribution of fluid particles when the user input specifies a shallow penetration cut. A relatively small fluid particle may have a diameter less than the wavelength of the electromagnetic energy and, similarly, a relatively large fluid particle may have a diameter which is greater than the wavelength of the electromagnetic energy.

The electromagnetic energy source preferably is an erbium, chromium, yttrium, scandium, gallium garnet (Er, Cr:YSGG) solid state laser, which generates electromagnetic energy having a wavelength in a range of 2.70 to 2.80 microns. According to other embodiments of the present invention, the electromagnetic energy source may be an erbium, yttrium, scandium, gallium garnet (Er:YSGG) solid state laser, which generates electromagnetic energy having a wavelength in a range of 2.70 to 2.80 microns; an erbium, yttrium, aluminum garnet (Er:YAG) solid state laser, which generates electromagnetic energy having a wavelength of 2.94 microns; chromium, thulium, erbium, yttrium, aluminum garnet (CTE:YAG) solid state laser, which generates electromagnetic energy having a wavelength of 2.69 microns; erbium, yttrium orthoaluminate (Er:YALO3) solid state laser, which generates electromagnetic energy having a wavelength in a range of 2.71 to 2.86 microns; holmium, yttrium, aluminum garnet (Ho:YAG) solid state laser, which generates electromagnetic energy having a wavelength of 2.10 microns; quadrupled neodymium, yttrium, aluminum garnet (quadrupled Nd:YAG) solid state laser, which generates electromagnetic energy having a wavelength of 266 nanometers; argon fluoride (ArF) excimer laser, which generates electromagnetic energy having a wavelength of 193 nanometers; xenon chloride (XeCl) excimer laser, which generates electromagnetic energy having a wavelength of 308 nanometers; krypton fluoride (KrF) excimer laser, which generates electromagnetic energy having a wavelength of 248 nanometers; and carbon dioxide (CO2), which generates electromagnetic energy having a wavelength in a range of 9.0 to 10.6 microns.

When the electromagnetic energy source is configured according to the preferred embodiment, the repetition rate is greater than 1 Hz, the pulse duration range is between 1 picosecond and 1000 microseconds, and the energy is greater than 1 milliJoule per pulse. According to one preferred operating mode of the present invention, the electromagnetic energy source has a wavelength of approximately 2.78 microns, a repetition rate of 20 Hz, a pulse duration of 140 microseconds, and an energy between 1 and 300 milliJoules per pulse. The atomized fluid particles provide the mechanical cutting forces when they absorb the electromagnetic energy within the interaction zone. These atomized fluid particles, however, provide a second function of cleaning and cooling the fiberoptic guide from which the electromagnetic energy is output.

The optical cutter according to one embodiment of the present invention combats the problem of poor coupling between the two laser fiberoptics of Figure 1b. The optical cutter according to one embodiment of the present invention provides a focusing optic for efficiently directing the energy from the first fiberoptic guide to the second fiberoptic guide, to thereby reduce dissipation of laser energy between the first fiberoptic guide and the second fiberoptic guide. This optical cutter includes a housing having a lower portion, an upper portion, and an interfacing portion. The first fiberoptic tube is surrounded at its upper portion by a first abutting member, and the second fiberoptic tube is surrounded at its proximal end by a second abutting member. A cap is placed over the second fiberoptic tube and the second abutting member. Either fiberoptic tube may be formed of calcium fluoride (CaF), calcium oxide (CaO2), zirconium oxide (ZrO2), zirconium fluoride (ZrF), sapphire, hollow waveguide, liquid core, TeX glass, quartz silica, germanium sulfide, arsenic sulfide, and germanium oxide (GeO2).

The electromagnetically induced mechanical cutter of the present invention efficiently and accurately cuts both hard and soft tissue. This hard tissue may include tooth enamel, tooth dentin, tooth cementum, bone, and cartilage, and the soft tissues may include skin, mucosa, gingiva, muscle, heart, liver, kidney, brain, eye, and vessels.

According to one aspect of the present invention, a laser delivery system provides several mechanisms for combating heat generation. Heat resistant ferrules, concentric crystalline fibers, and air paths directed around these fibers are provided. The present invention further provides added strength to the laser fiber guide, by enclosing the laser fiber guide in concentric tubular members. Additionally, the handpiece of the laser delivery system according to a further aspect of the present invention is configured into an outer sleeve component and an inner shaft component for enhanced operability and sterility. Bent crystalline fibers may be used in conjunction with the laser delivery system of the present invention for delivering energy to a target surface. Additionally, an illumination line adapted for emitting coherent and non-coherent light might be provided within the housing for illuminating a target surface, and a medication line for delivering medications to a target surface is also provided within the housing.

According to a further aspect of the present invention, a medical handpiece includes a housing having a proximal end and a radiation delivery end. A first fiber guide for conducting laser radiation from an external source toward the radiation delivery end of the housing is disposed within the housing, and a first ferrule formed of a ceramic or crystalline material (such as sapphire) is secured to an output end of the first fiber guide. A second fiber guide has a receiving end, which is adapted for receiving laser radiation from the output end of the first fiber guide, and a second ferrule secures the second fiber guide to the housing so that the receiving end of the second fiber guide faces the output end of the first fiber guide. The second ferrule is formed of a ceramic or crystalline material (such as sapphire). The first ferrule and the second ferrule together form a gas flow path in a direction from the first fiber guide toward the receiving end of the second fiber guide.

According to anther aspect of the present invention, an electromagnetic energy conducting apparatus includes a housing and an inner electromagnetic energy guide disposed within the housing. The inner electromagnetic energy guide has a proximal end, a distal end, and an axis extending between the proximal end and the distal end. The electromagnetic energy conducting apparatus further includes an outer electromagnetic energy guide, which surrounds and contacts the inner electromagnetic energy guide. The outer electromagnetic energy guide has an axis that is substantially co-linear with the axis of the inner electromagnetic energy guide. Both the outer electromagnetic energy guide and the inner electromagnetic energy guide may be disposed within a ferrule, or the outer electromagnetic energy guide may be a ferrule. Both electromagnetic energy guides preferably are made of a crystalline material, such as sapphire. The electromagnetic energy conducting apparatus may further include a source electromagnetic energy guide, which has a diameter that is substantially larger than a diameter of the inner electromagnetic energy guide.

According to another aspect of the present invention, the apparatus comprises an optical energy conducting apparatus which includes a housing and a crystalline fiber disposed within the housing. The crystalline fiber is adapted for conducting optical energy therethrough A gas flow path is disposed within the housing, as well. The gas flow path envelops the crystalline fiber and extends from a proximal end of the crystalline fiber to a distal end of the crystalline fiber.

The crystalline fiber may be bent according to another aspect of the present invention. In accordance with this aspect of the invention there is provided an optical energy conducting apparatus, comprising a bent crystalline fiber adapted for conducting optical energy therethrough, the bent crystalline fiber having a proximal end, a distal end, and an axis extending between the proximal end and the distal end, a first portion of the axis near the proximal end of the bent crystalline fiber not being parallel with a second portion of the axis near the distal end of the bent crystalline fiber. The angle formed by the bend of the crystalline fiber is preferably 90 degrees. The bent crystalline fiber of the present invention is suited for conducting coherent light. The coherent light preferably has a wavelength on an order of approximately 2.5 to 3 microns, and preferably the wavelength is in the range of 2.78 to 2.94 microns.

According to another aspect of the present invention, the apparatus includes a medical handpiece which comprises a housing and a source of electromagnetic energy disposed within the housing and adapted for emitting electromagnetic energy from a distal end of the housing. An illumination source is disposed within the housing for projecting light from the distal end of the housing onto a target surface. The illumination source may include a fiberoptic bundle. According to a different aspect of the invention, a medication line be disposed within the housing for outputting medication through a distal end of the housing onto a target surface.

According to another aspect of the present invention, the apparatus comprises a medical handpiece delivery system which includes a housing, a fiber guide, and a ferrule disposed around a distal end of the fiber guide. A proximal end and an intermediate portion of the fiber guide are surrounded by an inner protective tube. An outer protective tube is disposed around the inner protective tube. The outer protective tube is slidably disposed over the inner protective tube. The fiber guide may also include a tubular jacket, which is disposed on an outer surface of the fiber guide and within the inner protective tube. Other protective tubes may be disposed around the outer protective tube.

The ferrule includes a proximal end and a distal end, the inner protective tube includes a proximal end and a distal end, and the outer protective tube includes a proximal end and a distal end. The proximal end of the inner protective tube and the proximal end of the outer protective tube are disposed near an SMA connector, which surrounds a proximal end of the fiber guide. The SMA connector has a proximal end and a distal end, and the fiber guide spans a distance between the proximal end of the ferrule and the distal end of the SMA connector. The lengths of the inner protective tube and the outer protective tube between the proximal end of the ferrule and the distal end of the SMA connector, however, are less than the length of the fiber guide between these two items, e.g. at least one quarter of an inch less. Both the inner protective tube and the outer protective tube preferably include a flexible plastic material. These two tubes may be surrounded by a metal tube near the SMA connector and, additionally, the metal tube may be surrounded by another plastic tube near the SMA connector.

According to another aspect of the present invention, the apparatus comprises a medical handpiece which includes a sleeve housing and a shaft assembly adapted for being removably disposed within the sleeve housing. The sleeve housing is preferably autoclavable, is adapted to be held by a hand of a user, and includes a proximal sleeve housing end, an intermediate sleeve housing portion, a radiation delivery end, and an elongate aperture extending within the sleeve housing from the proximal sleeve housing end to the intermediate sleeve housing portion. The sleeve housing includes a delivery fiber guide, which extends between the intermediate sleeve housing portion and the radiation delivery end. The shaft assembly fits within the elongate aperture, and includes a source fiber guide, which is adapted for supplying electromagnetic energy to the intermediate sleeve housing portion. A collar fits around the proximal sleeve housing end when the shaft assembly is disposed with the sleeve housing. The collar applies a radially inwardly directed pressure onto the proximal sleeve housing end, to thereby frictionally hold the shaft assembly within the elongate aperture of the sleeve housing. The distal end of the source fiber guide is surrounded by a first ferrule, the proximal end of the delivery fiber guide is surrounded by a second ferrule, and a third ferrule surrounds a portion of the delivery fiber guide near the radiation delivery end of the sleeve housing. The shaft assembly includes an air supply line, which is disposed around the source fiber guide, and the sleeve housing includes an air line, a water line, an illumination line, and a medication line.

The fluid conditioning system of the present invention is adaptable to most existing medical and dental cutting, irrigating, evacuating, cleaning, and drilling apparatuses. Flavored fluid is used in place of regular tap water during drilling operations. In the case of a laser surgical operation, electromagnetic energy is focused in a direction of the tissue to be cut, and a fluid router routes flavored fluid in the same direction. The flavored fluid may appeal to the taste buds of the patient undergoing the surgical procedure, and may include any of a variety of flavors, such as a fruit flavor or a mint flavor. In the case of a mist or air spray, scented air may be used to mask the smell of burnt or drilled tissue. The scent may function as an air freshener, even for operations outside of dental applications.

In accordance with the foregoing, the invention further includes an apparatus for implementing a medical procedure comprising a medical instrument for performing a medical-treatment function on an operating site located inside of or connected to a human body, and a router for routing one of a scented medium, an ionized solution, a medication medium, or a pigmented fluid in the direction of the operating site.

The fluids used for cooling a surgical site and/or removing tissue may further include an ionized solution, such as a biocompatible saline solution, and may further include fluids having predetermined densities, specific gravities, pH levels, viscosities, or temperatures, relative to conventional tap water. Additionally, the fluids may include a medication, such as an antibiotic, a steroid, an anesthetic, an anti-inflammatory, an antiseptic or disinfectant, adrenaline, epinephrine, or an astringent. The fluid may also include vitamins, herbs, or minerals.

Introduction of any of the above-mentioned conditioning agents to the conventional water of a cutting or drilling operation may be controlled by a user input. Thus, for example, a user may adjust a knob or apply pressure to a foot pedal in order to introduce iodine into the water after a cutting operation has been performed. The amount of conditioning applied to the air, water, or mist may be a function of the position of the foot pedal, for example.

With the method of the present invention, a mist of atomized particles is placed into a volume of air above the tissue to be cut, and a source of electromagnetic energy, such as a laser, is focused into the volume of air. The electromagnetic energy has a wavelength, which is substantially absorbed by the atomized particles in the volume air. This absorption of the electromagnetic energy by the atomized particles causes the atomized particles to explode and impart mechanical cutting forces onto the tissue. According to this feature, the electromagnetic energy source does not directly cut the tissue but, rather, the exploded fluid particles are used to cut the tissue. These fluid particles may be conditioned with flavors, scents, ionization, medications, disinfectants, and other agents, as previously mentioned.

Since the electromagnetic energy is focused directly on the atomized conditioned fluid particles, the cutting forces are changed, depending upon the conditioning of the atomized fluid particles. The mechanical cutting efficiency is proportional (related) to the absorption of the electromagnetic energy by the fluid spray. The absorption characteristic can be modified by changing the fluid composition. For example, introduction of a salt into the water before atomization, resulting in an ionized solution, will exhibit slower cutting properties than does regular water. This slower cutting may be desirable, or the laser power may be increased to compensate for the ionized, atomized fluid particles. Additionally, the atomized fluid particles may be pigmented to either enhance or retard absorption of the electromagnetic energy, to thereby additionally control the cutting power of the system. Two sources of fluid may be used, with one of the sources having a pigment and the other not having a pigment.

Another feature of the present invention places a disinfectant in the air, mist, or water used for dental applications. This disinfectant can be periodically routed through the air, mist, or water lines to disinfect the interior surfaces of these lines. This routing of disinfectant can be performed between patients, daily, or at any other predetermined intervals. A mouthwash may be used, for example, at the end of each procedure to both clean the patient's mouth and clean the air and water tubes.

According to another feature of the present invention, when disinfectant is routed through the lines during a medical procedure, the disinfectant stays with the. water or mist, as the water or mist becomes airborne and settles on surrounding surfaces within the dental operating room. Bacteria growth within the lines, and from the condensation, is significantly attenuated, since the disinfectant retards bacteria growth on the moist surfaces.

The present invention can also include an apparatus for implementing a medical procedure, comprising a medical instrument for performing a medical-treatment function on an operating site located inside of or connected to a human body, and a fluid router for routing a fluid, which has a measurable fluid parameter different than a corresponding measurable fluid parameter of water, in the direction of the operating site. The measurable fluid parameter may comprise one of a density, a specific gravity, a ph level, a viscosity, and a temperature.

In accordance with a further embodiment, the present invention comprises an apparatus for disinfecting both a patient's mouth and a dental instrument within a vicinity of the patient's mouth, comprising a surgical tool for cutting or drilling material (e.g., tissue) within a patient's mouth, and a fluid router for routing disinfectant into the patient's mouth, a portion of the routed disinfectant becoming airborne and settling onto the dental instrument to thereby disinfect the dental instrument. The invention can also be used for attenuating bacteria growth caused by accumulation of condensation on and within a dental instrument, comprising the following steps:
placing a surgical tool for cutting or drilling material within a patient's mouth; and
routing a disinfectant fluid into the patient's mouth, a portion of the routed disinfectant fluid becoming airborne and settling onto the dental instrument to thereby attenuate bacteria growth on the dental instrument.

The dental instrument may comprise at least one of a dental chair and a dental unit.

The apparatus of the present invention can also include a tubing disinfecting unit for disinfecting an interior surface of at least one of an air tube, a vacuum tube, and a water tube, the unit comprising a source of disinfectant, and a router for routing the disinfectant through the at least one of the air tube, the vacuum tube, and the water tube.

The invention can also be used disinfecting an interior surface of at least one of an air tube, a vacuum tube, and a water tube, the method comprising in this care the following further steps:
connecting a source of disinfectant to the at least one of the air tube, the vacuum tube, and the water tube; and
routing the disinfectant through the at least one of the air tube, the vacuum tube, and the water tube to thereby disinfect an interior surface of the at least one of the air tube, the vacuum tube, and the water tube.

The invention, together with additional features and advantages thereof may best be understood by reference to the following description taken in connection with the accompanying illustrative drawings.

### Brief Description of the Drawings

Figure 1a illustrates a conventional dental/medical work station;
Figure 1b is a conventional optical cutter apparatus;
Figure 2 is an optical cutter with the focusing optic and forming part of an embodiment of the present invention;
Figure 3 is a schematic block diagram illustrating the electromagnetically induced mechanical cutter of the present invention;
Figure 4 illustrates one embodiment of the electromagnetically induced mechanical cutter of the present invention;
Figure 5 illustrates another embodiment of the electromagnetically induced mechanical cutter of the present invention;
Figure 6 illustrates a control panel for programming the combination of atomized fluid particles according to the presently preferred embodiment;
Figure 7 is a plot of particle size versus fluid pressure;
Figure 8 is a plot of particle velocity versus fluid pressure;
Figure 9 is a schematic diagram illustrating a fluid particle, a source of electromagnetic energy, and a target surface;
Figure 10 is a schematic diagram illustrating the "grenade" effect of the present invention;
Figure 11 is a schematic diagram illustrating the "explosive ejection" effect of the present invention;
Figure 12 is a schematic diagram illustrating the "explosive propulsion" effect of the present invention;
Figure 13 is a schematic diagram illustrating a combination of Figures 10-12;
Figure 14 is a schematic diagram illustrating the "cleanness" of cut obtained by the present invention;
Figure 15 is a schematic diagram illustrating the roughness of cut obtained by prior art systems;
Figure 16 is a cross-sectional view of the laser delivery system of a presently preferred embodiment;
Figures 17 and 17b illustrate a partially disassembled state of the laser delivery system of a presently preferred embodiment;
Figure 18 illustrates a bent crystalline fiber;
Figure 19 illustrates a portion of the laser delivery system adapted for being connected to a laser source, according to an embodiment of the invention;
Figure 20 illustrates a dental/medical work station according to one embodiment of the present invention;
Figure 21 is a schematic block diagram illustrating an electromagnetic cutter using conditioned fluid, according to one embodiment of the present invention;
Figure 22a illustrates a mechanical drilling apparatus according to the present invention;
Figure 22b illustrates a syringe according to the present invention;
Figure 23 illustrates one embodiment of the fluid conditioning system;
Figure 24 illustrates one embodiment of the fluid conditioning unit of the present invention; and
Figure 25 illustrates one embodiment of the air conditioning unit.

### Description of the Present Embodiments

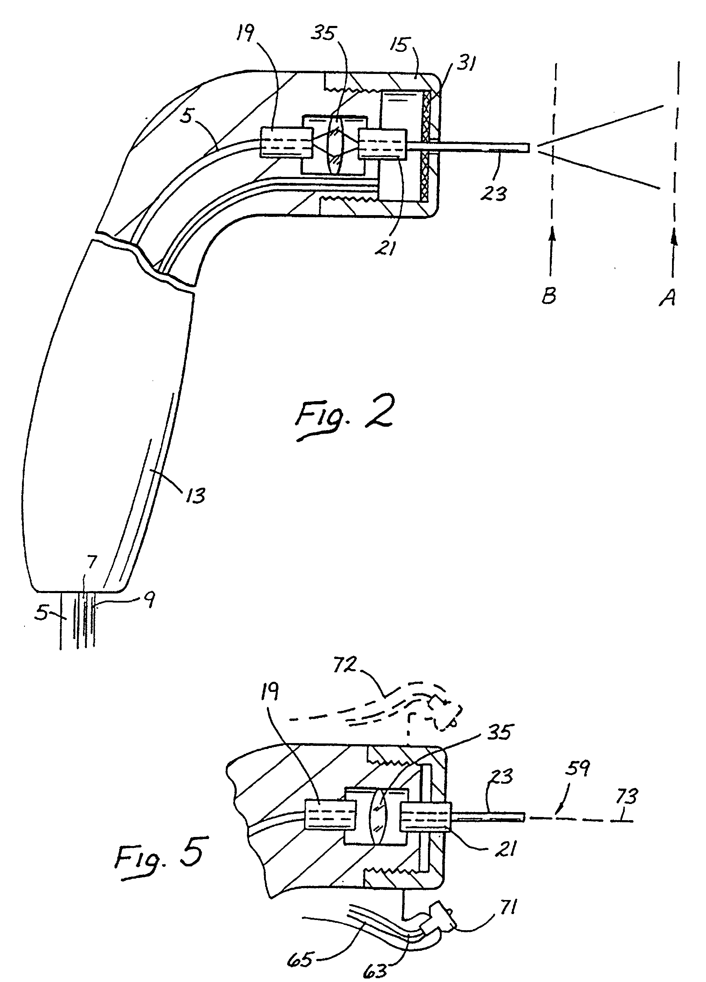

Figure 2 shows an optical cutter according to the present invention. The optical cutter 13 comprises many of the conventional elements shown in Figure 1b. A focusing optic 35 is placed between the two metal cylindrical objects 19 and 21. The focusing optic 35 prevents undesired dissipation of laser energy from the fiber guide tube 5. Specifically, energy from the fiber guide tube 5 dissipates slightly before being focused by the focusing optic 35. The focusing optic 35 focuses energy from the fiber guide tube 5 into the fiber guide tube 23. The efficient transfer of laser energy from the fiber guide tube 5 to the fiber guide tube 23 vitiates any need for the conventional air knife cooling system 11 (Figure 1b), since little laser energy is dissipated. The first fiber guide tube 5 comprises a trunk fiberoptic, which comprises one of calcium fluoride (CaF), calcium oxide (CaO2), zirconium oxide (ZrO2), zirconium fluoride (ZrF), sapphire, hollow waveguide, liquid core, TeX glass, quartz silica, germanium sulfide, arsenic sulfide, and germanium oxide (GeO2).

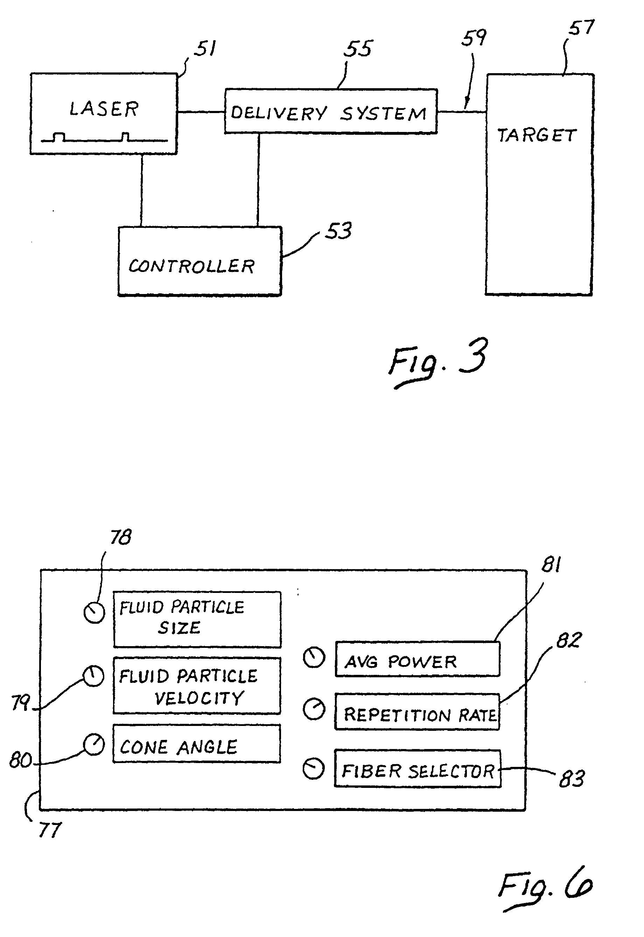

Figure 3 is a block diagram illustrating the electromagnetically induced mechanical cutter of the present invention. An electromagnetic energy source 51 is coupled to both a controller 53 and a delivery system 55. The delivery system 55 imparts mechanical forces onto the target surface 57. As presently embodied, the delivery system 55 comprises a fiberoptic guide for routing the laser 51 into an interaction zone 59, located above the target surface 57. The delivery system 55 further comprises an atomizer for delivering user-specified combinations of atomized fluid particles into the interaction zone 59. The controller 53 controls various operating parameters of the laser 51, and further controls specific characteristics of the user-specified combination of atomized fluid particles output from the delivery system 55.

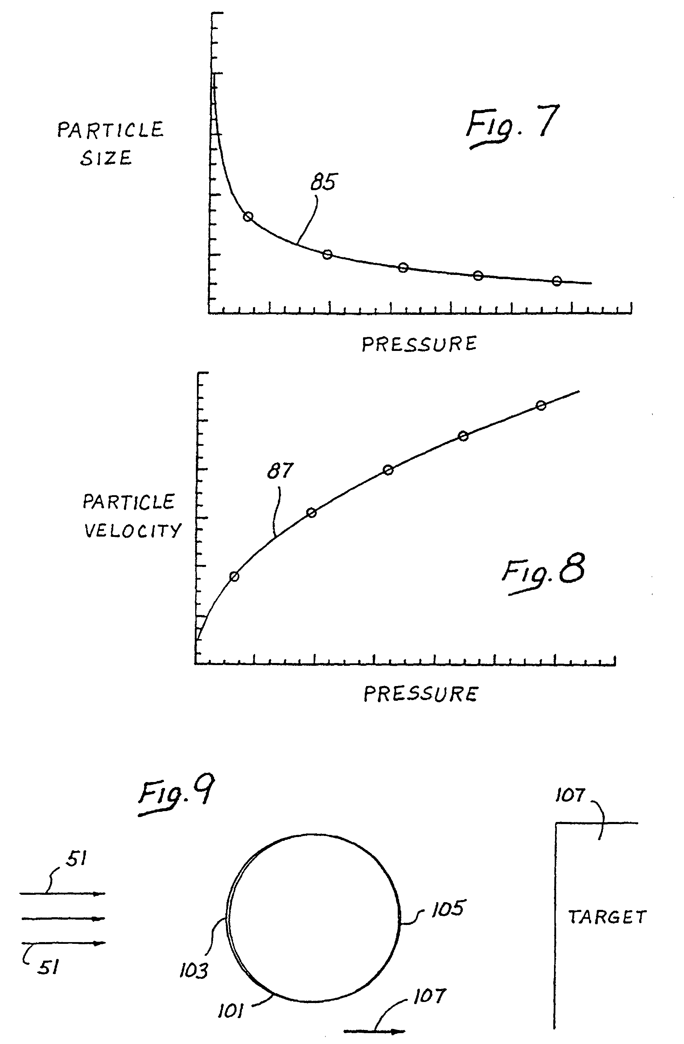

Figure 4 shows a simple embodiment of the electromagnetically induced mechanical cutter of the present invention, in which a fiberoptic guide 61, an air tube 63, and a water tube 65 are placed within a hand-held housing 67. The water tube 65 is preferably operated under a relatively low pressure, and the air tube 63 is preferably operated under a relatively high pressure. The laser energy from the fiberoptic guide 61 focuses onto a combination of air and water, from the air tube 63 and the water tube 65, at the interaction zone 59. Atomized fluid particles in the air and water mixture absorb energy from the laser energy of the fiberoptic tube 61, and explode. The explosive forces from these atomized fluid particles impart mechanical cutting forces onto the target 57.

Turning back to Figure 1b, the prior art optical cutter focuses laser energy on a target surface at an area A, for example, and the electromagnetically induced mechanical cutter of the present invention focuses laser energy into an interaction zone B, for example. The prior art optical cutter uses the laser energy directly to cut tissue, and the electromagnetically induced mechanical cutter of the present invention uses the laser energy to expand atomized fluid particles to thus impart mechanical cutting forces onto the target surface. The prior art optic cutter must use a large amount of laser energy to cut the area of interest, and also must use a large amount of water to both cool this area of interest and remove cut tissue.

In contrast, the electromagnetically induced mechanical cutter of the present invention uses a relatively small amount of water and, further, uses only a small amount of laser energy to expand atomized fluid particles generated from the water. According to the electromagnetically induced mechanical cutter of the present invention, water is not needed to cool the area of surgery, since the exploded atomized fluid particles are cooled by exothermic reactions before they contact the target surface. Thus, atomized fluid particles of the present invention are heated, expanded, and cooled before contacting the target surface. The electromagnetically induced mechanical cutter of the present invention is thus capable of cutting without charring or discoloration.

Figure 5 illustrates the presently preferred embodiment of the electromagnetically induced mechanical cutter. The atomizer for generating atomized fluid particles comprises a nozzle 71, which may be interchanged with other nozzles (not shown) for obtaining various spatial distributions of the atomized fluid particles, according to the type of cut desired. A second nozzle 72, shown in phantom lines, may also be used. The cutting power of the electromagnetically induced mechanical cutter is further controlled by the user control (not shown). In a simple embodiment, the user control controls the air and water pressure entering into the nozzle 71. The nozzle 71 is thus capable of generating many different user-specified combinations of atomized fluid particles and aerosolized sprays.

Intense energy is emitted from the fiberoptic guide 23. This intense energy is preferably generated from a coherent source, such as a laser. In the presently preferred embodiment, the laser comprises an erbium, chromium, yttrium, scandium, gallium garnet (Er, Cr:YSGG) solid state laser, which generates light having a wavelength in a range of 2.70 to 2.80 microns. As presently preferred, this laser has a wavelength of approximately 2.78 microns. Although the fluid emitted from the nozzle 71 preferably comprises water, other fluids may be used and appropriate wavelengths of the electromagnetic energy source may be selected to allow for high absorption by the fluid.

When fluids besides mere water are used, the absorption of the light energy changes and cutting efficiency is thus affected. Alternataively, when using certain fluids containing pigments or dyes, laser systems of different wavelengths such as Neodymium yttrium aluminum garnet-Nd:YAG wavelengths may be selected to allow for high absorption by the fluid.

Other possible laser systems include an erbium, yttrium, scandium, gallium garnet (Er:YSGG) solid state laser, which generates electromagnetic energy having a wavelength in a range of 2.70 to 2.80 microns; an erbium, yttrium, aluminum garnet (Er:YAG) solid state laser, which generates electromagnetic energy having a wavelength of 2.94 microns; chromium, thulium, erbium, yttrium, aluminum garnet (CTE:YAG) solid state laser, which generates electromagnetic energy having a wavelength of 2.69 microns; erbium, yttrium orthoaluminate (Er:YALO3) solid state laser, which generates electromagnetic energy having a wavelength in a range of 2.71 to 2.86 microns; holmium, yttrium, aluminum garnet (Ho:YAG) solid state laser, which generates electromagnetic energy having a wavelength of 2.10 microns; quadrupled neodymium, yttrium, aluminum garnet (quadrupled Nd:YAG) solid state laser, which generates electromagnetic energy having a wavelength of 266 nanometers; argon fluoride (ArF) excimer laser, which generates electromagnetic energy having a wavelength of 193 nanometers; xenon chloride (XeCl) excimer laser, which generates electromagnetic energy having a wavelength of 308 nanometers; krypton fluoride (KrF) excimer laser, which generates electromagnetic energy having a wavelength of 248 nanometers; and carbon dioxide (CO2), which generates electromagnetic energy having a wavelength in a range of 9.0 to 10.6 microns. Water is chosen as the preferred fluid because of its biocompatibility, abundance, and low cost. The actual fluid used may vary as long as it is properly matched (meaning it is highly absorbed) to the selected electromagnetic energy source (i.e. laser) wavelength.

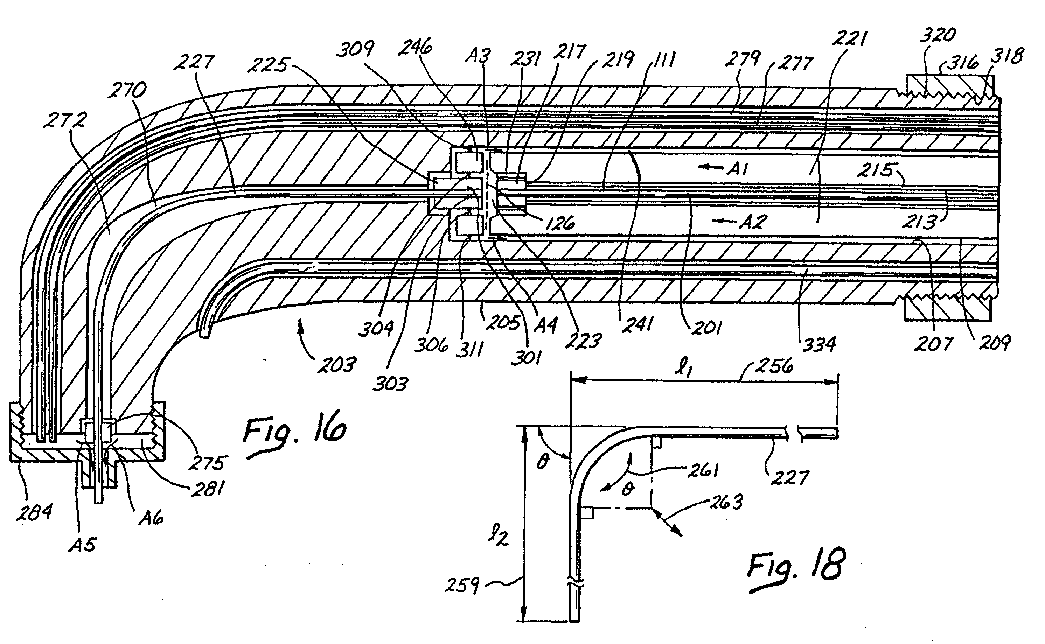

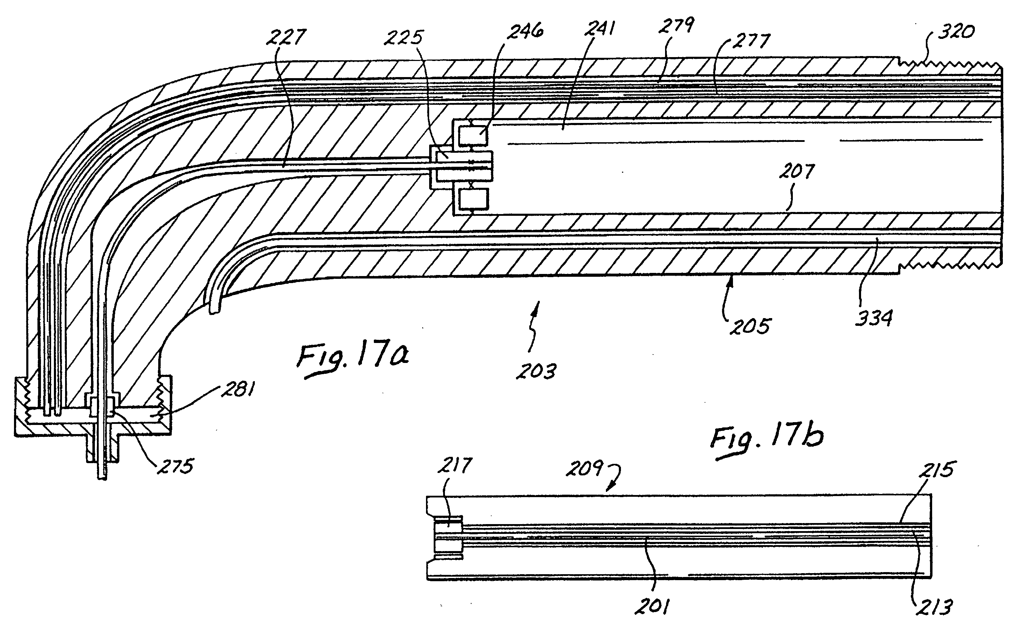

The delivery system 55 for delivering the electromagnetic energy includes a fiberoptic energy guide or equivalent which attaches to the laser system and travels to the desired work site. Fiberoptics or waveguides are typically long, thin and lightweight, and are easily manipulated. Fiberoptics can be made of calcium fluoride (CaF), calcium oxide (CaO2), zirconium oxide (ZrO2), zirconium fluoride (ZrF), sapphire, hollow waveguide, liquid core, TeX glass, quartz silica, germanium sulfide, arsenic sulfide, germanium oxide (GeO2), and other materials. Other delivery systems include devices comprising mirrors, lenses and other optical components where the energy travels through a cavity, is directed by various mirrors, and is focused onto the targeted cutting site with specific lenses. As will be discussed later with reference to Figures 16-19, the preferred embodiment of light delivery for medical applications of the present invention is through a fiberoptic conductor, because of its light weight, lower cost, and ability to be packaged inside of a handpiece of familiar size and weight to the surgeon, dentist, or clinician. In industrial applications, non-fiberoptic systems may be used.

The nozzle 71 is employed to create an engineered combination of small particles of the chosen fluid. The nozzle 71 may comprise several different designs including liquid only, air blast, air assist, swirl, solid cone, etc. When fluid exits the nozzle 71 at a given pressure and rate, it is transformed into particles of user-controllable sizes, velocities, and spatial distributions.

Figure 6 illustrates a control panel 77 for allowing user-programmability of the atomized fluid particles. By changing the pressure and flow rates of the fluid, for example, the user can control the atomized fluid particle characteristics. These characteristics determine absorption efficiency of the laser energy, and the subsequent cutting effectiveness of the electromagnetically induced mechanical cutter. This control panel may comprise, for example, a fluid particle size control 78, a fluid particle velocity control 79, a cone angle control 80, an average power control 81, a repetition rate 82, and a fiber selector 83.

The cone angle may be controlled, for example, by changing the physical structure of the nozzle 71. For example, various nozzles 71 may be interchangeably placed on the electromagnetically induced mechanical cutter. Alternatively, the physical structure of a single nozzle 71 may be changed.

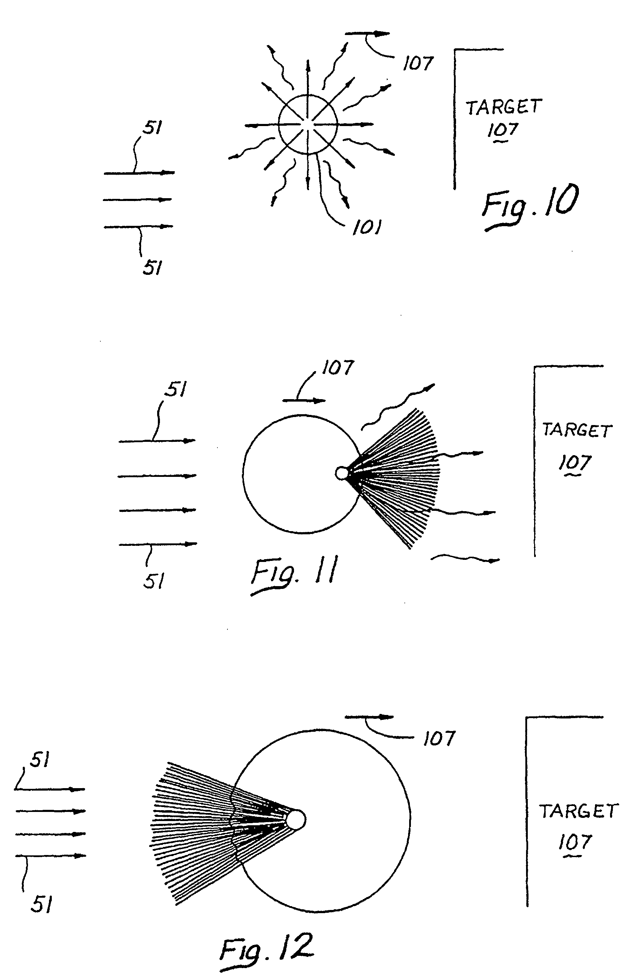

Figure 7 illustrates a plot 85 of mean fluid particle size versus pressure. According to this figure, when the pressure through the nozzle 71 is increased, the mean fluid particle size of the atomized fluid particles decreases. The plot 87 of Figure 8 shows that the mean fluid particle velocity of these atomized fluid particles increases with increasing pressure.

According to the present invention, materials are removed from a target surface by mechanical cutting forces, instead of by conventional thermal cutting forces. Laser energy is used only to induce mechanical forces onto the targeted material. Thus, the atomized fluid particles act as the medium for transforming the electromagnetic energy of the laser into the mechanical energy required to achieve the mechanical cutting effect of the present invention. The laser energy itself is not directly absorbed by the targeted material. The mechanical interaction of the present invention is safer, faster, and eliminates the negative thermal side-effects typically associated with conventional laser cutting systems.

The fiberoptic guide 23 (Figure 5) can be placed into close proximity of the target surface. This fiberoptic guide 23, however, does not actually contact the target surface. Since the atomized fluid particles from the nozzle 71 are placed into the interaction zone 59, the purpose of the fiberoptic guide 23 is for placing laser energy into this interaction zone, as well. A novel feature of the present invention is the formation of the fiberoptic guide 23 of sapphire. Regardless of the composition of the fiberoptic guide 23, however, another novel feature of the present invention is the cleaning effect of the air and water, from the nozzle 71, on the fiberoptic guide 23.

Applicants have found that this cleaning effect is optimal when the nozzle 71 is pointed somewhat directly at the target surface. For example, debris from the mechanical cutting are removed by the spray from the nozzle 71.

Additionally, applicants have found that this orientation of the nozzle 71, pointed toward the target surface, enhances the cutting efficiency of the present invention. Each atomized fluid particle contains a small amount of initial kinetic energy in the direction of the target surface. When electromagnetic energy from the fiberoptic guide 23 contacts an atomized fluid particle, the spherical exterior surface of the fluid particle acts as a focusing lens to focus the energy into the water particle's interior.

As shown in Figure 9, the water particle 101 has an illuminated side 103, a shaded side 105, and a particle velocity 107. The focused electromagnetic energy is absorbed by the water particle 201, causing the interior of the water particle to heat and explode rapidly. This exothermic explosion cools the remaining portions of the exploded water particle 201. The surrounding atomized fluid particles further enhance cooling of the portions of the exploded water particle 201. A pressure-wave is generated from this explosion. This pressure-wave, and the portions of the exploded water particle 201 of increased kinetic energy, are directed toward the target surface 107. The incident portions from the original exploded water particle 201, which are now traveling at high velocities with high kinetic energies, and the pressure-wave, impart strong, concentrated, mechanical forces onto the target surface 107.

These mechanical forces cause the target surface 107 to break apart from the material surface through a "chipping away" action. The target surface 107 does not undergo vaporization, disintegration, or charring. The chipping away process can be repeated by the present invention until the desired amount of material has been removed from the target surface 107. Unlike prior art systems, the present invention does not require a thin layer of fluid. In fact, it is preferred that a thin layer of fluid does not cover the target surface, since this insulation layer would interfere with the above-described interaction process.

Figures 10, 11 and 12 illustrate various types of absorptions of the electromagnetic energy by atomized fluid particles. The nozzle 71 is preferably configured to produce atomized sprays with a range of fluid particle sizes narrowly distributed about a mean value. The user input device for controlling cutting efficiency may comprise a simple pressure and flow rate gauge or may comprise a control panel as shown in Figure 6, for example. Upon a user input for a high resolution cut, relatively small fluid particles are generated by the nozzle 71. Relatively large fluid particles are generated for a user input specifying a low resolution cut. A user input specifying a deep penetration cut causes the nozzle 71 to generate.a relatively low density distribution of fluid particles, and a user input specifying a shallow penetration cut causes the nozzle 71 to generate a relatively high density distribution of fluid particles. If the user input device comprises a simple pressure and flow rate gauge, then a relatively low density distribution of relatively small fluid particles can be generated in response to a user input specifying a high cutting efficiency. Similarly, a relatively high density distribution of relatively large fluid particles can be generated in response to a user input specifying a low cutting efficiency. Other variations are also possible.

These various parameters can be adjusted according to the type of cut and the type of tissue. Hard tissues include tooth enamel, tooth dentin, tooth cementum, bone, and cartilage. Soft tissues, which the electromagnetically induced mechanical cutter of the present invention is also adapted to cut, include skin, mucosa, gingiva, muscle, heart, liver, kidney, brain, eye, and vessels. Other materials may include glass or crystalline materials and semiconductor chip surfaces, for example. In the case of bone tissues, for example, a portion of cancer affected bone may be removed by the electromagnetically induced mechanical cutter of the present invention. The electromagnetically induced mechanical cutter of the present invention provides a clean, high-precision cut with minimized cross-contamination, and thus allows for a precise removal of the cancer affected bone. After the bone is cut, it tends to grow back with an increased success rate and with a reduction in the likelihood of cross-contamination.

In the case of glass or crystalline materials, for example, the surface of the glass or crystalline material may be conventionally prepared using acid before silver or other dielectric materials are adhered to the glass or crystalline material surface to make a mirror. The conventional use of acid can undesirably slightly degrade the surface of the glass or crystalline material by unevenly reacting with the surface and by changing the structure on the surface. The electromagnetically induced mechanical cutter of the present invention, however, can be used to remove a thin layer from the surface in a uniform manner, to thereby clean and degrease the surface in preparation for adhesion of the silver or other dielectric material. Use of the electromagnetically induced mechanical cutter of the present invention on the surface further does not change the microscopical structure of the glass or crystalline material.

In the case of semi-conductor chips, these chips are formed from silicon wafers. A silicon crystal is first grown, before the silicon crystal is sliced into silicon wafers. Many different fabrication procedures are available. According to a series of substeps used in one exemplary procedure, each silicon wafer is coated with a layer of silicon dioxide using conventional means. The goal is to selectively deposit dopants into the silicon wafer, to thereby form conductive paths or circuits in the silicon wafer. In order to accomplish this goal, portions of the silicon dioxide layer are selectively removed in places where the dopant is to be deposited. The dopant is then deposited over the entire wafer, but only the portions of the wafer not covered by the silicon dioxide layer receive the dopant. The areas covered by the silicon dioxide layer are not penetrated by the dopant, since the dopant over these areas is absorbed by the layer of silicon dioxide.

A fairly involved procedure is used to accomplish the selective removal of the portions of the layer of silicon dioxide, before the introduction of dopants into the silicon wafer. The first step required for the conventional selective removal of portions of the silicon dioxide layer involves application of a coat of light-sensitive polymer material commonly referred to as a resist. A few drops of the resist are conventionally applied to the wafer as the wafer is spun rapidly, in order to apply an even coat of the resist and in order to effectuate drying thereof. Next, a partially transparent photographic negative or photomask is placed over the wafer and aligned using a microscope, for example. The photomask is transparent only in areas where silicon dioxide is to be removed, for positive resist, and the opposite stands true for negative resist. The photomask is then exposed to ultraviolet or near ultraviolet light. The transparent portions of the photomask pass the light onto corresponding portions of the resist. The regions of the resist that receive the light are structurally changed, and the regions of the resist that do not receive the light (those regions beneath the photomask) are not affected. For a negative resist, the molecules of the resist which are illuminated become cross linked (polymerized). For a positive resist, the molecular bonds of the resist that are illuminated are broken. The unpolymerized areas of the resist can then be dissolved, using a solvent such as trichloroethylene. The polymerized areas of the resist are acid-resistant and thus are not affected by the solvent, so that the photomask is replicated by the remaining protective coating of oxide.

The remaining resist, however, must then be removed with a chemical and water compound. Preferably, the chemical and water compound will completely remove the resist, and will be completely washed away without any remnants remaining on the silicon wafer. After the resist and the chemical and water compound are removed, the dopants are implanted into the silicon wafer using ion implantation, for example. Subsequently, a portion of the silicon wafer, which was originally covered with the resist and the chemical and water compound, will have a conductor adhered thereto. A light acid etch may be applied to these areas before the adhesion of a conductor thereto, to thereby slightly roughen the silicon wafer surface and improve adhesion.

In one application of the present invention, the electromagnetically induced mechanical cutter may be used to directly selectively etch the layer of silicon dioxide. In such an application, the electromagnetically induced mechanical cutter is focussed directly onto the silicon dioxide layer to thereby remove portions thereof. Resist, photomasks, ultraviolet light, solvents, chemical and water compounds, and acids are not needed in this application, since the portions of the silicon dioxide layer are removed directly with the electromagnetically induced mechanical cutter. The control panel 77 of Figure 6 may be used to control the cutting resolution and the cutting depth of the electromagnetically induced mechanical cutter. Precision equipment for implementing cutting patterns corresponding to images of the photomask, for example, are preferably used to control the removal of portion of the silicon dioxide layer by the electromagnetically induced mechanical cutter.

In another application of the present invention, the electromagnetically induced mechanical cutter may be used in place of the chemical and water compound, to remove the layer of resist. In this application, the chemical and water compound is not needed, resulting in a savings in water, for example. Also, a very good contaminant-free surface for adhesion ion implantation is formed, since the chemical and water compound is not used. This contaminant-free surface is suitable for any subsequent adhesion of a conductor to a dopant-implanted portion of the silicon wafer. The electromagnetically induced mechanical cutter may be used with atomized fluid particles comprising distilled water, which is relatively free of contaminants, for example. A very shallow cut or ablation is preferably generated to remove only the layer of remaining resist. The use of precision equipment, in combination with the control panel 77 of Figure 6, is preferred for implementing shallow surface layer removal patterns on the silicon wafer corresponding to regions of resist that need to be removed. The ablating may be done using a focussed cutting beam of the electromagnetically induced mechanical cutter, or the cutting beam of the electromagnetically induced mechanical cutter may be dispersed in order to cover a larger portion of the chip. For example, a focussed cutting beam may be rapidly scanned across portions of resist; or a larger defocussed cutting beam, or a number of beams, may be scanned or applied without scanning onto the portions of resist.

A wide variety of other semiconductor chip fabrication procedures are available, including CMOS, bipolar, C4 and other multi-chip module or flip-chip technologies, which may be used to fabricate various active and passive components, including resistors, transistors, and capacitors. Additionally, fabrication of other non-component elements, such as vias, may be used with the electromagnetically induced mechanical cutter of the present invention. The electromagnetically induced mechanical cutter may be used to cut or remove any of a variety of materials in any of these or other similar procedures.

A user may adjust the combination of atomized fluid particles exiting the nozzle 71 to efficiently implement cooling and cleaning of the fiberoptics 23 (Figure 5). According to the presently preferred embodiment, the combination of atomized fluid particles may comprise a distribution, velocity, and mean diameter to effectively cool the fiberoptic guide 23, while simultaneously keeping the fiberoptic guide 23 clean of particular debris which may be introduced thereon by the surgical site.

Looking again at Figure 9, electromagnetic energy contacts each atomized fluid particle 101 on its illuminated side 103 and penetrates the atomized fluid particle to a certain depth. The focused electromagnetic energy is absorbed by the fluid, inducing explosive vaporization of the atomized fluid particle 101.

The diameters of the atomized fluid particles can be less than, almost equal to, or greater than the wavelength of the incident electromagnetic energy. In each of these three cases, a different interaction occurs between the electromagnetic energy and the atomized fluid particle. Figure 10 illustrates a case where the atomized fluid particle diameter is less than the wavelength of the electromagnetic energy (d<λ). This case causes the complete volume of fluid inside of the fluid particle 101 to absorb the laser energy, inducing explosive vaporization. The fluid particle 101 explodes, ejecting its contents radially. Applicants refer to this phenomena as the "explosive grenade" effect. As a result of this interaction, radial pressure-waves from the explosion are created and projected in the direction of propagation. The direction of propagation is toward the target surface 107, and in the presently preferred embodiment, both the laser energy and the atomized fluid particles are traveling substantially in the direction of propagation.

The resulting portions from the explosion of the water particle 101, and the pressure-wave, produce the "chipping away" effect of cutting and removing of materials from the target surface 107. Thus, according to the "explosive grenade" effect shown in Figure 10, the small diameter of the fluid particle 101 allows the laser energy to penetrate and to be absorbed violently within the entire volume of the liquid. Explosion of the fluid particle 101 can be analogized to an exploding grenade, which radially ejects energy and shrapnel. The water content of the fluid particle 101 is evaporated due to the strong absorption within a small volume of liquid, and the pressure-waves created during this process produce the material cutting process.

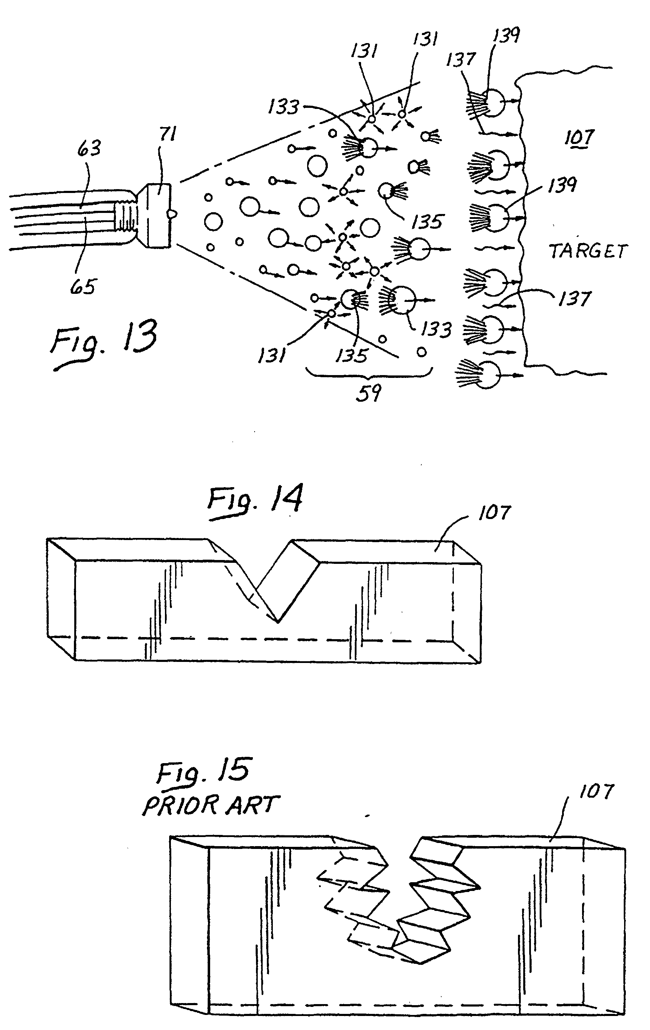

Figure 11 shows a case where the fluid particle 101 has a diameter, which is approximately equal to the wavelength of the electromagnetic energy (d≈λ). According to this "explosive ejection" effect, the laser energy travels through the fluid particle 101 before becoming absorbed by the fluid therein. Once absorbed, the fluid particle's shaded side heats up, and explosive vaporization occurs. In this case, internal particle fluid is violently ejected through the fluid particle's shaded side, and moves rapidly with the explosive pressure-wave toward the target surface. As shown in Figure 11, the laser energy is able to penetrate the fluid particle 101 and to be absorbed within a depth close to the size of the particle's diameter. The center of explosive vaporization in the case shown in Figure 11 is closer to the shaded side 105 of the moving fluid particle 101. According to this "explosive ejection" effect shown in Figure 11, the vaporized fluid is violently ejected through the particle's shaded side toward the target surface 107.

A third case shown in Figure 12 is the "explosive propulsion" effect. In this case, the diameter of the fluid particle is larger than the wavelength of the electromagnetic energy (d>λ). In this case, the laser energy penetrates the fluid particle 101 only a small distance through the illuminated surface 103 and causes this illuminated surface 103 to vaporize. The vaporization of the illuminated surface 103 tends to propel the remaining portion of the fluid particle 101 toward the targeted material surface 107. Thus, a portion of the mass of the initial fluid particle 101 is converted into kinetic energy, to thereby propel the remaining portion of the fluid particle 101 toward the target surface with a high kinetic energy. This high kinetic energy is additive to the initial kinetic energy of the fluid particle 101. The effects shown in Figure 12 can be visualized as a micro-hydro rocket with a jet tail, which helps propel the particle with high velocity toward the target surface 107. The exploding vapor on the illuminated side 103 thus supplements the particle's initial forward velocity.

The combination of Figures 10-12 is shown in Figure 13. The nozzle 71 produces the combination of atomized fluid particles which are transported into the interaction zone 59. The laser 51 is focused on this interaction zone 59. Relatively small fluid particles 131 explode via the "grenade" effect, and relatively large fluid particles 133 explode via the "explosive propulsion" effect. Medium sized fluid particles, having diameters approximately equal to the wavelength of the laser 51 and shown by the reference number 135, explode via the "explosive ejection" effect. The resulting pressure-waves 137 and exploded fluid particles 139 impinge upon the target surface 107.

Figure 14 illustrates the clean, high resolution cut produced by the electromagnetically induced mechanical cutter of the present invention. Unlike the cut of the prior art shown in Figure 15, the cut of the present invention is clean and precise. Among other advantages, this cut provides an ideal bonding surface, is accurate, and does not stress remaining materials surrounding the cut,

Turning to Figure 16, the presently preferred embodiment of light delivery for medical applications comprises a fiberoptic conductor or trunk fiber 201, housed within a medical handpiece 203. The medical handpiece 203 comprises a sleeve housing 205 having an elongate aperture 207 for accommodating a shaft assembly 209. The shaft assembly 209 accommodates the trunk fiber 201, which is wrapped with one or more jackets 211 in the presently preferred embodiment. The jackets 211 are snugly formed around the trunk fiber 201. In addition to the jackets 211, an inner protective tube 213 and an outer protective tube 215 are disposed around the trunk fiber 201. In contrast to the jacket 201, both the inner protective tube 213 and the outer protective tube 215 are slidably disposed over the trunk fiber 201. According to the present invention, the inner protective tube 213 and the outer protective tube 215 add strength to the trunk fiber 201 and prevent the trunk fiber 201 from being damaged or breaking as a result of bending or other stresses placed on the trunk fiber 201. For example, the permanent ferrule 217, as presently embodied, houses only the trunk fiber 201 and the jackets 211, and comprises a rigid material. Any bending or other stresses placed on the trunk fiber 201 would, conventionally, put the trunk fiber 201 at a high risk of breaking either within the permanent ferrule 217 or at the proximal end 219 of the ferrule 217. The inner protective tube 213 and the outer protective tube 215 distribute bending and other stressful forces on the trunk fiber 201 across a larger surface area of the trunk fiber 201, to thereby attenuate risks of breaking of the trunk fiber 201. As presently embodied, the inner protection tube 213 is secured (preferably glued) to both the permanent ferrule 217 and the SMA connector 341, but the outer protective tube 215 is secured to only the SMA connector 341 and abuts against the permanent ferrule 217. The inner protection tube 213 is preferably secured to both the permanent ferrule 217 and the SMA connector 341 to prevent rotation of the trunk fiber 201, when the permanent ferrule 217 and the SMA connector 341 are rotated relative to one another. These two protective tubes 213 and 215 are therefore able to slide over both one another and the jacket 211. Although two protective tubes 213 and 215 are presently embodied, a single protective tube may be used, or three or more protective tubes may used, according to desired operational parameters. The protective tubes preferably comprise a plastic material, but may also comprise metals or Teflon.

The permanent ferrule 217 is secured to the shaft assembly 209 via an outer housing, which defines an air conditioning input chamber 221. The air conditioning input chamber 221 is similar to the air knife line 11 of Figure 1b in that the air conditioning input chamber 221 provides air to the interface 223 for cooling the distal end of the trunk fiber 201, the permanent ferrule 217, the intermediate ferrule 225, and the proximal end of the bent fiber tip 227. The permanent ferrule 217 and the intermediate ferrule 225 are separated by a spacer 126. Air travels through the air conditioning input chamber 221 in the direction of the arrows A1 and A2 and passes through a plurality of drilled apertures, shown generally at 231 and 233, within the permanent ferrule 217. The air from the air conditioning input chamber 221 exits from the drilled apertures 231 and 233 into the interface 223, and passes from the interface 223 into the air cooling exhaust chamber 241. The air travels through the air cooling exhaust chamber 241 in the directions of the arrows A3 and A4 back out of the medical handpiece 203. As presently embodied, the air conditioning input chamber 221 and the air cooling exhaust chamber 241 are both angularly shaped and concentric.

According to the present invention, the permanent ferrule 217, the intermediate ferrule 225, and the spacer 226 comprise a heat resistant material, such as ceramic. This heat resistant material facilitates greater operating temperatures of the permanent ferrule 217, the intermediate ferrule 225, and the spacer 226 and, consequently, requires less air flow through the air conditioning input chamber 221 and the air cooling exhaust chamber 241. The permanent ferrule 217, the intermediate ferrule 225, and the spacer 226 may also comprise a crystalline material, such as sapphire. A fiber-to-fiber coupler 246 surrounds the intermediate ferrule 225, and may comprise any heat resistant material similar to that of the intermediate ferrule 225 or, alternatively, may comprise a metal such as aluminum or stainless steel.

Optical energy is coupled from the distal end of the trunk fiber 201 into the proximal end of the bent fiber tip 227. The bent fiber tip 227 provides a desirable curvature, and, consequently, does not require the fiberoptic to be stressed or strained from bending, for example. According to the present invention, the bent fiber tip 227 is manufactured of a crystalline material, and is pre-formed before installation into a bent configuration having a predetermined angle.

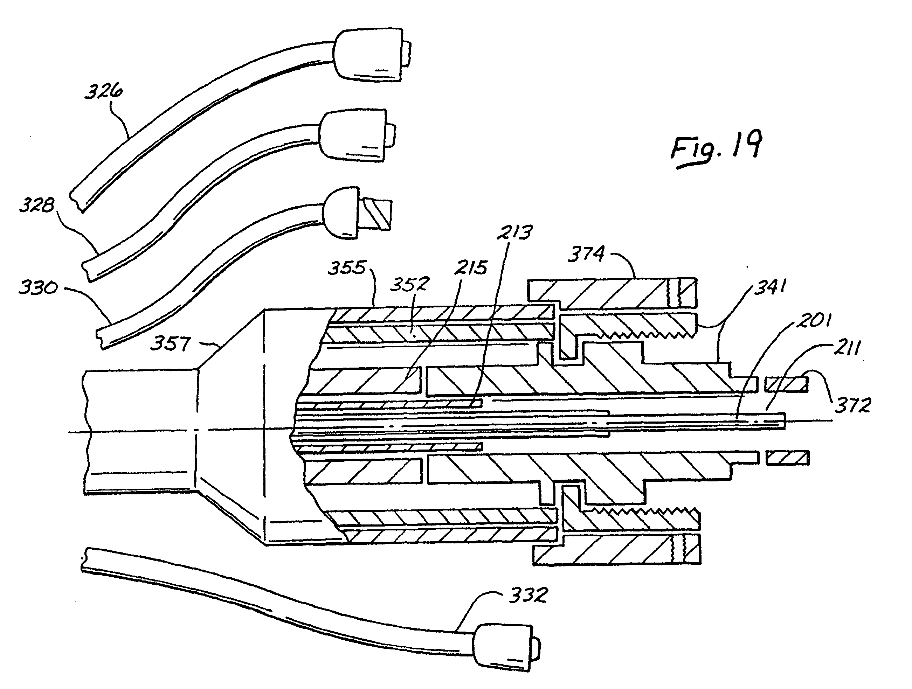

As illustrated in Figure 18, the presently preferred embodiment of the bent fiber tip 227 has a first length 256 of approximately 30 millimeters, a second length 259 of approximately 20 millimeters, and an angle 261 of approximately 90 degrees. A radial length 263 is preferably 10 millimeters. According to the present invention, the crystalline fiber 227 is bent by hand under a flame of approximately 2000 degrees Fahrenheit (1093°C). The bent fiber tip 227 fits within a cavity 270 (Figure 16), which has an enlarged cavity area at the bending portion 272 to accommodate bent fiber tips 227 having slight manufacturing deviations. The bent crystalline material must subsequently be tested to insure that the bent crystalline material has ideal optical qualities. Although the presently preferred embodiment of the bent fiber tip 227 comprises an angle of approximately 90 degrees, other bent tips may be manufactured having one or more angles from zero to 180 degrees, and different shapes, according to design preference. Also, although the bent fiber tip 227 of the presently preferred embodiment extends from the intermediate ferrule 225 to a tip ferrule 275, a variety of other configurations are possible. As just one example, a small-diameter, flexible bent crystalline fiber may extend from the laser source (not shown) and standard miniature type-A (SMA) connector 341 (Figure 19) to the tip ferrule 275. As presently embodied, the bent fiber tip 227 comprises sapphire which, according to the present invention, is ideal for carrying wavelengths on the order of approximately 3 microns. In the presently preferred embodiment, the bent fiber tip 227 formed of sapphire conducts wavelengths of 2.78 and 2.94 microns. In addition to sapphire, any other crystalline materials suitable for conducting optical energy may be used, according to design parameters.

The tip ferrule 275 holds the bent fiber tip 227 securely in place near the radiation delivery end of the medical handpiece 203. The tip ferrule 275 may comprise a heat resistant material, such as described above with reference to the permanent ferrule 217 and the intermediate ferrule 225. An air line 277 and a water line 279 disposed within the sleeve housing 205 output air and water into the mixing chamber 281, which is defined by the radiation delivery end of the sleeve housing 205 and the cap 284. The air and water from the air line 277 and the water line 279 form an air assist spray within the mixing chamber 281. The air assist spray exits the mixing chamber 281 in the directions of the arrows A5 and A6 along the output end of the bent fiber tip 227.

According to another feature of the present invention, another crystalline material suitable for conducting optical energy may be disposed around the bent fiber tip 227 near the interface 223. This additional crystalline material may comprise the intermediate ferrule 225 or, alternatively, may be a cylindrical crystalline material disposed around the bent fiber tip 227 near the interface 223 and also surrounded by the intermediate ferrule 225. In some instances, it may be desirable to form the bent fiber tip 227 with a smaller diameter than the diameter of the trunk fiber 201. The trunk fiber 201 may be formed of a relatively large diameter, for example, for added strength. If the bent fiber tip 227 is very small in diameter, such as, for example, 50 microns, then the proximal end of this bent fiber tip 227 near the interface 223 may be damaged from a relatively large amount of energy output from the distal end of the trunk fiber 201. The additional annular crystalline fiber disposed around the bent fiber tip 227 near the interface 223 helps to protect the bent fiber tip 227 from damage. One or more annular crystalline fibers may be disposed around the proximal end of the bent fiber tip 227, and each of these annular crystalline fibers may extend along the length of the intermediate ferrule 225 or longer. When one or more of these annular crystalline fibers are used, as presently preferred, any of the sealed passages 301, 303, 304, 306, 309, and 311 may be opened to facilitate additional air flow and cooling therethrough. Opening of any of these passage 301-311 further facilitates an air flow path leading along the bent fiber tip 227 and into the mixing chamber 281, according to the present invention.

Figures 17a and 17b illustrate the medical handpiece 203 with the shaft assembly 209 removed from the elongate aperture 207 of the sleeve housing 205, according to the present invention. As presently embodied, a collar 316 (Figure 16) is provided with threads 318. The threads 318 of the collar 316 engage threads 320 of the proximal end of the sleeve housing 205 and allow the collar 316 to exert radially inwardly directed forces onto the sleeve housing 205. These radially inwardly directed forces from the collar 316 onto the sleeve housing 205 frictionally engage the shaft assembly 209 within the elongate aperture 207 and prevent the shaft assembly 209 from movement within the elongate aperture 207 of the sleeve housing 205.

Figure 19 illustrates many of the components which supply resources to the medical handpiece 203 of Figure 16. The water line 326 is adapted for supplying water to the water line 279 of the medical handpiece 203. Similarly, the air line 328 is adapted for supplying air to the air line 277, and the air conditioning line 330 is adapted for supplying air to the air conditioning input chamber 221. An illumination and/or medication cable 332 provides illumination and/or medication to the illumination and/or medication line 334 of the medical handpiece 203. Although the medical handpiece 203 is illustrated for operation with a laser-cutting assembly, such as that shown in Figure 1b, the presently preferred embodiment of the medical handpiece 203 operates in combination with an electromagnetically-induced mechanical cutter having a head similar to that shown in Figure 5. According to the presently preferred embodiment, the air line 277 and the water line 279 preferably correspond to the air tube 63 and the water tube 65 of the head assembly shown in Figure 5. In this presently preferred embodiment, the cap 284 of Figure 16 is not necessarily required. In one embodiment, the air line 277 corresponding to the air tube 63 is not used.

Since the distal end of the bent fiber tip 227 of the presently preferred embodiment is placed into close proximity of the target surface, a source of coherent or non-coherent illumination through the illumination line 334 (Figure 16) can be quite advantageous. On the other hand, when a traditional laser cutting mechanism is used, the illumination from the illumination line 334 may not be as advantageous and a conventional aiming beam may be routed through the illumination line 334.

The illumination and/or medication line 334 may comprise only a medication, or may comprise both an illumination line and a medication line. The medication line facilitates introduction of medications, such as anesthetics, to the target area of the patient.

Looking at Figure 19, the trunk fiber 201 and the jackets 211 are secured within a sum miniature type A (SMA) connector 341. The SMA connector facilitates introduction and securing of the trunk fiber 201 and the jacket 211 into a laser source (not shown).

The inner protective tube 213 and the outer protective tube 215 extend from the medical handpiece 203 all of the way to the SMA connector 341 and provide similar protection functions to the trunk fiber 201 at the interface of the SMA connector 341. As mentioned previously, only one protective tube may be used or, alternatively, three or more protective tubes may be used. In the presently preferred embodiment, a metal tube 352 surrounds the inner protective tube 213 and the outer protective tube 215, and an additional outer plastic tube covering 355 surrounds the metal tube 352.

A plastic protective covering 357 encases these elements surrounding the trunk fiber 201. In the presently preferred embodiment, the plastic protective covering 357 is secured to the water line 326, the air line 328, the air conditioning line 330, and the illumination and/or medication cable 332 and routed into close proximity to the medical handpiece 203. The metal tube 352 and the outer tube covering 355 do not extend to the medical handpiece and preferably terminate at a short distance from the SMA connector 341. Of course, other distances may be set according to design parameters. A power cap 372 and a grippable knob 374 further facilitate the attachment of the trunk fiber 201 to the laser source.

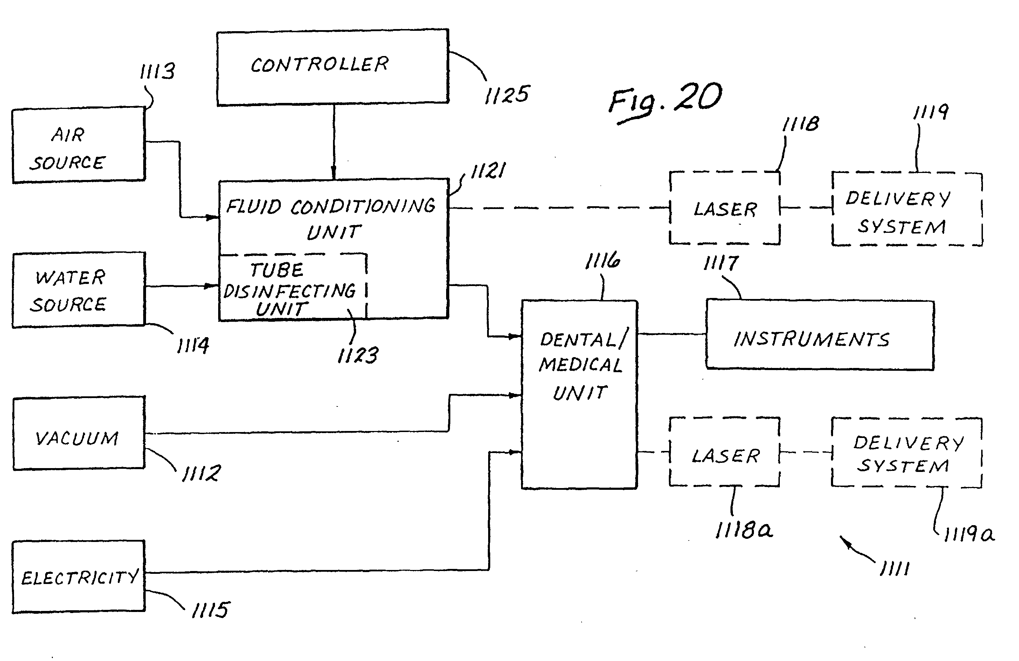

The dental/medical work station 1111 of the present invention is shown in Figure 20. The dental/medical work station 1111 comprises a conventional air line 1113 and a conventional water line 1114 for supplying air and water, respectively. A vacuum line 1112 and an electrical outlet 1115 supply negative air pressure and electricity to the dental/medical unit 1116, similarly to the vacuum 8 and electrical 14 lines shown in Figure 1a. The fluid conditioning unit 1121 may, alternatively, be placed between the dental/medical unit 1116 and the instruments 1117, for example. According to the present invention, the air line 1113 and the water line 1114 are both connected to a fluid conditioning unit 1121.

A controller 1125 allows for user inputs, to control whether air from the air line 1113, water from the water line 1114, or both, are conditioned by the fluid conditioning unit 1121. A variety of agents may be applied to the air or water by the fluid conditioning unit 1121, according to a configuration of the controller 1125, for example, to thereby condition the air or water, before the air or water is output to the dental/medical unit 1116. Flavoring agents and related substances, for example, may be used, such as disclosed in 21 C.F.R. Sections 172.510 and 172.515, the details of which are incorporated herein by reference. Colors, for example, may also be used for conditioning, such as disclosed in 21 C.F.R. Section 73.1 to Section 73.3126.

Similarly to the instruments 18 shown in Figure 1a, the instruments 1117 may comprise an electrocauterizer, an electromagnetic energy source, a laser, a mechanical drill, a mechanical saw, a canal finder, a syringe, and/or an evacuator. All of these instruments 1117 use air from the air line 1113 and/or water from the water line 1114, which may or may not be conditioned depending on the configuration of the controller 1125. Any of the instruments 1117 may alternatively be connected directly to the fluid conditioning unit 1121 or directly to any of the air 1113, water 1114, vacuum 1112, and/or electric 1115 lines. For example, a laser 1118 and delivery system 1119 is shown in phantom connected to the fluid conditioning unit 1121. The laser 1118a and delivery system 1119a may be connected to the dental/medical unit 1116, instead of being grouped with the instruments 1117.

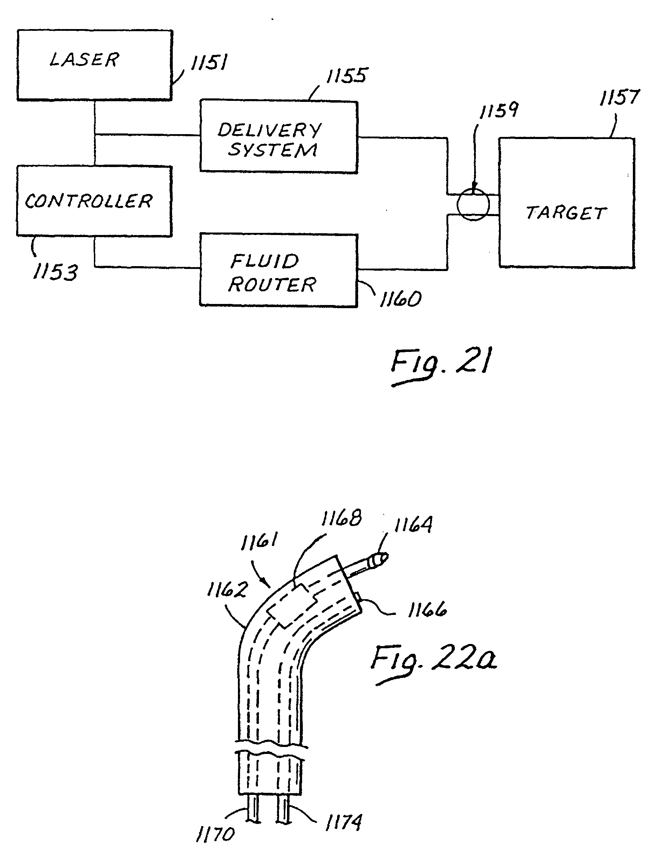

The block diagram shown in Figure 21 illustrates one embodiment of a laser 1151 directly coupled with, for example, the air 1113, water 1114, and power 1115 lines of Figure 20. A separate fluid conditioning system is used in this embodiment. As an alternative to the laser, or any other tool being connected directly to any or all of the four supply lines 1113-1115 and having an independent fluid conditioning unit, any of these tools may instead, or additionally, be connected to the dental/medical unit 1116 or the fluid conditioning unit 1121, or both.

According to the exemplary embodiment shown in Figure 21, an electromagnetically induced mechanical cutter is used for cutting. Details of this cutter are disclosed in US-A-5 741 247 to the assignee of this application. The electromagnetic cutter energy source 1151 is connected directly to the outlet 1115 (Figure 20), and is coupled to both a controller 1153 and a delivery system 1155. The delivery system 1155 routes and focuses the laser 1151. In the case of a conventional laser system, thermal cutting forces are imparted onto the target 1157. The delivery system 1155 preferably comprises a fiberoptic guide for routing the laser 1151 into an interaction zone 1159, located above the target surface 1157. The fluid router 1160 preferably comprises an atomizer for delivering user-specified combinations of atomized fluid particles into the interaction zone 1159. The atomized fluid particles are conditioned, according to the present invention, and may comprise flavors, scents, saline, and other agents, as discussed below.

In the case of a conventional laser, a stream or mist of conditioned fluid is supplied by the fluid router 1160. The controller 1153 may control various operating parameters of the laser 1151, the conditioning of the fluid from the fluid router 1160, and the specific characteristics of the fluid from the fluid router 1160.

Although the present invention may be used with conventional drills and lasers, for example, one preferred embodiment is the electromagnetically induced mechanical cutter. Other preferred embodiments include an electrocauterizer, a syringe, an evacuator, or any air or electrical driver, drilling, filling, or cleaning mechanical instrument. Figure 4 shows a simple embodiment of the electromagnetically induced mechanical cutter, in which a fiberoptic guide 61, an air tube 63, and a fluid tube 65 are placed within a hand-held housing 67. Although a variety of connections are possible, the air tube 63 and water tube 65 are preferably connected to either the fluid conditioning unit 1121 or the dental/medical unit 1116 of Figure 20. The fluid tube 65 is preferably operated under a relatively low pressure, and the air tube 63 is preferably operated under a relatively high pressure.

According to the present invention, either the air from the air tube 63 or the fluid from the fluid tube 65, or both, are selectively conditioned by the fluid conditioning unit 1121, as controlled by the controller 1125.

A mechanical drill 1161 is shown in Figure 22a, comprising a handle 1162, a drill bit 1164, and a water output 1166. The mechanical drill 1161 comprises a motor 1168, which may be electrically driven, or driven by pressurized air.

When the motor 1168 is driven by air, for example, the fluid enters the mechanical drill 1161 through the first supply line 1170. Fluid entering through the first supply line 1170 passes through the motor 1168, which may comprise a turbine, for example, to thereby provide rotational forces to the drill bit 1164. A portion of the fluid, which may not appeal to a patient's taste and/or smell, may exit around the drill bit 1164, coming into contact with the patient's mouth and/or nose. The majority of the fluid exits back through the first supply line 1170.

In the case of an electric motor, for example, the first supply line 1170 provides electric power. The second supply line 1174 supplies fluid to the fluid output 1166. The water and/or air supplied to the mechanical drill 1161 may be selectively conditioned by the fluid conditioning unit 1121, according to the configuration of the controller 1125.

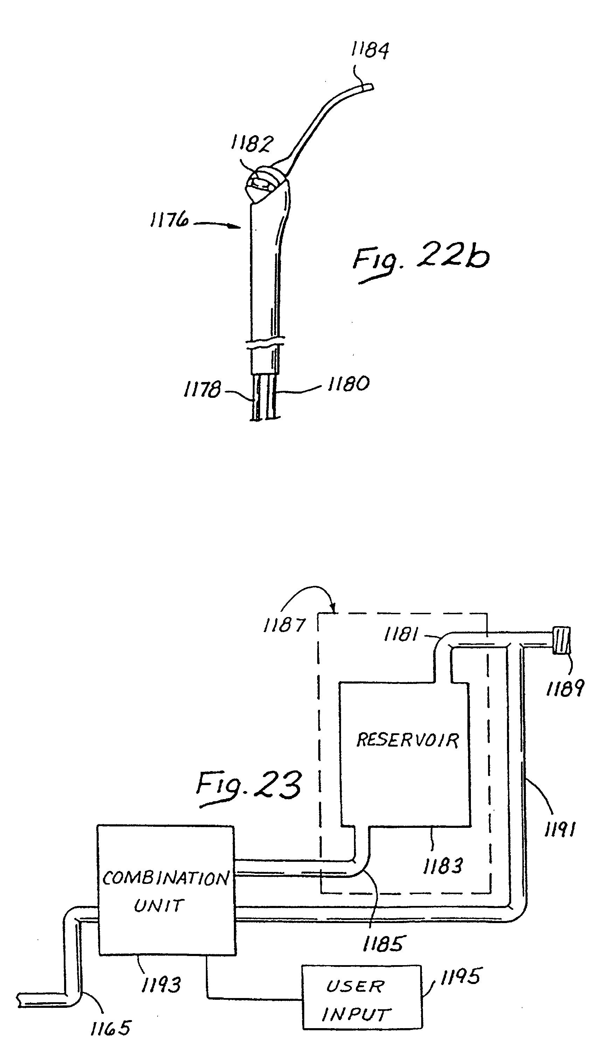

The syringe 1176 shown in Figure 22b comprises an air input line 1178 and a water input line 1180. A user control 1182 is movable between a first position and a second position. The first position supplies air from the air line 1178 to the output tip 1184, and the second position supplies water from the water line 1180 to the output tip 1184. Either the air from the air line 1178, the water from the water line 1180, or both, may be selectively conditioned by the fluid conditioning unit 1121, according to the configuration of the controller 1125, for example.

Turning to Figure 23, a portion of the fluid conditioning unit 1121 (Figure 20) is shown. This fluid conditioning unit 1121 is preferably adaptable to existing water lines 1114, for providing conditioned fluid to the dental/medical unit 1116 as a substitute for regular tap water in drilling and cutting operations, for example. The interface 1189 connects to an existing water line 1114 and feeds water through the fluid-in line 1181 and the bypass line 1191. The reservoir 1183 accepts water from the fluid-in line 1181 and outputs conditioned fluid to the fluid-out line 1185. The fluid-in line 1181, the reservoir 1183, and the fluid-out line 1185 together comprise a fluid conditioning subunit 1187.

Conditioned fluid is output from the fluid conditioning subunit 1187 into the combination unit 1193. The fluid may be conditioned by conventional means, such as the addition of a tablet, liquid syrup, or a flavor cartridge. Also input into the combination unit 1193 is regular water from the bypass line 1191. A user input 1195 into the controller 1125, for example, determines whether fluid output from the combination unit 1193 into the fluid tube 1165 comprises only conditioned fluid from the fluid-out line 1185, only regular water from the bypass line 1191, or a combination thereof. The user input 1195 comprises a rotatable knob, a pedal, or a foot switch, operable by a user, for determining the proportions of conditioned fluid and regular water. These proportions may be determined according to the pedal or knob position. In the pedal embodiment, for example, a full-down pedal position corresponds to only conditioned fluid from the fluid out-line 1185 being output into the fluid tube 1165, and a full pedal up position corresponds to only water from the bypass line 1191 being output into the fluid tube 1165. The bypass line 1191, the combination unit 1193, and the user input 1195 provide versatility, but may be omitted, according to preference. A simple embodiment for conditioning fluid would comprises only the fluid conditioning subunit 1187.

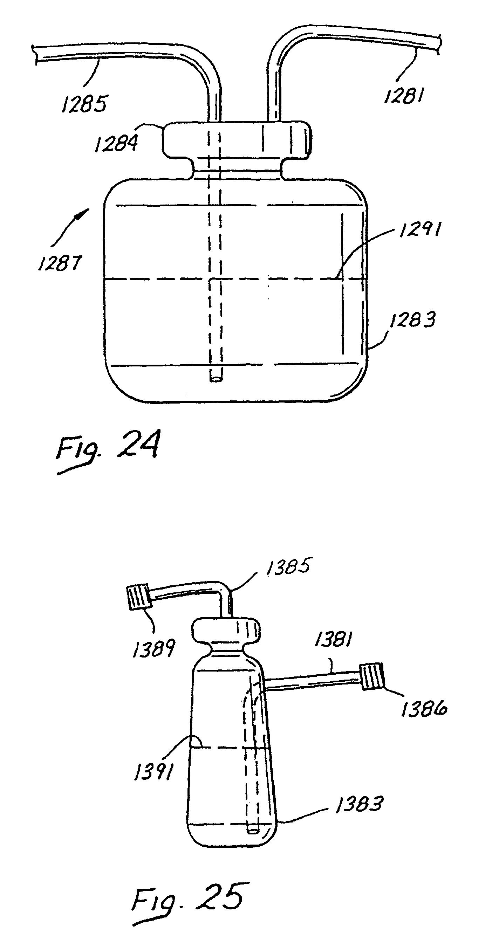

An alternative embodiment of the fluid conditioning subunit 1187 is shown in Figure 24. The fluid conditioning subunit 1287 inputs air from air line 1113 via an air input line 1281, and outputs conditioned fluid via a fluid output line 1285. The fluid output line 1285 preferably extends vertically down into the reservoir 1283 into the fluid 1291 located therein. The lid 1284 may be removed and conditioned fluid inserted into the reservoir 1283. Alternatively, a solid or liquid form of fluid conditioner may be added to water already in the reservoir 1283. The fluid is preferably conditioned, using either a scent fluid drop or a scent tablet (not shown), and may be supplied with fungible cartridges, for example.

The fluid 1291 within the reservoir 1283 may be conditioned to achieve a desired flavor, such as a fruit flavor or a mint flavor, or may be conditioned to achieve a desired scent, such as an air freshening smell. A conditioned fluid having a scent, a scented mist, or a scented source of air, may be particularly advantageous for implementation in connection with an air conditioning unit, as shown in Figure 25 and discussed below. In addition to flavor and scents, other conditioning agents may be selectively added to a conventional water line, mist line, or air line. For example, an ionized solution, such as saline water, or a pigmented solution may be added, as discussed below. Additionally, agents may be added to change the density, specific gravity, pH, temperature, or viscosity of water and/or air supplied to a drilling or cutting operation. Medications, such as antibiotics, steroids, anesthetics, anti-inflammatories, disinfectants, adrenaline, epinephrine, or astringents may be added to the water and/or air used in a drilling or cutting operation. For example, an astringent may be applied to a surgical area, via the water line to reduce bleeding. Vitamins, herbs, or minerals may also be used for conditioning the air or water used in a cutting or drilling procedure. An anesthetic or anti-inflammatory applied to a surgical wound may reduce discomfort to the patient or trauma to the wound, and an antibiotic or disinfectant may prevent infection to the wound.

The air conditioning subunit shown in Figure 25 is connectible into an existing air line 1113, via interfaces 1386 and 1389. Conventional air enters the conditioning subunit via the air input line 1381, and exits an air output line 1385. The air input line 1381 preferably extends vertically into the reservoir 1383 into a fluid 1391 within the reservoir 1383. The fluid 1391 is preferably conditioned, using either a scent fluid drop or a scent tablet (not shown). The fluid 1391 may be conditioned with other agents, as discussed above in the context of conditioning water. According to the present invention, water in the water line 1131 or air in the air line 1132 of a conventional laser cutting system (Figure 1b) is conditioned. As presently preferred, either the fluid tube 65 or the air tube 63 (Figure 4) of the electromagnetically induced mechanical cutter is conditioned. In addition to laser operations, the air and/or water of a dental drilling, irrigating, suction, or electrocautery system may also be conditioned.

Many of the above-discussed conditioning agents may change the absorption of the electromagnetic energy into the atomized fluid particles in the electromagnetically induced mechanical cutting environment of the presently preferred embodiment. Accordingly, the type of conditioning may effect the cutting power of an electromagnetic or an electromagnetically induced mechanical cutter. Thus, in addition to the direct benefits achievable through these various conditioning agents discussed above, such as flavor or medication, these various conditioning agents further provide versatility and programmability to the type of cut resulting from the electromagnetic or electromagnetically induced mechanical cutter. For example, introduction of a saline solution will reduce the speed of cutting. Such a biocompatible saline solution may be used for delicate cutting operations or, alternatively, may be used with a higher laser-power setting to approximate the cutting power achievable with regular water.

Pigmented fluids may also be used with the electromagnetic or the electromagnetically induced mechanical cutter, according to the present invention. The electromagnetic energy source may be set for maximum absorption of atomized fluid particles having a certain pigmentation, for example. These pigmented atomized fluid particles may then be used to achieve the mechanical cutting. A second water or mist source may be used in the cutting operation, but since this second water or mist is not pigmented, the interaction with the electromagnetic energy source is minimized. As just one example of many, this secondary mist or water source could be flavored.

According to another configuration, the atomized fluid particles may be unpigmented, and the electromagnetic or the electromagnetically induced energy source may be set to provide maximum energy absorption for these unpigmented atomized fluid particles. A secondary pigmented fluid or mist may then be introduced into the surgical area, and this secondary mist or water would not interact significantly with the electromagnetic energy source. As another example, a single source of atomized fluid particles may be switchable between pigmentation and non-pigmentation, and the electromagnetic energy source may be set to be absorbed by one of the two pigment states to thereby provide a dimension of controllability as to exactly when cutting is achieved.

Disinfectant may be added to an air or water source in order to combat bacteria growth within the air and water lines, and on surfaces within a dental operating room. The air and water lines of the dental unit 1116, for example, may be periodically flushed with a disinfectant selected by the controller 1125 and supplied by the fluid conditioning unit 1121. An accessory tube disinfecting unit 1123 may accommodate disinfecting cartridges and perform standardized or preprogrammed periodic flushing operations.

Even in a dental or medical procedure, an appropriate disinfectant may be used. The disinfectant may be applied at the end of a dental procedure as a mouthwash, for example, or may be applied during a medical or dental procedure. The air and water used to cool the tissue being cut or drilled within the patient's mouth, for example, is often vaporized into the air to some degree. According to the present invention, a conditioned disinfectant solution will also be vaporized with air or water, and condensate onto surfaces of the dental equipment within the dental operating room. Any bacteria growth on these moist surfaces is significantly attenuated, as a result of the disinfectant on the surfaces.

Although an exemplary embodiment of the invention has been shown and described, many changes, modifications and substitutions may be made by one having ordinary skill in the art without necessarily departing from the scope of this invention, as defined by the appended claims.

## Claims

1. An apparatus comprising:
a fluid output comprising a fluid during use and being constructed to direct the fluid to a first vicinity relative to the fluid output; and
an electromagnetic energy source for supplying electromagnetic energy to a second vicinity;
the first vicinity and the second vicinity being disposed at respective locations relative to the fluid output;
**characterised in that** said first vicinity and said second vicinity intersect in a volume (59) relative to the fluid output, said fluid output further comprises an atomizer (63,65;71) for generating a combination of atomized fluid particles, and for placing the combination of atomized fluid particles into said volume; said electromagnetic energy source comprising a specifically configured electromagnetic energy source (51) that is arranged, when said apparatus is in use, to supply electromagnetic energy of a wavelength which is substantially absorbed by said combination of atomized fluid particles and to direct a peak concentration of said electromagnetic energy into said volume; said combination of atomized fluid particles being sized and distributed in such a way that, when placed into said volume and irradiated with said focused electromagnetic energy, said electromagnetic energy is substantially absorbed by at least a portion of said combination of atomized fluid particles wherein disruptive mechanical forces are imparted to a target surface (107).

2. The apparatus of Claim 1 wherein said combination of fluid particles comprises a combination of fluid particles which are sized and distributed in such a way that when placed into said volume and irradiated with said electromagnetic energy source, said fluid particles expand.

3. The apparatus of Claim 1 or Claim 2 wherein said output comprises an atomizer, and said combination of fluid particles comprises a combination of atomized fluid particles having diameters narrowly distributed abut a mean value.

4. The apparatus according to anyone of the preceding Claims wherein when said apparatus is positioned in use such that the volume is positioned above the target surface, the portion of fluid particles expand and impart disruptive mechanical forces to said target surface.

5. The apparatus of any one of the preceding claims wherein the electromagnetic energy is highly absorbed by the portion of fluid particles.

6. The apparatus of any one of the preceding claims wherein when said apparatus is positioned in use such that said volume is positioned above a target surface said apparatus imparts disruptive cutting and ablating mechanical forces onto the target surface.

7. The apparatus according to any of the preceding claims wherein the output is fed with an air input line and a water input line.

8. The apparatus according to any one of the preceding claims, wherein the energy is delivered through a fiberoptic (23); and
wherein the fluid particles contact the fiberoptic to thereby cool and clean the fiberoptic.

9. The apparatus of Claim 8, wherein the fluid particles contact the fiberoptic to thereby remove particulate debris from the fiberoptic.

10. The apparatus of Claim 8 or Claim 9 wherein the fiberoptic comprises sapphire.

11. An apparatus according to Claim 1 further comprising :
a user control for inputting a user-specified combination of atomized fluid particles, the combination of atomized fluid particles corresponding to a user-specified average size, spatial distribution, and velocity of atomized fluid particles; wherein the atomizer is responsive to the user control.

12. The apparatus according to any one of the preceding claims, wherein the absorption of the electromagnetic energy by the portion of fluid particles in said volume causes said portion of fluid particles to impart the disruptive mechanical forces to said target surface.

13. The apparatus of Claim 12, wherein:
the disruptive mechanical forces comprise cutting and ablating mechanical-forces; and
when said apparatus is positioned in use such that said volume is positioned above the target surface, said portion of the fluid particles are caused to impart the cutting and ablating mechanical forces onto the target surface.

14. Apparatus according to Claim 11 or Claim 12, wherein said user control comprises a user input device (77) for specifying one of a high resolution cut and a low resolution cut, and for specifying one of a deep-penetration cut and a shallow penetration cut; and
said atomizer is responsive to the user input device to generate:
(1) a combination of atomized fluid particles comprising relatively small fluid particles, in response to a user input specifying a high resolution cut;
(2) a combination of atomized fluid particles comprising relatively large fluid particles, in response to a user input specifying a low resolution cut;
(3) a combination of atomized fluid particles comprising a relatively low-density distribution of fluid particles, in response to a user input specifying a deep-penetration cut; and
(4) a combination of atomized fluid particles which comprises a relatively high-density distribution of fluid particles, in response to a user input specifying a shallow-penetration cut.

15. The apparatus according to Claim 14, wherein the user input device comprises a single input for controlling the cutting efficiency.

16. The apparatus according to Claim 15, wherein the user input device generates a relatively low-density distribution of relatively small fluid particles when the single input specifies a high cutting efficiency and a relatively high-density distribution of relatively large fluid particles when the single input specifies a low cutting efficiency.

17. The apparatus to Claim 16, wherein each of the relatively small fluid particles has a fluid-particle diameter, and
wherein a mean fluid-particle diameter of the fluid-particle diameters of the relatively small fluid particles is less than the wavelength of the electromagnetic energy focused into the volume of air adjacent to the target surface.

18. The apparatus according to Claim 16, wherein each of the relatively large fluid particles has a fluid-particle diameter, and
wherein a mean fluid-particle diameter of the fluid-particle diameters of the relatively large fluid particles is greater than the wavelength of the electromagnetic energy focused into the volume of air adjacent to the target surface.

19. The apparatus according to any one of the preceding claims, wherein the electromagnetic energy source (51) is an erbium, chromium, yttrium scandium gallium garnet (Er, Cr:YSGG) solid state laser, which generates electromagnetic energy having a wavelength of approximately 2.78 microns.

20. The apparatus according to any of Claims 1-19, wherein the fluid comprises water, and
wherein the electromagnetic energy source (51) is an erbium, chromium, yttrium, scandium, galliuin garnet (Er, Cr:YSGG) solid state laser, which generates light having a wavelength in a range of about 2.70 to about 2.80 microns.

21. The apparatus according to any of Claims 1 to 19, wherein the fluid comprises water, and
wherein the electromagnetic energy source (51) comprises one of the following:
(a) erbium, yttrium, scandium, gallium garnet (Er:YSGG) solid state laser, which generates electromagnetic energy having a wavelength in a range of about 2.70 to about 2.80 microns;
(b) erbium, yttrium, aluminium garnet (Er:YAG) solid state laser, which generates electromagnetic energy having a wavelength of about 2.94 microns;
(c) chromium, thulium, erbium, yttrium, aluminium garnet (CTE: YAG) solid state laser, which generates electromagnetic energy having a wavelength of about 2.69 microns;
(d) erbium, yttrium orthoaluminate (Er:YAL03) solid state laser, which generates electromagnetic energy having a wavelength in a range of about 2.71 to about 2.86 microns;
(e) holmium, yttrium, aluminium garnet (Ho:YAG) solid state laser, which generates electromagnetic energy having a wavelength of about 2.10 microns;
(f) quadrupled neodymium, yttrium, aluminum garnet (quadrupled Nd:YAG) solid state laser, which generates electromagnetic energy having a wavelength of about 266 nanometers;
(g) argon fluoride (ArF) excimer laser, which generates electromagnetic energy having a wavelength of about 193 nanometers;
(h) xenon chloride (XeCl) excimer laser, which generates electromagnetic energy having a wavelength of about 308 nanometers;
(i) krypton fluoride (KrF) excimer laser, which generates electromagnetic energy having a wavelength of about 248 nanometers; and
(j) carbon dioxide (CO2), which generates electromagnetic energy having a wavelength in a range of about 9.0 to about 10.6 microns.

22. The apparatus according to Claim 21, wherein the Er, Cr:YSGG solid state laser has a repetition rate greater than about 1 Hz, a pulse duration range between about 1 picosecond and about 1000 microseconds, and an energy greater than about 1 milliJoule per pulse.

23. The apparatus according to Claim 21, wherein the Er, Cr:YSGG solid state laser has a repetition rate of about 20 Hz, a pulse duration of about 140 microseconds, and an energy between about 1 and about 300 milliJoules per pulse.

24. The apparatus according to any of Claims 1-23, suitable for use with a target surface (107) which comprises a hard tissue.

25. The apparatus according to Claim 24, said hard tissue comprising one of a tooth, tooth enamel, tooth dentin, tooth cementum, bone, and cartilage.

26. The apparatus according to any of Claims 1-25, suitable for use with a target surface (107) which comprises a soft tissue.

27. The apparatus according to Claim 26, said soft tissue comprising one of skin, mucosa, gingiva, muscle, heart, liver, kidney, brain, eye, and vessels.

28. The apparatus according to any of Claims 1-27, suitable for use with a target surface (107) which comprises one of a glass material a crystalline material, and a semiconductor chip surface.

29. The apparatus according to Claim 28, wherein the electromagnetically induced mechanical cutter is arranged such that, in use, it slightly ablates the glass or crystalline material surface making it suitable for silver or another dielectric material to be adhered to the glass or crystalline material target surface to form a mirror.

30. The apparatus according to Claim 29, wherein the electromagnetically induced mechanical cutter is arranged such that, in use, it cleans and degreases the glass or crystalline material surface making it suitable for silver or other dielectric material to be adhered to the glass or crystalline material target surface to form the mirror.

31. The apparatus according to Claim 28, said semiconductor chip surface comprising an oxide layer, which is selectively removed by the electromagnetically induced mechanical cutter to thereby form windows in the oxide layer for dopant implantations into the semiconductor chip surface.

32. The apparatus according to Claim 31, said the oxide layer comprising silicon dioxide.

33. The apparatus according to Claim 22, wherein the electromagnetically induced mechanical cutter is adaptable, in use, for removing portions of the silicon dioxide layer without a need of resist, photomasks, ultraviolet light, solvents, chemical and water compounds, and acids.

34. The apparatus according to Claim 28, said target surface being a semiconductor chip surface having a layer of resist and the electromagnetically induced mechanical cutter being adapted for, in use, removing the layer of resist from the semiconductor chip surface.

35. The apparatus according to Claim 34, wherein the electronmagnetically induced mechanical cutter is adapted for slightly ablating the semiconductor chip surface to thereby remove the layer of resist and condition the semiconductor chip surface for adhesion of a conductor to the semiconductor chip surface.

36. The apparatus according to Claim 11 or Claim 12 , wherein said user control (77) comprises a specification input for specifying at least one of a cutting resolution and a penetration level for the cutting efficiency; and
said atomizer comprises means for selecting one of a plurality of fluid spray nozzles (71, 72), in response to a user specification of the cutting resolution, and means for selecting an upstream fluid pressure for the selected fluid spray nozzle in response to a user specification of the penetration level;
said atomizer being arranged to apply the upstream fluid pressure to the fluid spray nozzle, to thereby generate the user-specified combination of atomized fluid particles.

37. The apparatus according to Claim 36, wherein the specification input comprises:
a first user input for specifying a level of resolution for the cutting efficiency, the level of resolution including one of a high resolution cut and a low resolution cut; and
a second user input for specifying a level of penetration for the cutting efficiency, the level of penetration including one of a deep-penetration cut and a shallow-penetration cut.

38. The apparatus according to Claim 37, wherein the atomizer generates a combination of atomized fluid particles comprising relatively small fluid particles, in response to the first user input specifying a high resolution cut,
wherein the atomizer generates a combination of atomized fluid particles comprising relatively large fluid particles, in response to the first user input specifying a low resolution cut,
wherein the atomizer generates a combination of atomized fluid particles which comprises a relatively low-density distribution of fluid particles, in response to the second user input specifying a deep-penetration cut, and
wherein the atomizer generates a combination of atomized fluid particles which comprises a relatively high-density distribution of fluid particles, in response to the second user input specifying a shallow penetration cut.

39. Anon therapeutic method comprising the following steps:
supplying fluid to a first vicinity of the target surface; and
supplying electromagnetic energy to a second vicinity of the target surface;
the first vicinity and the second vicinity intersecting in a volume adjacent to the target surface;
said step of supplying fluid comprising generating a combination of atomized fluid particles and placing said combination of atomized fluid particles into said volume adjacent to the target surface;
said step of supplying electromagnetic energy comprising directing a peak concentration of electromagnetic energy, which has a wavelength that is substantially absorbed by said fluid, into said volume so as to be absorbed by at least a portion of said fluid particles to cause the portion of atomized fluid particles to expand, wherein disruptive mechanical forces are imparted to said target surface.

40. The method according to Claim 39, wherein the step of placing atomized fluid particles into the interaction zone includes a substep of placing atomized water particles into the interaction zone.

41. The method of Claim 40, wherein the step of focusing electromagnetic energy onto the atomized fluid particles in the interaction zone comprises a substep of focussing electromagnetic energy from an erbium, chromium, yttrium scandium gallium garnet (Er, Cr:YSGG) solid state laser, which generates electromagnetic energy having a wavelength of approximately 2.78 microns, onto the atomized water particles in the interaction zone.

42. The method of Claim 40 comprising the step providing electromagnetically induced mechanical cutting forces onto a target surface to thereby remove portions of the target surface, and
further comprising the step of inputting via a user control a user-specified combination of atomized fluid particles, the user-specified combination of atomized fluid particles corresponding to a user-specified average size, spatial distribution, and velocity of atomized fluid particles; and wherein
in said step of generating a combination of particles, said user-specified combination of atomized fluid particles is generated in response to the input from the user control; and
said portion of said atomized fluid particles is a portion of the user-specified combination of atomized fluid particles.

43. The method according to any of Claims 39-42, wherein the target surface comprises at least one of cartilage, a bone, or a tooth.

44. The method according to any of Claims 39-42, wherein the target surface comprises one of a glass material, a crystalline material, and a semiconductor chip surface.

45. The method of Claim 44 comprising mechanically removing portions of a target surface, wherein the glass or crystalline material surface is slightly ablated before silver or another dielectric material is adhered to the glass or crystalline material target surface to form a mirror.

46. The method of Claim 44 comprising mechanically removing portions of a target surface, wherein the semiconductor chip surface comprises an oxide layer, which is selectively removed to thereby form windows in the oxide layer for dopant implantations into the semiconductor chip surface.

47. The method of Claim 44 comprising mechanically removing portions of a target surface, wherein a layer of resist is removed from the semiconductor chip surface.

48. A method according to Claim 41, wherein said step of inputting comprises specifying at least one of a cutting resolution and a penetration level for the cutting efficiency; and
said step of generating comprises selecting one of a plurality of fluid spray nozzles, in response to a specification of the cutting resolution;
selecting an upstream fluid pressure for the selected fluid spray nozzle, in response to a specification of the penetration level; and
applying the upstream fluid pressure to the fluid spray nozzle, to thereby generate the user-specified combination of atomized fluid particles.

49. The method according to Claim 48, the step of specifying at least one of a cutting resolution and a penetration level for the cutting efficiency further comprising the following steps:
specifying, via a user input, one of a high resolution cut and a low resolution cut; and
specifying, via a user input, one of a deep penetration cut and a shallow-penetration cut.

50. The method of Claim 49 comprising the step of controlling a cutting efficiency of an electromagnetically induced mechanical cutter wherein the step of applying the upstream fluid pressure to the fluid spray nozzle comprises the following substeps:
generating a combination of atomized fluid particles comprising relatively small fluid particles, in response to a user input specifying a high resolution cut;
generating a combination of atomized fluid particles comprising relatively large fluid particles, in response to a user input specifying a low resolution cut;
generating a combination of atomized fluid particles which comprises a relatively low-density distribution of fluid particles, in response to a user input specifying a deep-penetration cut; and
generating a combination of atomized fluid particles which comprises a relatively high-density distribution of fluid particles, in response to a user input specifying a shallow-penetration cut.

51. The method of Claim 50 comprising the step of controlling a cutting efficiency of an electromagnetically induced mechanical cutter wherein the step of applying the upstream fluid pressure to. the fluid spray nozzle further comprises the following substeps:
generating atomized fluid particles with relatively high kinetic energies, in response to at least one of a user specification for a deep-penetration cut and a user specification for high resolution cut; and
generating atomized fluid particles with relatively low kinetic energies, in response to at least one of a user specification for a shallow-penetration cut and a user specification for low resolution cut.

52. An optical cutter for dental use, comprising apparatus according to any of Claims 1-14, and
a housing having a lower portion, an upper portion, and an interfacing portion;
the electromagnetic energy source comprising:
a first fiber optic tube a for carrying laser energy through the housing to the upper portion of the housing:
a first abutting member fitting around the first fiber optic tube at the upper portion of the housing;
a second fiber optic tube having a proximal end and a distal end;
a second abutting member surrounding the second fiber optic tube at the proximal end and contacting-the interfacing portion of the housing; and
a focusing optic positioned between the first abutting member and the second abutting member, the focussing optic focusing laser energy as the laser energy passes from the first fiber optic to the second fiber optic to thereby reduce dissipation of laser energy between the first fiber optic and the second fiber optic.

53. The optical cutter for dental use according to Claim 52, further comprising a cap having an input portion and an output portion, the cap fitting over the interfacing portion of the housing.

54. The optical cutter for dental use according to Claim 52, wherein the second fiberoptic tube comprises sapphire.

55. A medical handpiece, comprising apparatus according to any of Claims 1 to 14, and
a housing including a proximal housing end and a radiation delivery end;
said electromagnetic energy source including a first fiber guide having an output end and being disposed within the housing for conducting laser radiation from an external laser source toward the radiation delivery end of the housing;
a first ferrule comprising a ceramic or crystalline material and being adapted for securing the first fiber guide to the housing so that the output end of the first fiber guide faces the radiation delivery end of the housing;
a second fiber guide having a receiving end adapted for receiving laser radiation from the output end of the first fiber guide; and
a second ferrule comprising a ceramic or crystalline material and being adapted for securing the second fiber guide to the housing so that the receiving end of the second fiber guide faces the output end of the first fiber guide.

56. The medical handpiece as recited in Claim 55, the first ferrule detachably securing the first fiber guide to the housing so that the output end of the first fiber guide faces the radiation delivery end of the housing.

57. The medical handpiece as recited in Claim 56, the first ferrule and the second ferrule forming a gas flow path in a direction from the first fiber guide toward the receiving end of the second fiber guide.

58. The medical handpiece as recited in Claim 57, the gas flow path extending across the receiving end of the second fiber guide and across the second ferrule.

59. Apparatus according to Claim 1 to 14, further comprising an electromagnetic energy conducting apparatus, comprising:
a housing;
an inner electromagnetic energy guide disposed within the housing, the inner electromagnetic energy guide having a proximal end, a distal end, and an axis extending between the proximal end and the distal end; and
an outer electromagnetic energy guide surrounding and contacting the inner electromagnetic energy guide, the outer electromagnetic energy guide having an axis that is substantially co-linear with the axis of the inner electromagnetic energy guide.

60. The apparatus as defined in Claim 59, the outer electromagnetic energy guide comprising a ferrule.

61. The apparatus as defined in Claim 60, both the inner electromagnetic energy guide and the outer electromagnetic energy guide comprising a crystalline material.

62. The apparatus as defined in Claim 59, further comprising at least one other electromagnetic energy guide surrounding and contacting the outer electromagnetic energy guide, the other electromagnetic energy guide having an axis that is substantially co-linear with the axis of the outer electromagnetic energy guide.

63. The apparatus as defined in Claim 59, further comprising a source electromagnetic energy guide for inputting electromagnetic energy into both the inner electromagnetic energy guide and the outer electromagnetic energy guide.

64. The apparatus as defined in Claim 60, a diameter of the source electromagnetic energy guide being greater than a diameter of the inner electromagnetic energy guide.

65. The apparatus as recited in Claim 59, the inner electromagnetic energy guide and the outer electromagnetic energy guide being concentric.

66. The apparatus as recited in Claim 59, the inner electromagnetic energy guide and the outer electromagnetic energy guide sharing the same axis.

67. The apparatus as recited in Claim 66, at least one of the inner electromagnetic energy guide and the outer electromagnetic energy guide comprising sapphire.

68. Apparatus according to any of Claims 1 to 14, further comprising an optical energy conducting apparatus, comprising a bent crystalline fiber adapted for conducting optical energy therethrough, the bent crystalline fiber having a proximal end, a distal end, and an axis extending between the proximal end and the distal end, a first portion of the axis near the proximal end of the bent crystalline fiber not being parallel with a second portion of the axis near the distal end of the bent crystalline fiber.

69. The apparatus as recited in Claim 68, the bent crystalline fiber extending between a laser source and a distal end of a laser delivery device.

70. The apparatus as recited in Claim 68, an angle formed between the first portion of the axis and the second portion of the axis being approximately 90 degrees.

71. The apparatus as recited in Claim 68, further comprising a housing, the bent crystalline fiber being disposed within the housing.

72. The apparatus as recited in Claim 71, the housing being connected to a medical handpiece, and the bent crystalline fiber being cooled by a gas flow path within the medical handpiece.

73. The apparatus as recited in Claim 68, the optical energy comprising a source of coherent light.

74. The apparatus as recited in Claim 73, the coherent light having a wavelength on an order of approximately 2.5 to 3.0 microns.

75. The apparatus as recited in Claim 74, the coherent light having a wavelength within a range of about 2.78 to about 2.94 microns.

76. The apparatus as recited in Claim 75, the bent crystalline fiber comprising sapphire.

77. Apparatus according to any of Claims 1 to 14, comprising an optical energy conducting apparatus, comprising:
a housing;
a crystalline fiber disposed within the housing, the crystalline fiber being adapted for conducting optical energy therethrough, the crystalline fiber having a proximal end, a distal end, and an axis extending between the proximal end and the distal end; and
a gas flow path within the housing, the gas flow path enveloping the crystalline fiber and extending from the proximal end of the crystalline fiber to the distal end of the crystalline fiber.

78. The apparatus as recited in Claim 77, the crystalline fiber being bent.

79. A medical handpiece, comprising:
a housing including a proximal housing end and a distal housing end;
apparatus according to any one of Claims 1 to 27, disposed at the distal housing end and adapted for cutting tissue; and
a medication line having a proximal line end and a distal line end, the medication line adapted for receiving medication into the proximal line end and outputting the medication through the distal line end, which is disposed near the distal housing end.

80. The medical handpiece as recited in Claim 79, the tissue cutter comprising one of a laser and a drill.

81. The medical handpiece as recited in Claim 80, the tissue cutter further being adapted for ablating tissue.

82. A medical handpiece delivery system, comprising apparatus according to any of Claims 1 to 14, and
a housing including a proximal housing end and a radiation delivery end;
a fiber guide having an output end and being disposed within the housing for conducting laser radiation from an external laser source toward the radiation delivery end of the housing;
a ferrule adapted for securing the fiber guide to the housing so that the output end of the fiber guide faces the radiation delivery end of the housing;
an inner protective tube disposed over the fiber guide; and
an outer protective tube disposed over the inner protective tube.

83. The medical handpiece delivery system as recited in Claim 82, the fiber guide comprising a plurality of tubular jackets surrounding and affixed to the fiber guide.

84. The medical handpiece delivery system as recited in Claim 82, the ferrule comprising a proximal end and a distal end, the inner protective tube comprising a proximal end and a distal end, the outer protective tube comprising a proximal end and a distal end, and at least one of the distal end of the inner protective tube and the distal end of the outer protective tube contacting the proximal end of the ferrule.

85. The medical handpiece delivery system as recited in Claim 84, the ferrule comprising an aperture for accommodating the fiber guide, a diameter of the aperture being approximately equal to an outer diameter of the fiber guide.

86. The medical handpiece delivery system as recited in Claim 84, the proximal end of the inner protective tube and the proximal end of the outer protective tube being disposed near an SMA connector, which surrounds a proximal end of the fiber guide.

87. The medical handpiece delivery system as recited in Claim 86, the SMA connector having a proximal end and a distal end, and the fiber guide spanning a distance between the proximal end of the ferrule and the distal end of the SMA connector.
a length of at least one of the inner protective tube and the outer protective tube being less than the distance spanned by the fiber guide between the proximal end of the ferrule and the distal end of the SMA connector.

88. The medical handpiece delivery system as recited in Claim 87, a length of at least one of the inner protective tube and the outer protective tube being at least one quarter of an inch less than the distance spanned by the fiber guide between the proximal end of the ferrule and the distal end of the SMA connector.

89. The medical handpiece delivery system as recited in Claim 88, the shorter length of the at least one of the inner protective tube and the outer protective tube facilitating sliding of the at least one of the inner protective tube and the outer protective tube over the fiber guide.

90. The medical handpiece delivery system as recited in Claim 86, the SMA connector being adapted for attaching the fiber guide to an electromagnetic energy source;
the inner protective tube and the outer protective tube comprising one of a flexible plastic material and a flexible metallic material; and
the medical handpiece delivery system further comprising a metal tube disposed around the outer protective tube near the proximal end of the outer protective tube.

91. The medical handpiece delivery system as recited in Claim 90, the electromagnetic energy source comprising a laser.

92. The medical handpiece delivery system as recited in Claim 90, the medical handpiece delivery system further comprising a plastic tube surrounding and contacting the metal tube.

93. The medical handpiece delivery system as recited in Claim 92, both the inner protective tube and the outer protective tube reducing a risk of damage to the fiber guide.

94. The medical handpiece delivery system as recited in Claim 93, both the inner protective tube and the outer protective tube reducing a probability that bending of the fiber guide will result in breaking of the fiber guide.

95. The medical handpiece delivery system as recited in Claim 92, both the inner protective tube and the outer protective tube reducing a probability of the fiber guide breaking near the ferrule.

96. The medical handpiece delivery system as recited in Claim 95, the inner protective tube and the outer protective tube comprising a flexible plastic material.

97. The medical handpiece delivery system as recited in Claim 82, the inner protective tube being slidably disposed over the fiber guide.

98. The medical handpiece delivery system as recited in Claim 82, the outer protective tube being slidably disposed over the inner protective tube.

99. The medical handpiece delivery system as recited in Claim 98, further comprising at least one other protective tube slidably disposed over the outer protective tube.

100. The medical handpiece delivery system as recited in Claim 99, the inner protective tube being slidably disposed over the fiber guide.

101. The medical handpiece delivery system as recited in Claim 82, further comprising:
a second fiber guide having a receiving end adapted for receiving laser radiation from the output end of the fiber guide;
a second ferrule adapted for securing the second fiber guide to the housing so that the receiving end of the second fiber guide faces the output end of fiber guide; and
a spacer disposed between the ferrule and the second ferrule.

102. The medical handpiece delivery system as recited in Claim 101, at least one of the ferrule, the second ferrule, and the spacer comprising at least one of a ceramic and a crystalline material.

103. The medical handpiece delivery system as recited in Claim 102, the crystalline material comprising sapphire.

104. A medical handpiece, comprising apparatus according to Claims 1, 11 or 12,
a sleeve housing adapted to be held by a hand of a user and including a proximal sleeve housing end, an intermediate sleeve housing portion, a radiation delivery end, and an elongate aperture extending within the sleeve housing from the proximal sleeve housing end to the intermediate sleeve housing portion, the sleeve housing comprising a delivery fiber guide extending between the intermediate sleeve housing portion and the radiation delivery end, the delivery fiber guide having a proximal end and a distal end; and
a shaft assembly adapted for being removably disposed within the elongate aperture, the shaft assembly comprising a source fiber guide adapted for supplying electromagnetic energy to the intermediate sleeve housing portion, the source,fiber guide having a proximal end and a distal end.

105. The medical handpiece as recited in Claim 104, further comprising a collar for fitting around the proximal sleeve housing end when the shaft assembly is disposed within the sleeve housing, the collar holding the shaft assembly within the sleeve housing.

106. The medical handpiece as recited in Claim 105, the proximal sleeve housing end comprising threads for accommodating threads of the collar.

107. The medical handpiece as recited in Claim 104, the distal end of the source fiber guide being surrounded by a first ferrule, and
the proximal end of the delivery fiber guide being surrounded by a second ferrule.

108. The medical handpiece as recited in Claim 107, further comprising a third ferrule surrounding a portion of the delivery fiber guide near the radiation delivery end of the sleeve housing.

109. The medical handpiece as recited in Claim 107, the shaft assembly comprising an air supply line disposed around the source fiber guide.

110. The medical handpiece as recited in Claim 107, the fiber comprising a plurality of protective tubes disposed around the source fiber guide and an air supply line disposed around the plurality of protective tubes.

111. The medical handpiece as recited in Claim 109 the sleeve housing comprising an air line extending from the proximal sleeve housing end to the radiation delivery end; and a water line extending from the proximal sleeve housing end to the radiation delivery end.

112. The medical handpiece as recited in Claim 110 the sleeve housing further comprising a medication line extending generally form the proximal sleeve housing end to the radiation delivery end.

113. An apparatus for implementing a medical procedure comprising:
apparatus according to any one of Claims 1 to 27 for performing a medical treatment function on an operating site located inside of or connected to a human body; and
a fluid router for routing flavoured fluid in the direction of the operating site.

114. The apparatus for implementing a medical procedure according to Claim 113, wherein a flavoured fluid is selected in use to be flavoured to appeal to taste buds of the patient undergoing the surgical procedure.

115. The apparatus for implementing a medical procedure according to Claim 114, wherein the flavoured fluid is selected in use to be one which comprises one of a fruit flavour and a mint flavour.

116. The apparatus for implementing a medical procedure according to Claim 113, wherein the medical instrument comprises one of an electrocauterizer, an electromagnetic energy source, a laser, a mechanical drill, a mechanical saw, a canal finder, a syringe, and an evacuator.

117. The apparatus for implementing a medical procedure according to Claim 116, wherein the electromagnetic energy source focuses the electromagnetic energy on the operating site; and
wherein the fluid router routes flavoured fluid onto the operating site, to thereby cool the operating site as the operating site is being cut.

118. The apparatus for implementing a medical procedure according to Claim 116, wherein the fluid router is operable between a first mode where the routed fluid is flavoured, and a second mode where the routed fluid is not flavoured.

119. The apparatus for implementing a medical procedure according to Claim 115, wherein the fluid router comprises an atomizer for atomizing the flavoured fluid into atomized flavoured fluid particles before routing the flavoured fluid in the direction of the operating site.

120. The apparatus for implementing a medical procedure according to Claim 119, wherein the atomizer is arranged to route the atomized flavoured fluid particles into a volume of air above the operating site; and
wherein the electromagnetic energy source is arranged to focus electromagnetic energy into the volume of air, the electromagnetic energy having a wavelength which is substantially absorbed by the atomized flavoured fluid particles in the volume of air, the absorption of the electromagnetic energy by the atomized flavoured fluid particles causing the atomized flavoured fluid particles to explode and impart mechanical forces onto the operating site.

121. The apparatus for implementing a medical procedure according to Claim 120, wherein the fluid router is operable between a first mode where the routed fluid is flavoured, and a second mode where the routed fluid is not flavoured.

122. An apparatus for implementing a medical procedure, comprising:
apparatus according to any one of Claims 1 to 27 for performing a medical treatment function on an operating site located inside of or connected to a human body; and
a router for routing a scented medium in the direction of the operating site.

123. The apparatus for implementing a medical procedure according to Claim 122, wherein the scented medium is selected in use to be one which is scented to appeal to a sense of smell of the patient undergoing the surgical procedure.

124. The apparatus for implementing a medical procedure according to Claim 123, wherein the scented medium is selected in use to comprise one of a fruit scent, a mint scent, and an air freshener.

125. The apparatus for implementing a medical procedure according to Claim 122, wherein the medical instrument comprises one of an electrocauterizer, an electromagnetic energy source, a laser, a mechanical drill, a mechanical saw, a canal finder, a syringe, and an evacuator.

126. The apparatus for implementing a medical procedure according to Claim 123, said scented medium comprising scented air.

127. The apparatus for implementing a medical procedure according to Claim 122, wherein the electromagnetic energy source is arranged, in use, to focus the electromagnetic energy on the operating site,
said scented medium comprising scented water, and
said router being arranged to route, in use, the scented water onto the operating site, to thereby cool the operating site as the operating site is being cut by the electromagnetic energy source.

128. The apparatus for implementing a medical procedure according to Claim 123, wherein the electromagnetic energy source is arranged, in use, to focus the electromagnetic energy on the operating site, and
said scented medium comprising a scented mist, and
said router being arranged to route, in use, the scented mist onto the operating site, to thereby cool the operating site as the operating site is being cut by the electromagnetic energy source.

129. The apparatus for implementing a medical procedure according to Claim 122, wherein the router is operable between a first mode where the routed medium is scented, and a second mode where the routed medium is not scented.

130. The apparatus for implementing a medical procedure according to Claim 123, wherein the router comprises an atomizer for atomizing the scented medium before routing the scented medium in the direction of the operating site.

131. The apparatus for implementing a medical procedure according to Claim 127, wherein the atomizer is arranged to route, in use, the atomized scented medium into a volume of air above the operating site; and
wherein the electromagnetic energy source is arranged to focus, in use, electromagnetic energy into the volume of air, the electromagnetic energy having a wavelength which is substantially absorbed by portions of the atomized scented medium in the volume of air, the absorption of the electromagnetic energy by the portions of the atomized scented medium causing the portions of the atomized scented medium to explode and impart mechanical forces onto the operating site.

132. The apparatus for implementing a medical procedure according to Claim 130, wherein the router is operable between a first mode where the routed medium is scented, and a second mode where the routed medium is not scented.

133. An apparatus for implementing a medical procedure, comprising:
a cutter according to any one of Claims 1 to 27 for performing a medical treatment function on an operating site located inside of or connected to a human body; and
a fluid router for routing, in use, an ionized solution in the direction of the operating site.

134. The apparatus for implementing a medical procedure according to Claim 133, said ionized solution comprising a biocompatible saline solution.

135. The apparatus for implementing a medical procedure according to Claim 133, wherein the medical instrument comprises one of an electrocauterizer, an electromagnetic energy source, a laser, a mechanical drill, a mechanical saw, a canal finder, a syringe, and an evacuator.

136. The apparatus for implementing a medical procedure according to Claim 135, wherein the electromagnetic energy source focuses the electromagnetic energy onto the operating site; and
wherein the fluid router routes the ionized solution onto the operating site, to thereby cool the operating site as the operating site is being cut.

137. The apparatus for implementing a medical procedure according to Claim 135, wherein the fluid router comprises an atomizer for atomizing the ionized solution into atomized particles before routing the atomized particles in the direction of the operating site.

138. The apparatus for implementing a medical procedure according to Claim 137, wherein the atomizer routes the atomized particles into a volume of air above the operating site; and
wherein the electromagnetic energy source focuses electromagnetic energy into the volume of air, the electromagnetic energy having a wavelength which is substantially absorbed by the atomized particles in the volume of air, the absorption of the electromagnetic energy by the atomized particles causing the atomized particles to explode and impart mechanical forces onto the operating site.

139. The apparatus for implementing a medical procedure according to Claim 137, wherein the fluid router is operable between a first mode where solution routed therefrom is ionized, and a second mode where the solution routed therefrom is not ionized.

140. The apparatus for implementing a medical procedure according to Claim 139 wherein the fluid router is operable in a plurality of modes between the first mode and the second mode to thereby continuously control a cutting power of the electromagnetic energy source.

141. The apparatus for implementing a medical procedure according to Claim 140, wherein the cutting power of the electromagnetic energy source decreases as the fluid from the fluid router becomes more ionized.

142. The apparatus for implementing a medical procedure according to Claim 141, wherein a strength of the electromagnetic energy source is adjustable to control the cutting power of the electromagnetic energy source.

143. The apparatus for implementing a medical procedure according to Claim 142, wherein the strength of the electromagnetic energy. source can be increased when the fluid from the fluid router becomes more ionized, to thereby maintain a constant cutting power of the electromagnetic energy source; and
wherein the strength of the electromagnetic energy source can be decreased when the fluid from the fluid router becomes less ionized, to thereby maintain a constant cutting power of the electromagnetic energy source.

144. Apparatus according to anyone of Claims 1 to 27, wherein
said atomizer is arranged to place, in use, pigmented atomized fluid particles into said predefined volume.

145. Apparatus according to any one of Claims 1 to 27, further comprising:
a fluid source for delivering, in use, pigmented fluid to an approximate vicinity of the target surface, the pigmented fluid having a color which is substantially unabsorbed by the electromagnetic energy.

146. Apparatus according to any one of Claims 1 to 27 further comprising a fluid source for delivering fluid to an approximate vicinity of the target surface, the fluid having a color which is substantially unabsorbed by the electromagnetic energy.

147. Apparatus according to any one of Claims 1 to 27 further comprising supplying means connected to the atomizer, for selectively supplying a pigmentation to the atomized fluid particles.

148. The apparatus according to Claim 147, wherein the pigmentation is selectively supplied to the atomized fluid particles according to a user input.

149. The apparatus according to Claim 147, wherein the electromagnetic energy has a wavelength that is substantially absorbed by the atomized fluid particles when the pigmentation is supplied to the atomized fluid particles.

150. The apparatus according to Claim 147, wherein the electromagnetic energy has a wavelength that is substantially absorbed by the atomized fluid particles when the pigmentation is not supplied to the atomized fluid particles.

151. The apparatus according to Claim 147, wherein the atomizer comprises a water source line and an air source line; and
wherein the pigmentation is selectively supplied to the atomized fluid particles via one of the air source line and the water source line.

152. An apparatus for implementing a medical procedure, comprising:
apparatus according to any one of Claims 1 to 27 for performing a medical treatment function on an operating site located inside of or connected to a human body; and
a fluid router for routing a fluid, which has a measurable fluid parameter different than a corresponding measurable fluid parameter of water, in the direction of the operating site.

153. The apparatus according to Claim 152 wherein the measurable fluid parameter comprises one of a density, a specific gravity a pH level, a viscosity, and a temperature.

154. An apparatus for implementing a medical procedure comprising:
a cutter according to any one of Claims 1 to 27 for performing a medical treatment function on an operating site located inside of or connected to a human body; and a router for routing a medication medium in the direction of the operating site.

155. The apparatus for implementing a medical procedure according to Claim 154 wherein, in use, the medication medium may comprise one of an antibiotic, iodine, a steroid, an anaesthetic, an anti-inflammatory, a disinfectant, adrenaline, epinephrine, an antiseptic, and a stringent.

156. The apparatus for implementing a medical procedure according to Claim 154 wherein, in use, the medication medium may comprise one of vitamins, herbs, and minerals.

157. The apparatus for implementing a medical procedure according to Claim 154, wherein the medical instrument comprises one of an electrocauterizer, an electromagnetic energy source, a laser, a mechanical drill, a mechanical saw, a canal finder, a syringe, and an evacuator.

158. The apparatus for implementing a medical procedure according to Claim 157 wherein the electromagnetic energy source is arranged to focus, in use, the electromagnetic energy on the operating site; and
wherein the router, in use, routes the medication medium onto the operating site to thereby cool and medicate the operating site as the operating site is being cut.

159. The apparatus for implementing a medical procedure according to Claim 158, wherein the router is operable between a first mode where the routed medium is medicated, and a second mode where the routed medium is not medicated.

160. The apparatus for implementing a medical procedure according to Claim 159, wherein the router comprises an atomizer for atomizing the medication medium into atomized particles before routing the medication medium in the direction of the operating site.

161. The apparatus for implementing a medical procedure according to Claim 160, wherein the atomizer is arranged, in use, to route, in use, the atomized particles into a volume of air above the operating site; and
wherein the electromagnetic energy source is arranged, in use, to focus electromagnetic energy into the volume of air, the electromagnetic energy having a wavelength which is substantially absorbed by the atomized particles in the volume of air, the absorption of the electromagnetic energy by the atomized particles causing the atomized particles to explode and impart mechanical forces onto the operating site.

162. The apparatus for implementing a medical procedure according to Claim 160, wherein the fluid router is operable between a first mode where the fluid routed therefrom is medicated, and a second mode where the fluid routed therefrom is not medicated.

163. An apparatus for killing bacteria in or on both a patient's mouth and a dental instrument within a vicinity of the patient's mouth, comprising:
a surgical tool comprising an apparatus according to Claim 1 for cutting or drilling material within a patient's mouth; and
a fluid router for, routing disinfectant into the patient's mouth, a portion of the routed disinfectant becoming airborne and settling onto the dental instrument to thereby disinfect the dental instrument.

164. A drilling and conditioning apparatus, comprising:
apparatus according to any one of Claims 1 to 27 for performing a drilling operation on a tooth within a patient's mouth; and
a fluid router for routing conditioned fluid in the direction of the tooth being operated upon, the conditioned fluid having a measurable property that is different than a corresponding measurable property of water.

165. The drilling and conditioning apparatus according to Claim 164, said measurable property comprising at least one of a flavour detectable by taste buds of the patient, a scent detectable by olfactory senses of the patient, a salinity a pigment, a medication, and a disinfectant.

166. The drilling and conditioning apparatus according to claim 165, said medication comprising at least one of an antibiotic, a steroid, an anaesthetic an anti-inflammatory, a disinfectant, adrenaline, epinephrine, an antiseptic, and a stringent.

167. The drilling and conditioning apparatus according to Claim 165, wherein the medication comprises one of vitamins, herbs, and minerals.

168. An apparatus for implementing a medical procedure, comprising:
apparatus according to any one of Claims 1 to 27 for performing a medical treatment function on an operating site located inside of or connected to a human body; and
a fluid router for selectively routing pigmented fluid in the direction of the tissue.

169. The apparatus for implementing a medical procedure according to Claim 168, wherein the medical instrument comprises one of an electrocauterizer, an electromagnetic energy source, a mechanical drill, a mechanical saw, a canal finder, a syringe, and an evacuator.

170. The apparatus for implementing a medical procedure according to Claim 169, wherein the fluid router is operable between a first mode where the routed fluid is pigmented, and a second mode where the routed fluid is not pigmented.

171. The apparatus for implementing a medical procedure according to Claim 170, wherein a wavelength of the electromagnetic energy source is substantially absorbed by the pigmented fluid.

## Patentansprüche

1. Apparatur, umfassend
eine Fluidausgabeeinrichtung, die beim Gebrauch ein Fluid aufweist und so konstruiert ist, dass sie das Fluid in eine erste Nachbarschaft bezüglich der Fluidausgabeeinrichtung lenkt, und
eine Quelle für elektromagnetische Energie zur Bereitstellung elektromagnetischer Energie in einer zweiten Nachbarschaft,
wobei die erste Nachbarschaft und die zweite Nachbarschaft an bestimmten Orten bezüglich der Fluidausgabeeinrichtung lokalisiert sind,
**dadurch gekennzeichnet, dass** die erste Nachbarschaft und die zweite Nachbarschaft sich in einem Volumen (59) bezüglich der Fluidausgabeeinrichtung überschneiden, wobei die Fluidausgabeeinrichtung ferner einen Zerstäuber (63,65,71) zur Erzeugung einer Kombination aus zerstäubten Fluidteilchen und zur Einbringung der Kombination der zerstäubten Fluidteilchen in das Volumen aufweist, wobei die Quelle für elektromagnetische Energie eine spezifisch konfigurierte Quelle (51) für elektromagnetische Energie umfasst, die beim Gebrauch der Apparatur so angeordnet ist, dass sie elektromagnetische Energie einer Wellenlänge bereit stellt, die im wesentlichen von der Kombination der zerstäubten Fluidteilchen absorbiert wird, und dass sie eine Spitzenkonzentration der elektromagnetischen Energie in das Volumen lenkt, wobei die Kombination der zerstäubten Fluidteilchen eine Größe hat und auf eine Weise verteilt wird, dass, wenn sie in das Volumen eingebracht und mit der fokussierten elektromagnetischen Energie bestrahlt wird, die elektromagnetische Energie im wesentlichen von wenigstens einem Teil der Kombination der zerstäubten Fluidteilchen absorbiert wird, wobei zerstörende mechanische Kräfte auf eine Zieloberfläche (107) ausgeübt werden.

2. Apparatur nach Anspruch 1, wobei die Kombination der Fluidteilchen eine Kombination von Fluidteilchen umfasst, die bezüglich ihrer Größe ausgewählt und auf solche Weise verteilt sind, dass sich die Fluidteilchen, wenn sie in das Volumen eingebracht und mit der Quelle der elektromagnetischen Energie bestrahlt werden, ausdehnen.

3. Apparatur nach Anspruch 1 oder Anspruch 2, wobei die Ausgabeeinrichtung einen Zerstäuber umfasst und die Kombination der Fluidteilchen eine Kombination aus zerstäubten Fluidteilchen mit Durchmessern umfasst, die eng um einen Mittelwert herum verteilt sind.

4. Apparatur gemäß einem beliebigen der vorangehenden Ansprüche, wobei, wenn die Apparatur bei ihrem Gebrauch so angeordnet ist, dass das Volumen oberhalb der Zieloberfläche angeordnet ist, sich der Anteil der Fluidteilchen ausdehnt und zerstörende Kräfte auf die Zieloberfläche ausübt.

5. Apparatur nach einem beliebigen der vorangehenden Ansprüche, wobei die elektromagnetische Energie vom Anteil der Fluidteilchen stark absorbiert wird.

6. Apparatur nach einem beliebigen der vorangehenden Ansprüche, wobei, wenn die Apparatur bei ihrem Gebrauch so angeordnet ist, dass das Volumen oberhalb einer Zieloberfläche angeordnet ist, die Apparatur schneidende und abtragende mechanische Zerstörkräfte auf die Zieloberfläche ausübt.

7. Apparatur gemäß einem beliebigen der vorangehenden Ansprüche, wobei die Ausgabeeinrichtung mit einer Luft-Zuführleitung und einer Wasser-Zuführleitung versorgt wird.

8. Apparatur gemäß einem beliebigen der vorangehenden Ansprüche, wobei die Energie über eine Faseroptik (23) zugeführt wird, und
wobei die Fluidteilchen mit der Faseroptik in Kontakt treten, um die Faseroptik dadurch zu kühlen und zu säubern.

9. Apparatur nach Anspruch 8, wobei die Fluidteilchen mit der Faseroptik in Kontakt treten, um dadurch teilchenförmige Verunreinigungen von der Faseroptik zu entfernen.

10. Apparatur nach Anspruch 8 oder Anspruch 9, wobei die Faseroptik einen Saphir aufweist.

11. Apparatur gemäß Anspruch 1, ferner umfassend
eine Benutzersteuerung für das Zuführen einer benutzerspezifizierten Kombination zerstäubter Fluidteilchen, wobei die Kombination der zerstäubten Fluidteilchen einer benutzerspezifizierten Durchschnittsgröße, räumlichen Verteilung und Geschwindigkeit der zerstäubten Teilchen entspricht, wobei der Zerstäuber auf die Benutzersteuerung anspricht.

12. Apparatur gemäß einem beliebigen der vorangehenden Ansprüche, wobei die Absorption der elektromagnetischen Energie durch den Anteil der Fluidteilchen im Volumen dazu führt, dass der Anteil der Fluidteilchen die zerstörenden mechanischen Kräfte auf die Zieloberfläche ausübt.

13. Apparatur nach Anspruch 12, wobei
die zerstörenden mechanischen Kräfte schneidende und abtragende mechanische Kräfte umfassen, und,
wenn die Apparatur beim Gebrauch so angeordnet ist, dass das Volumen oberhalb der Zieloberfläche gelegen ist, der Anteil der Fluidteilchen dazu gebracht wird, die schneidenden und abtragenden mechanischen Kräfte auf die Zieloberfläche auszuüben.

14. Apparatur gemäß Anspruch 11 oder Anspruch 12, wobei die Benutzersteuerung eine Benutzereingabevorrichtung (77) für die Festlegung eines Schneidens mit entweder hoher Auflösung oder niedriger Auflösung und für die Festlegung eines Schneidens mit entweder tiefer Eindringung oder flacher Eindringung umfasst, und
der Zerstäuber auf die Benutzereingabevorrichtung anspricht, um zu erzeugen
(1) eine Kombination aus zerstäubten Fluidteilchen, die relativ kleine Fluidteilchen umfasst, als Reaktion auf eine Benutzereingabe, die ein Schneiden mit hoher Auflösung vorgibt,
(2) eine Kombination aus zerstäubten Fluidteilchen, die relativ große Fluidteilchen umfasst, als Reaktion auf eine Benutzereingabe, die ein Schneiden mit geringer Auflösung vorgibt,
(3) eine Kombination aus zerstäubten Fluidteilchen, die eine Verteilung der Fluidteilchen mit relativ geringer Dichte umfasst, als Reaktion auf eine Benutzereingabe, die ein Schneiden mit hoher Eindringtiefe vorgibt, und
(4) eine Kombination aus zerstäubten Fluidteilchen, die eine Verteilung der Fluidteilchen mit relativ hoher Dichte umfasst, als Reaktion auf eine Benutzereingabe, die ein Schneiden mit flacher Eindringtiefe vorgibt.

15. Apparatur gemäß Anspruch 14, wobei die Benutzereingabevorrichtung eine einzige Eingabe für die Steuerung der Schneideeffizienz umfasst.

16. Apparatur gemäß Anspruch 15, wobei die Benutzereingabevorrichtung eine Verteilung relativ kleiner Fluidteilchen mit relativ geringer Dichte erzeugt, wenn die einzige Eingabe eine hohe Schneideeffizienz vorgibt, und eine Verteilung relativ großer Fluidteilchen mit relativ großer Dichte, wenn die einzige Eingabe eine geringe Schneideeffizienz vorgibt.

17. Apparatur nach Anspruch 16, wobei jedes der relativ kleinen Fluidteilchen einen Fluidteilchendurchmesser aufweist und
wobei ein mittlerer Fluidteilchendurchmesser der Fluidteilchendurchmesser der relativ kleinen Fluidteilchen kleiner ist als die Wellenlänge der elektromagnetischen Energie, die in das Volumen der Luft, das an die Zieloberfläche angrenzt, fokussiert ist.

18. Apparatur gemäß Anspruch 16, wobei jedes der relativ großen Fluidteilchen einen Fluidteilchendurchmesser aufweist und
wobei ein mittlerer Fluidteilchendurchmesser der Fluidteilchendurchmesser der relativ großen Fluidteilchen größer ist als die Wellenlänge der elektromagnetischen Energie, die in das Volumen der Luft, das an die Zieloberfläche angrenzt, fokussiert ist.

19. Apparatur gemäß einem beliebigen der vorangehenden Ansprüche, wobei die Quelle (51) der elektromagnetischen Energie ein Erbium-Chrom-Yttrium-Scandium-Gallium-Granat (Er,Cr:YSGG)-Feststofflaser ist, der elektromagnetische Energie mit einer Wellenlänge von ungefähr 2,78 Mikrometer erzeugt.

20. Apparatur gemäß einem beliebigen der Ansprüche 1-19, wobei das Fluid Wasser umfasst, und
wobei die Quelle (51) der elektromagnetischen Energie ein Erbium-Chrom-Yttrium-Scandium-Gallium-Granat (Er,Cr:YSGG)-Feststofflaser ist, der Licht mit einer Wellenlänge im Bereich von ungefähr 2,70 bis ungefähr 2,80 Mikrometer erzeugt.

21. Apparatur gemäß einem beliebigen der Ansprüche 1-19, wobei das Fluid Wasser umfasst, und
wobei die Quelle (51) der elektromagnetischen Energie eines der Folgenden umfasst:
(a) einen Erbium-Yttrium-Scandium-Gallium-Granat (Er:YSGG)-Feststofflaser, der elektromagnetische Energie mit einer Wellenlänge im Bereich von ungefähr 2,70 bis ungefähr 2,80 Mikrometer erzeugt,
(b) einen Erbium-Yttrium-Aluminium-Granat (Er:YAG)-Feststofflaser, der elektromagnetische Energie mit einer Wellenlänge von ungefähr 2,94 Mikrometer erzeugt,
(c) einen Chrom-Thulium-Erbium-Yttrium-Aluminium-Granat (CTE:YAG)-Feststofflaser, der elektromagnetische Energie mit einer Wellenlänge von ungefähr 2,69 Mikrometer erzeugt,
(d) einen Erbium-Yttrium-Orthoaluminat (Er:YAL03)-Feststofflaser, der elektromagnetische Energie mit einer Wellenlänge im Bereich von ungefähr 2,71 bis ungefähr 2,86 Mikrometer erzeugt,
(e) einen Holmium-Yttrium-Aluminium-Granat (Ho:YAG)-Feststofflaser, der elektromagnetische Energie mit einer Wellenlänge im Bereich von ungefähr 2,10 Mikrometer erzeugt,
(f) einen Quadrupol-Neodym-Yttrium-Aluminium-Granat (Quadrupol-Nd:YAG)-Feststofflaser, der elektromagnetische Energie mit einer Wellenlänge von ungefähr 266 Nanometer erzeugt,
(g) einen Argonfluorid (ArF)-Excimer-Laser, der elektromagnetische Energie mit einer Wellenlänge von ungefähr 193 Nanometer erzeugt,
(h) einen Xenonchlorid (XeCl)-Excimer-Laser, der elektromagnetische Energie mit einer Wellenlänge von ungefähr 308 Nanometer erzeugt,
(i) einen Kryptonfluorid (KrF)-Excimer-Laser, der elektromagnetische Energie mit einer Wellenlänge von ungefähr 248 Nanometer erzeugt,
(j) einen Kohlendioxid (CO₂)-Laser, der elektromagnetische Energie mit einer Wellenlänge im Bereich von ungefähr 9,0 bis ungefähr 10,6 Mikrometer erzeugt.

22. Apparatur gemäß Anspruch 21, wobei der Er,Cr:YSGG-Feststofflaser eine Repetitionsfrequenz von über ungefähr 1 Hz hat, eine Pulsdauer im Bereich zwischen ungefähr 1 Picosekunde und ungefähr 1000 Mikrosekunden und eine Energie von über ungefähr 1 Millijoule pro Puls.

23. Apparatur gemäß Anspruch 21, wobei der Er,Cr:YSGG-Feststofflaser eine Repetitionsfrequenz von ungefähr 20 Hz hat, eine Pulsdauer von ungefähr 140 Mikrosekunden und eine Energie zwischen ungefähr 1 und ungefähr 300 Millijoule pro Puls.

24. Apparatur gemäß einem beliebigen der Ansprüche 1-23, die für eine Verwendung mit einer Zieloberfläche (107) geeignet ist, die ein hartes Gewebe umfasst.

25. Apparatur gemäß Anspruch 24, wobei das harte Gewebe eines aus der Gruppe umfasst, die besteht aus einem Zahn, Zahnschmelz, Zahndentin, Zahnzement, Knochen und Knorpel.

26. Apparatur gemäß einem beliebigen der Ansprüche 1-25, die für eine Verwendung mit einer Zieloberfläche (107) geeignet ist, die ein weiches Gewebe umfasst.

27. Apparatur gemäß Anspruch 26, wobei das weiche Gewebe eines aus der Gruppe umfasst, die besteht aus Haut, Schleimhaut, Gingiva, Muskel, Herz, Leber, Niere, Gehirn, Auge und Blutgefäßen.

28. Apparatur gemäß einem beliebigen der Ansprüche 1-27, die für eine Verwendung mit einer Zieloberfläche (107) geeignet ist, die eine aus der Gruppe umfasst, die aus der Oberfläche eines Glasmaterials, eines kristallinen Materials und eines Halbleiterchips besteht.

29. Apparatur gemäß Anspruch 28, wobei das elektromagnetisch induzierte mechanische Schneidegerät so angeordnet ist, dass es bei seinem Gebrauch die Oberfläche des Glasmaterials oder des kristallinen Materials geringfügig abträgt, was es Silber oder einem anderen dielektrischen Material ermöglicht, an der Zieloberfläche des Glasmaterials oder des kristallinen Materials zu haften, so dass ein Spiegel gebildet wird.

30. Apparatur gemäß Anspruch 29, wobei das elektromagnetisch induzierte mechanische Schneidegerät so angeordnet ist, dass es bei seinem Gebrauch die Oberfläche des Glasmaterials oder des kristallinen Materials säubert und entfettet, was es Silber oder einem anderen dielektrischen Material ermöglicht, an der Zieloberfläche des Glasmaterials oder des kristallinen Materials zu haften, so dass der Spiegel gebildet wird.

31. Apparatur gemäß Anspruch 28, wobei die Oberfläche des Halbleiterchips eine Oxidschicht umfasst, die selektiv vom elektromagnetisch induzierten mechanischen Schneidegerät entfernt wird, wodurch Fenster in der Oxidschicht für Implantierungen von Dotierungsmitteln in die Oberfläche des Halbleiterchips gebildet werden.

32. Apparatur gemäß Anspruch 31, wobei die Oxidschicht Siliziumdioxid umfasst.

33. Apparatur gemäß Anspruch 22, wobei das elektromagnetisch induzierte mechanische Schneidegerät bei seinem Gebrauch für die Entfernung von Teilen der Siliziumdioxidschicht auslegbar ist, ohne das ein Resist, Photomasken, ultraviolettes Licht, Lösemittel, chemische und wässrige Verbindungen und Säuren benötigt werden.

34. Apparatur gemäß Anspruch 28, wobei die Zieloberfläche eine Oberfläche eines Halbleiterchips ist, die eine Schicht aus einem Resist aufweist, und wobei das elektromagnetisch induzierte mechanische Schneidegerät bei seinem Gebrauch für die Entfernung der Schicht des Resists von der Oberfläche des Halbleiterchips ausgelegt ist.

35. Apparatur gemäß Anspruch 34, wobei das elektromagnetisch induzierte mechanische Schneidegerät bei seinem Gebrauch für die geringfügige Abtragung der Oberfläche des Halbleiterchips ausgelegt ist, um somit die Schicht des Resists zu entfernen und die Oberfläche des Halbleiterchips für die Adhäsion eines Leiters an die Oberfläche des Halbleiterchips zu konditionieren.

36. Apparatur gemäß Anspruch 11 oder Anspruch 12, wobei die Benutzersteuerung (77) eine Spezifikationseingabe für das Vorgeben von wenigstens einem der Schneideauflösung und der Eindringtiefe für die Schneideeffizienz umfasst, und
der Zerstäuber eine Einrichtung für die Auswahl einer aus einer Vielzahl von Fluid-Sprühdüsen (71,72) als Reaktion auf eine Benutzervorgabe der Schneideauflösung und eine Einrichtung für die Auswahl eines nach oben gerichteten Fluiddrucks für die gewählte Fluid-Sprühdüse als Reaktion auf eine Benutzervorgabe der Eindringtiefe aufweist,
wobei der Zerstäuber so angeordnet ist, dass der nach oben gerichtete Fluiddruck auf die Fluid-Sprühdüse einwirkt, wodurch die benutzerspezifizierte Kombination aus zerstäubten Fluidteilchen erzeugt wird.

37. Apparatur gemäß Anspruch 36, wobei die Spezifikationseingabe umfasst
eine erste Benutzereingabe für die Vorgabe der Schneideeffizienz, des Ausmaßes der Auflösung, und zwar entweder eines Schneidens mit hoher Auflösung oder eines Schneidens mit geringer Auflösung, und
eine zweite Benutzereingabe für die Vorgabe der Eindringtiefe für die Schneideeffizienz, wobei die Eindringtiefe entweder ein Schneiden mit hoher Eindringtiefe oder ein Schneiden mit flacher Eindringtiefe einschließt.

38. Apparatur gemäß Anspruch 37, wobei der Zerstäuber eine Kombination zerstäubter Fluidteilchen erzeugt, die relativ kleine Fluidteilchen umfassen, als Reaktion auf die erste Benutzereingabe, die ein Schneiden mit hoher Eindringtiefe vorgibt,
wobei der Zerstäuber eine Kombination zerstäubter Fluidteilchen erzeugt, die relativ große Fluidteilchen umfassen, als Reaktion auf die erste Benutzereingabe, die ein Schneiden mit niedriger Auflösung vorgibt,
wobei der Zerstäuber eine Kombination zerstäubter Fluidteilchen erzeugt, die eine Verteilung relativ niedriger Dichte von Fluidteilchen aufweist, als Reaktion auf die zweite Benutzereingabe, die ein Schneiden mit hoher Eindringtiefe vorgibt,und
wobei der Zerstäuber eine Kombination zerstäubter Fluidteilchen erzeugt, die eine Verteilung relativ hoher Dichte von Fluidteilchen aufweist, als Reaktion auf die zweite Benutzereingabe, die ein Schneiden mit flacher Eindringtiefe vorgibt.

39. Nicht-therapeutisches Verfahren, das die folgenden Schritte umfasst:
Zuführen von Fluid in eine erste Nachbarschaft der Zieloberfläche, und
Zuführen elektromagnetischer Energie in eine zweite Nachbarschaft der Zieloberfläche,
wobei sich die erste Nachbarschaft und die zweite Nachbarschaft in einem Volumen, dass an die Zieloberfläche angrenzt, überschneiden,
wobei der Schritt des Zuführens von Fluid eine Kombination aus zerstäubten Fluidteilchen erzeugt, und Einbringen der Kombination von zerstäubten Fluidteilchen in das Volumen, dass an die Zieloberfläche angrenzt,
wobei der Schritt des Zuführens elektromagnetischer Energie das Lenken einer Spitzenkonzentration der elektromagnetischen Energie, die eine Wellenlänge hat, die von den Fluidteilchen im wesentlichen absorbiert wird, in das Volumen umfasst, so dass sie von wenigstens einem Teil der Fluidteilchen absorbiert wird, um den Teil der zerstäubten Fluidteilchen zum Expandieren zu bringen, damit die zerstörenden mechanischen Kräfte auf die Zieloberfläche ausgeübt werden.

40. Verfahren gemäß Anspruch 39, wobei der Schritt des Einbringens der zerstäubten Fluidteilchen in die Zone der Wechselwirkung einen Teilschritt des Einbringens zerstäubter Wasserteilchen in die Zone der Wechselwirkung einschließt.

41. Verfahren gemäß Anspruch 40, wobei der Schritt des Fokussierens der elektromagnetischen Energie auf die zerstäubten Fluidteilchen in der Zone der Wechselwirkung einen Teilschritt aus dem Fokussieren elektromagnetischer Energie eines Erbium-Chrom-Yttrium-Scandium-Gallium-Granat (Er,Cr:YSGG)-Feststofflasers, der elektromagnetische Energie mit einer Wellenlänge von ungefähr 2,78 Mikrometer erzeugt, auf die zerstäubten Wasserteilchen in der Zone der Wechselwirkung einschließt.

42. Verfahren nach Anspruch 40, umfassend den Schritt der Bereitstellung induzierter mechanischer Schneidekräfte auf eine Zieloberfläche, um dadurch Teile der Zieloberfläche zu entfernen, und
ferner umfassend den Schritt der Eingabe über eine Benutzersteuerung einer benutzerspezifizierten Kombination zerstäubter Fluidteilchen, wobei die benutzerspezifizierte Kombination der zerstäubten Fluidteilchen einer benutzerspezifizierten Durchschnittsgröße, räumlichen Verteilung und Geschwindigkeit der zerstäubten Fluidteilchen entspricht, und wobei
beim Schritt der Erzeugung von Teilchen die benutzerspezifizierte Kombination zerstäubter Fluidteilchen als Reaktion auf die Eingabe von der Benutzersteuerung erzeugt wird, und
der Teil der zerstäubten Fluidteilchen ein Teil der benutzerspezifizierten Kombination der zerstäubten Fluidteilchen ist.

43. Verfahren gemäß einem beliebigen der Ansprüche 39-42, wobei die Zieloberfläche wenigstens entweder Knorpel, einen Knochen oder einen Zahn umfasst.

44. Verfahren gemäß einem beliebigen der Ansprüche 39-42, wobei die Zieloberfläche entweder die Oberfläche eines Glasmaterials, eines kristallinen Materials oder eines Halbleiterchips umfasst.

45. Verfahren nach Anspruch 44, umfassend das mechanische Entfernen von Teilen einer Zieloberfläche, wobei die Oberfläche des Glasmaterials oder des kristallinen Materials geringfügig abgetragen wird, ehe Silber oder ein anderes dielektrisches Material auf der Zieloberfläche des Glasmaterials oder des kristallinen Materials angebracht wird, um einen Spiegel zu bilden.

46. Verfahren nach Anspruch 44, umfassend das mechanische Entfernen von Teilen einer Zieloberfläche, wobei die Oberfläche des Halbleiterchips eine Oxidschicht umfasst, die selektiv entfernt wird, wodurch Fenster in der Oxidschicht für Implantierungen von Dotierungsmitteln in die Oberfläche des Halbleiterchips gebildet werden.

47. Verfahren nach Anspruch 44, umfassend das mechanische Entfernen von Teilen einer Zieloberfläche, wobei eine Resistschicht von der Oberfläche des Halbleiterchips entfernt wird.

48. Verfahren gemäß Anspruch 44, wobei der Eingabeschritt das Vorgeben von wenigstens einem der Schneideauflösung und einer Eindringtiefe für die Schneideeffizienz umfasst, und
der Erzeugungsschritt das Auswählen einer Vielzahl von Fluid-Sprühdüsen als Reaktion auf eine Vorgabe der Schneideauflösung umfasst,
das Auswählen eines nach oben gerichteten Fluiddrucks für die gewählte Fluid-Sprühdüse als Reaktion auf eine Vorgabe der Eindringtiefe, und
das Anlegen des nach oben gerichteten Fluiddrucks an die Fluid-Sprühdüse, um dadurch die benutzerspezifizierte Kombination aus zerstäubten Fluidteilchen zu erzeugen.

49. Verfahren gemäß Anspruch 48, wobei der Schritt des Vorgebens von wenigstens entweder einer Schneideauflösung oder einer Endringtiefe für die Schneideeffizienz außerdem die folgenden Schritte umfasst:
Vorgeben, über eine Benutzereingabe, eines Schneidens mit hoher Auflösung oder eines Schneidens mit geringer Auflösung, und
Vorgeben, über eine Benutzereingabe, eines Schneidens mit hoher Eindringtiefe oder eines Schneidens mit flacher Eindringtiefe.

50. Verfahren nach Anspruch 49, umfassend den Schritt des Steuerns der Schneideeffizienz eines elektromagnetisch induzierten mechanischen Schneidegeräts, wobei der Schritt des Anlegens eines aufwärts gerichteten Fluiddrucks an die Fluid-Sprühdüse die folgenden Teilschritte umfasst:
Erzeugen einer Kombination zerstäubter Fluidteilchen, die relativ kleine Fluidteilchen umfassen, als Reaktion auf eine Benutzereingabe, die ein Schneiden mit hoher Auflösung vorgibt,
Erzeugen einer Kombination zerstäubter Fluidteilchen, die relativ große Fluidteilchen umfassen, als Reaktion auf eine Benutzereingabe, die ein Schneiden mit niedriger Auflösung vorgibt,
Erzeugen einer Kombination zerstäubter Fluidteilchen, die eine Verteilung relativ niedriger Dichte von Fluidteilchen aufweist, als Reaktion auf eine Benutzereingabe, die ein Schneiden mit hoher Eindringtiefe vorgibt, und
Erzeugen einer Kombination zerstäubter Fluidteilchen, die eine Verteilung relativ hoher Dichte von Fluidteilchen aufweist, als Reaktion auf eine Benutzereingabe, die ein Schneiden mit flacher Eindringtiefe vorgibt.

51. Verfahren nach Anspruch 50, umfassend den Schritt des Steuerns der Schneideeffizienz eines elektromagnetisch induzierten mechanischen Schneidegeräts, wobei der Schritt des Anlegens eines aufwärts gerichteten Fluiddrucks an die Fluid-Sprühdüse ferner die folgenden Teilschritte umfasst:
Erzeugen zerstäubter Fluidteilchen mit relativ hohen kinetischen Energien als Reaktion auf wenigstens eines aus einer Benutzervorgabe für ein Schneiden mit hoher Eindringtiefe und einer Benutzervorgabe für ein Schneiden mit hoher Auflösung, und
Erzeugen zerstäubter Fluidteilchen mit relativ niedrigen kinetischen Energien als Reaktion auf wenigstens eines aus einer Benutzervorgabe für ein Schneiden mit flacher Eindringtiefe und einer Benutzervorgabe für ein Schneiden mit niedriger Auflösung.

52. Optisches Schneidegerät für die zahnärztliche Verwendung, umfassend eine Apparatur gemäß einem beliebigen der Ansprüche 1-14, und
ein Gehäuse mit einem unteren Teil, einem oberen Teil und einem Koppelteil,
wobei die Quelle für die elektromagnetische Energie umfasst:
eine erste Faseroptik-Röhre für das Leiten der Laserenergie durch das Gehäuse in den oberen Teil des Gehäuses,
ein erstes angrenzendes Bauteil, das die erste Faseroptik-Röhre am oberen Teil des Gehäuses umschließt,
eine zweite Faseroptik-Röhre, die ein proximales Ende und ein distales Ende besitzt,
ein zweites angrenzendes Bauteil, das die zweite Faseroptik-Röhre am proximalen Ende umschließt und sich in Kontakt mit dem Koppelteil des Gehäuses befindet, und
eine Fokussierungsoptik, die zwischen dem ersten angrenzenden Bauteil und dem zweiten angrenzenden Bauteil angeordnet ist, wobei die Fokussierungsoptik Laserenergie fokussiert, wenn die Laserenergie aus der ersten Faseroptik in die zweite Faseroptik übertritt, um dadurch die Zerstreuung der Laserenergie zwischen der ersten Faseroptik und der zweiten Faseroptik zu vermindern.

53. Optisches Schneidegerät für die zahnärztliche Verwendung gemäß Anspruch 52, ferner umfassend eine Abdeckung mit einem Eingabeteil und einem Ausgabeteil, wobei die Abdeckung über den Koppelteil des Gehäuses passt.

54. Optisches Schneidegerät für die zahnärztliche Verwendung gemäß Anspruch 52, wobei die zweite Faseroptik-Röhre Saphir umfasst.

55. Medizinisches Handstück, umfassend eine Apparatur nach einem beliebigen der Ansprüche 1 bis 14, und
ein Gehäuse, das ein proximales Gehäuseende und ein Ende für die Abgabe der Strahlung einschließt,
wobei die Quelle der elektromagnetischen Energie eine erste Faserführung einschließt, die ein Eingabeende hat und im Gehäuse gelagert ist, für das Leiten der Laserstrahlung von einer äußeren Laserquelle zum Ende des Gehäuses für die Abgabe der Strahlung,
eine erste Endhülse (Ferrul), die ein keramisches oder kristallines Material umfasst und für das Befestigen der ersten Faserführung am Gehäuse ausgelegt ist, so dass das Ausgabeende der ersten Faserführung dem Ende des Gehäuses für die Abgabe der Strahlung gegenüber liegt,
eine zweite Faserführung mit einem Empfängerende, das für das Empfangen von Laserstrahlung aus dem Ausgabeende der ersten Faserführung ausgelegt ist, und
eine zweite Endhülse (Ferrul), die ein keramisches oder kristallines Material umfasst und für das Befestigen der zweiten Faserführung am Gehäuse ausgelegt ist, so dass das Empfängerende der zweiten Faserführung dem Ausgabeende der ersten Faserführung gegenüber liegt.

56. Medizinisches Handstück, wie es im Anspruch 55 aufgeführt wird, wobei die erste Endhülse die erste Faserführung abnehmbar am Gehäuse befestigt, so dass das Ausgabeende der ersten Faserführung dem Ende des Gehäuses für die Abgabe der Strahlung gegenüber liegt.

57. Medizinisches Handstück, wie es im Anspruch 56 aufgeführt wird, wobei die erste Endhülse und die zweite Endhülse einen Gasflusspfad in einer Richtung von der ersten Faserführung zum Empfängerende der zweiten Faserführung bilden.

58. Medizinisches Handstück, wie es im Anspruch 57 aufgeführt wird, wobei sich der Gasflusspfad über das Empfängerende der zweiten Faserführung und über die zweite Endhülse erstreckt.

59. Apparatur gemäß Anspruch 1 bis 14, ferner umfassend eine die elektromagnetische Energie leitende Apparatur, umfassend
ein Gehäuse,
eine innere, im Inneren des Gehäuses gelagerte Leitung für die elektromagnetische Energie, wobei die innere Leitung für die elektromagnetische Energie ein proximales Ende, ein distales Ende und eine Achse aufweist, die sich zwischen dem proximalen Ende und dem distalen Ende erstreckt, und
eine äußere Leitung für die elektromagnetische Energie, die die innere Leitung für die elektromagnetische Energie umgibt und mit dieser in Kontakt steht, wobei die äußere Leitung für die elektromagnetische Energie eine Achse aufweist, die im wesentlichen kolinear mit der Achse der inneren Leitung für die elektromagnetische Energie verläuft.

60. Apparatur, wie sie im Anspruch 59 definiert ist, wobei die äußere Leitung für die elektromagnetische Energie eine Endhülse (Ferrul) umfasst.

61. Apparatur, wie sie im Anspruch 60 definiert ist, wobei sowohl die innere Leitung für die elektromagnetische Energie als auch die äußere Leitung für die elektromagnetische Energie ein kristallines Material umfasst.

62. Apparatur, wie sie im Anspruch 59 definiert ist, ferner umfassend wenigstens eine andere Leitung für die elektromagnetische Energie, wobei die andere Leitung die äußere Leitung für die elektromagnetische Energie umgibt und mit dieser in Kontakt steht, wobei die andere Leitung für die elektromagnetische Energie eine Achse aufweist, die im wesentlichen kolinear mit der Achse der äußeren Leitung für die elektromagnetische Energie verläuft.

63. Apparatur, wie sie im Anspruch 59 definiert ist, ferner umfassend eine Leitung für die Quelle der elektromagnetische Energie zur Eingabe elektromagnetischer Energie sowohl in die innere Leitung für die elektromagnetische Energie als auch die äußere Leitung für die elektromagnetische Energie.

64. Apparatur, wie sie im Anspruch 60 definiert ist, wobei ein Durchmesser der Leitung für die Quelle der elektromagnetische Energie größer als ein Durchmesser der inneren Leitung für die elektromagnetische Energie ist.

65. Apparatur, wie sie im Anspruch 59 aufgeführt wird ist, wobei die innere Leitung für die elektromagnetische Energie und die äußere Leitung für die elektromagnetische Energie konzentrisch sind.

66. Apparatur, wie sie im Anspruch 59 aufgeführt wird ist, wobei die innere Leitung für die elektromagnetische Energie und die äußere Leitung für die elektromagnetische Energie die gleiche Achse teilen.

67. Apparatur, wie sie im Anspruch 66 aufgeführt wird ist, wobei wenigstens entweder die innere Leitung für die elektromagnetische Energie oder die äußere Leitung für die elektromagnetische Energie Saphir umfasst.

68. Apparatur gemäß einem beliebigen der Ansprüche 1 bis 14, ferner umfassend eine Apparatur zur Leitung optischer Energie, umfassend eine gebogene kristalline Faser, die für das Leiten optischer Energie durch sie hindurch ausgelegt ist, wobei die gebogene kristalline Faser ein proximales Ende, ein distales Ende und eine Achse aufweist, die sich zwischen dem proximalen Ende und dem distalen Ende erstreckt, wobei ein erster Teil der Achse nahe dem proximalen Ende der gebogenen kristallinen Faser nicht parallel zu einem zweiten Teil der Achse nahe dem distalen Ende der gebogenen kristallinen Faser verläuft.

69. Apparatur, wie sie im Anspruch 68 aufgeführt wird, wobei sich die gebogene kristalline Faser zwischen einer Laserquelle und einem distalen Ende einer Laserausgabevorrichtung erstreckt.

70. Apparatur, wie sie im Anspruch 68 aufgeführt wird, wobei zwischen dem ersten Teil der Achse und dem zweiten Teil der Achse ein Winkel von ungefähr 90 Grad vorliegt.

71. Apparatur, wie sie im Anspruch 68 aufgeführt wird, ferner umfassend ein Gehäuse, wobei die gebogene kristalline Faser innerhalb des Gehäuses angeordnet ist.

72. Apparatur, wie sie im Anspruch 71 aufgeführt wird, wobei das Gehäuse mit einem medizinischen Handstück verbunden ist und die gebogene kristalline Faser durch einen Gasflusspfad innerhalb des medizinischen Handstücks gekühlt wird.

73. Apparatur, wie sie im Anspruch 68 aufgeführt wird, wobei die optische Energie eine Quelle für kohärentes Licht umfasst.

74. Apparatur, wie sie im Anspruch 73 aufgeführt wird, wobei das kohärente Licht eine Wellenlänge in der Gegend von ungefähr 2,5 bis 3,0 Mikrometer aufweist.

75. Apparatur, wie sie im Anspruch 74 aufgeführt wird, wobei das kohärente Licht eine Wellenlänge innerhalb eines Bereiches von ungefähr 2,78 bis 2,94 Mikrometer aufweist.

76. Apparatur, wie sie im Anspruch 75 aufgeführt wird, wobei die gebogene kristalline Faser Saphir umfasst.

77. Apparatur gemäß einem beliebigen der Ansprüche 1 bis 14, umfassend eine optische Energie leitende Apparatur, umfassend
ein Gehäuse,
eine kristalline Faser, die innerhalb des Gehäuses gelagert ist, wobei die kristalline Faser für die Leitung optischer Energie durch sie hindurch ausgelegt ist, wobei die kristalline Faser ein proximales Ende, ein distales Ende und eine Achse aufweist, die sich zwischen dem proximalen Ende und dem distalen Ende erstreckt, und
einen Gasflusspfad innerhalb des Gehäuses, wobei der Gasflusspfad die kristalline Faser einhüllt und sich vom proximalen Ende der kristallinen Faser zum distalen Ende der kristallinen Faser erstreckt.

78. Apparatur, wie sie im Anspruch 77 aufgeführt wird, wobei die kristalline Faser gebogen ist.

79. Medizinisches Handstück, umfassend
ein Gehäuse, das ein proximales Gehäuseende und ein distales Gehäuseende einschließt,
eine Apparatur gemäß einem beliebigen der Ansprüche 1 bis 27, die am distalen Gehäuseende angeordnet ist und für das Schneiden von Gewebe ausgelegt ist, und
eine Leitung für ein Medikament, die ein proximales Leitungsende und ein distales Leitungsende hat, wobei die Leitung für ein Medikament für die Aufnahme eines Medikaments in das proximale Leitungsende und die Abgabe des Medikaments durch das distale Leitungsende, das in der Nähe des distalen Gehäuseendes angeordnet ist, ausgelegt ist.

80. Medizinisches Handstück, wie es im Anspruch 79 aufgeführt wird, wobei der Gewebeschneider einen Laser oder einen Bohrer umfasst.

81. Medizinisches Handstück, wie es im Anspruch 80 aufgeführt wird, wobei der Gewebeschneider ferner für das Abtragen von Gewebe ausgelegt ist.

82. Medizinisches Handstück-Abgabesystem, umfassend eine Apparatur nach einem beliebigen der Ansprüche 1 bis 14, und
ein Gehäuse, das ein proximales Gehäuseende und ein Ende für die Abgabe der Strahlung einschließt,
eine Faserführung, die ein Eingabeende hat und im Gehäuse gelagert ist, für das Leiten der Laserstrahlung von einer äußeren Laserquelle zum Ende des Gehäuses für die Abgabe der Strahlung,
eine Endhülse (Ferrul), die für das Befestigen der Faserführung am Gehäuse ausgelegt ist, so dass das Ausgabeende der Faserführung dem Ende des Gehäuses für die Abgabe der Strahlung gegenüber liegt,
eine innere Schutzröhre, die über der Faserführung gelagert ist, und
eine äußere Schutzröhre, die über der inneren Schutzröhre gelagert ist.

83. Medizinisches Handstück-Abgabesystem, wie es im Anspruch 82 aufgeführt wird, wobei die Faserführung eine Vielzahl röhrenförmiger Mäntel umfasst, die die Faserführung umgeben und an ihr befestigt sind.

84. Medizinisches Handstück-Abgabesystem, wie es im Anspruch 82 aufgeführt wird, wobei die Endhülse ein proximales Ende und ein distales Ende aufweist, die innere Schutzröhre ein proximales Ende und ein distales Ende aufweist, die äußere Schutzröhre ein proximales Ende und ein distales Ende aufweist, und wenigstens das distale Ende der inneren Schutzröhre oder das distale Ende der äußeren Schutzröhre mit dem proximalen Ende der Endhülse in Kontakt stehen.

85. Medizinisches Handstück-Abgabesystem, wie es im Anspruch 84 aufgeführt wird, wobei die Endhülse eine Öffnung für die Aufnahme der Faserführung umfasst, wobei ein Durchmesser der Öffnung ungefähr gleich einem Außendurchmesser der Faserführung ist.

86. Medizinisches Handstück-Abgabesystem, wie es im Anspruch 84 aufgeführt wird, wobei das proximale Ende der inneren Schutzröhre und das proximale Ende der äußeren Schutzröhre in der Nähe eines SMA-Steckers gelegen sind, der ein proximales Ende der Faserführung umgibt.

87. Medizinisches Handstück-Abgabesystem, wie es im Anspruch 86 aufgeführt wird, wobei der SMA-Stecker ein proximales Ende und ein distales Ende hat und die Faserführung einen Abstand zwischen dem proximalen Ende der Endhülse und dem distalen Ende des SMA-Steckers überbrückt,
wobei eine Länge von wenigstens einer der inneren Schutzröhre und der äußeren Schutzröhre kürzer ist als der Abstand, der durch die Faserführung zwischen dem proximalen Ende der Endhülse und dem distalen Ende des SMA-Steckers überbrückt wird.

88. Medizinisches Handstück-Abgabesystem, wie es im Anspruch 87 aufgeführt wird, wobei eine Länge von wenigstens einer der inneren Schutzröhre und der äußeren Schutzröhre um wenigstens ein Viertel eines Inch kürzer ist als der Abstand, der durch die Faserführung zwischen dem proximalen Ende der Endhülse und dem distalen Ende des SMA-Steckers überbrückt wird.

89. Medizinisches Handstück-Abgabesystem, wie es im Anspruch 88 aufgeführt wird, wobei die kürzere Länge von wenigstens einer der inneren Schutzröhre und der äußeren Schutzröhre das Schieben von wenigstens einer der inneren Schutzröhre und der äußeren Schutzröhre über die Faserführung ermöglicht.

90. Medizinisches Handstück-Abgabesystem, wie es im Anspruch 86 aufgeführt wird, wobei der SMA-Stecker für das Anbringen der Faserführung an einer Quelle für elektromagnetische Energie ausgelegt ist,
wobei die innere Schutzröhre und die äußere Schutzröhre ein flexibles Kunststoffmaterial umfassen, und
das medizinische Handstück-Abgabesystem ferner eine Metallröhre umfasst, die um die äußere Schutzröhre in der Nähe des proximalen Endes der äußeren Schutzröhre angeordnet ist.

91. Medizinisches Handstück-Abgabesystem, wie es im Anspruch 90 aufgeführt wird, wobei die Quelle für die elektromagnetische Energie einen Laser umfasst.

92. Medizinisches Handstück-Abgabesystem, wie es im Anspruch 90 aufgeführt wird, wobei das medizinische Handstück-Abgabesystem ferner eine Kunststoffröhre umfasst, die die Metallröhre umgibt und mit dieser in Kontakt steht.

93. Medizinisches Handstück-Abgabesystem, wie es im Anspruch 92 aufgeführt wird, wobei sowohl die innere Schutzröhre als auch die äußere Schutzröhre das Risiko einer Beschädigung der Faserführung vermindern.

94. Medizinisches Handstück-Abgabesystem, wie es im Anspruch 93 aufgeführt wird, wobei sowohl die innere Schutzröhre als auch die äußere Schutzröhre die Wahrscheinlichkeit vermindern, dass das Biegen der Faserführung zu einem Brechen der Faserführung führt.

95. Medizinisches Handstück-Abgabesystem, wie es im Anspruch 92 aufgeführt wird, wobei sowohl die innere Schutzröhre als auch die äußere Schutzröhre die Wahrscheinlichkeit vermindern, dass die Faserführung in der Nähe der Endhülse bricht.

96. Medizinisches Handstück-Abgabesystem, wie es im Anspruch 95 aufgeführt wird, wobei die innere Schutzröhre und die äußere Schutzröhre ein flexibles Kunststoffmaterial umfassen.

97. Medizinisches Handstück-Abgabesystem, wie es im Anspruch 82 aufgeführt wird, wobei die innere Schutzröhre verschiebbar über der Faserführung angeordnet ist.

98. Medizinisches Handstück-Abgabesystem, wie es im Anspruch 82 aufgeführt wird, wobei die äußere Schutzröhre verschiebbar über der Faserführung angeordnet ist.

99. Medizinisches Handstück-Abgabesystem, wie es im Anspruch 98 aufgeführt wird, ferner umfassend wenigstens eine weitere Schutzröhre, die verschiebbar über der äußeren Schutzröhre angeordnet ist.

100. Medizinisches Handstück-Abgabesystem, wie es im Anspruch 99 aufgeführt wird, wobei die innere Schutzröhre verschiebbar über der Faserführung angeordnet ist.

101. Medizinisches Handstück-Abgabesystem, wie es im Anspruch 82 aufgeführt wird, ferner umfassend
eine zweite Faserführung mit einem Empfängerende, das für den Empfang von Laserstrahlung aus dem Ausgabeende der Faserführung ausgelegt ist,
eine zweite Endhülse, die für das Befestigen der zweiten Faserführung am Gehäuse ausgelegt ist, so dass das Empfängerende der zweiten Faserführung dem Ausgabeende der Faserführung gegenüber liegt, und
einen Abstandshalter, der zwischen der Endhülse und der zweiten Endhülse angeordnet ist.

102. Medizinisches Handstück-Abgabesystem, wie es im Anspruch 101 aufgeführt wird, wobei wenigstens eine von der Endhülse, der zweiten Endhülse und dem Abstandshalter wenigstens eines von einem keramischen und einem kristallinen Material umfasst.

103. Medizinisches Handstück-Abgabesystem, wie es im Anspruch 102 aufgeführt wird, wobei das kristalline Material Saphir umfasst.

104. Medizinisches Handstück, umfassend eine Apparatur gemäß den Ansprüchen 1, 11 oder 12,
ein Hülsengehäuse, das so ausgelegt ist, dass es von einer Hand des Benutzers gehalten wird, und das ein proximales Ende des Hülsengehäuses, einen mittleren Abschnitt des Hülsengehäuses, ein Ende für die Strahlungsabgabe und eine längliche Öffnung einschließt, die sich im Hülsengehäuse vom proximalen Ende des Hülsengehäuses zum mittleren Abschnitt des Hülsengehäuses erstreckt, wobei das Hülsengehäuse eine Faserführung für die Abgabe aufweist, wobei sich die Faserführung zwischen dem mittleren Abschnitt des Hülsengehäuses und dem Ende für die Strahlungsabgabe erstreckt, wobei die Faserführung für die Abgabe ein proximales Ende und ein distales Ende aufweist, und
eine Schaftanordnung, die so ausgelegt ist, dass sie abnehmbar innerhalb der länglichen Öffnung angeordnet ist, wobei die Schaftanordnung eine Quellenfaserführung aufweist, die ausgelegt ist, elektromagnetische Energie für den mittleren Abschnitt des Hülsengehäuses zu liefern, wobei die Quellenfaserführung ein distales Ende aufweist.

105. Medizinisches Handstück, wie es im Anspruch 104 aufgeführt wird, das ferner einen Kragen umfasst, der um das proximale Ende des Hülsengehäuses passt, wenn die Schaftanordnung in das Hülsengehäuse eingeführt wird, wobei der Kragen die Schaftanordnung im Hülsengehäuse hält.

106. Medizinisches Handstück, wie es im Anspruch 105 aufgeführt wird, wobei das proximale Ende des Hülsengehäuses Gewinde für die Aufnahme des Gewindes des Kragens aufweist.

107. Medizinisches Handstück, wie es im Anspruch 104 aufgeführt wird, wobei das distale Ende der Quellenfaserführung von einer ersten Endhülse (Ferrul) umgeben ist, und
das proximale Ende der Faserführung für Abgabe von einer zweiten Endhülse umgeben ist.

108. Medizinisches Handstück, wie es im Anspruch 107 aufgeführt wird, das ferner eine dritte Endhülse umfasst, die einen Teil der Faserführung für Abgabe in der Nähe des Endes des Hülsengehäuses für die Abgabe der Strahlung umgibt.

109. Medizinisches Handstück, wie es im Anspruch 107 aufgeführt wird, wobei die Schaftanordnung eine Leitung für eine Luftzufuhr aufweist, die um die Quellenfaserführung angeordnet ist.

110. Medizinisches Handstück, wie es im Anspruch 107 aufgeführt wird, wobei die Faser eine Vielzahl schützender Röhren umfasst, die um die Quellenfaserführung herum angeordnet sind, und eine Leitung für die Luftzufuhr, die um die Vielzahl der schützenden Röhren angeordnet ist.

111. Medizinisches Handstück, wie es im Anspruch 109 aufgeführt wird, wobei das Hülsengehäuse eine Luftleitung aufweist, die sich vom proximalen Ende des Hülsengehäuses zum Ende für die Abgabe der Strahlung erstreckt, und
eine Wasserleitung, die sich vom proximalen Ende des Hülsengehäuses zum Ende für die Abgabe der Strahlung erstreckt.

112. Medizinisches Handstück, wie es im Anspruch 110 aufgeführt wird, wobei das Hülsengehäuse ferner eine Leitung für ein Medikament aufweist, die sich allgemein vom proximalen Ende des Hülsengehäuses zum Ende für die Abgabe der Strahlung erstreckt.

113. Apparatur zur Implementierung eines medizinischen Verfahrens, die umfasst
eine Apparatur gemäß einem beliebigen der Ansprüche 1 bis 27 für die Durchführung einer medizinischen Behandlung an einer Operationsstelle, die im Inneren eines menschlichen Körpers lokalisiert ist oder mit diesem in Verbindung steht, und
einen Fluidrouter für das Lenken eines aromatisierten Fluids in Richtung der Operationsstelle.

114. Apparatur zur Implementierung eines medizinischen Verfahrens gemäß Anspruch 113, wobei beim Gebrauch ein aromatisiertes Fluid gewählt wird, das aromatisiert ist, um für die Geschmacksknospen des Patienten, der sich dem chirurgischen Eingriff unterzieht, ansprechend zu sein.

115. Apparatur zur Implementierung eines medizinischen Verfahrens gemäß Anspruch 114, wobei das aromatisierte Fluid beim Gebrauch so gewählt wird, dass es eines ist, das entweder einen Fruchtgeschmack oder einen Pfefferminzgeschmack aufweist.

116. Apparatur zur Implementierung eines medizinischen Verfahrens gemäß Anspruch 115, wobei das medizinische Instrument eines aus der Gruppe umfasst, die besteht aus einem Elektrokauter, einer Quelle für elektromagnetische Energie, einem Laser, einem mechanischen Bohrer, einer mechanischen Säge, einem Kanalfinder, einer Spritze und einem Absauger.

117. Apparatur zur Implementierung eines medizinischen Verfahrens gemäß Anspruch 116, wobei die Quelle der elektromagnetischen Energie die elektromagnetische Energie auf die Operationsstelle fokussiert, und
wobei der Fluidrouter aromatisiertes Fluid auf die Operationsstelle lenkt, um so die Operationsstelle zu kühlen, während die Operationsstelle geschnitten wird.

118. Apparatur zur Implementierung eines medizinischen Verfahrens gemäß Anspruch 116, wobei der Fluidrouter in einem ersten Modus betrieben werden kann, bei dem das gelenkte Fluid aromatisiert ist, und in einem zweiten Modus, bei dem das gelenkte Fluid nicht aromatisiert ist.

119. Apparatur zur Implementierung eines medizinischen Verfahrens gemäß Anspruch 115, wobei der Fluidrouter einen Zerstäuber für das Zerstäuben des aromatisierten Fluids zu zerstäubten aromatisierten Fluidteilchen, ehe das aromatisierte Fluid in Richtung der Operationsstelle gelenkt wird, umfasst.

120. Apparatur zur Implementierung eines medizinischen Verfahrens gemäß Anspruch 119, wobei der Zerstäuber so angeordnet ist, das er die zerstäubten aromatisierten Fluidteilchen in ein Luftvolumen über der Operationsstelle lenkt, und
wobei die Quelle der elektromagnetischen Energie so angeordnet ist, dass sie die elektromagnetische Energie in das Luftvolumen lenkt, wobei die elektromagnetische Energie eine Wellenlänge hat, die im wesentlichen von den zerstäubten Fluidteilchen im Luftvolumen absorbiert wird, wobei die Absorption der elektromagnetischen Energie durch die zerstäubten aromatisierten Fluidteilchen dazu führt, dass die zerstäubten aromatisierten Fluidteilchen explodieren und mechanische Kräfte auf die Operationsstelle ausüben.

121. Apparatur zur Implementierung eines medizinischen Verfahrens gemäß Anspruch 120, wobei der Fluidrouter in einem ersten Modus betrieben werden kann, bei dem das gelenkte Fluid aromatisiert ist, und in einem zweiten Modus, bei dem das gelenkte Fluid nicht aromatisiert ist.

122. Apparatur zur Implementierung eines medizinischen Verfahrens, die umfasst
eine Apparatur gemäß einem beliebigen der Ansprüche 1 bis 27 für die Durchführung einer medizinischen Behandlung an einer Operationsstelle, die im Inneren eines menschlichen Körpers lokalisiert ist oder mit diesem in Verbindung steht, und
einen Router für das Lenken eines parfümierten Mediums in Richtung der Operationsstelle.

123. Apparatur zur Implementierung eines medizinischen Verfahrens gemäß Anspruch 122, wobei das parfümierte Medium beim Gebrauch so gewählt ist, das es eines ist, das parfümiert ist, um den Geruchssinn des Patienten, der sich dem chirurgischen Eingriff unterzieht, anzusprechen.

124. Apparatur zur Implementierung eines medizinischen Verfahrens gemäß Anspruch 123, wobei das parfümierte Medium beim Gebrauch so gewählt wird, dass es eines ist, das entweder ein Fruchtparfüm, ein Pfefferminzparfüm oder einen Luftverbesserer umfasst.

125. Apparatur zur Implementierung eines medizinischen Verfahrens gemäß Anspruch 122, wobei das medizinische Instrument eines aus der Gruppe umfasst, die besteht aus einem Elektrokauter, einer Quelle für elektromagnetische Energie, einem Laser, einem mechanischen Bohrer, einer mechanischen Säge, einem Kanalfinder, einer Spritze und einem Absauger.

126. Apparatur zur Implementierung eines medizinischen Verfahrens gemäß Anspruch 123, wobei das parfümierte Medium parfümierte Luft umfasst.

127. Apparatur zur Implementierung eines medizinischen Verfahrens gemäß Anspruch 122, wobei die Quelle der elektromagnetischen Energie beim Gebrauch so angeordnet ist, dass sie die elektromagnetische Energie auf die Operationsstelle fokussiert,
wobei das parfümierte Medium parfümiertes Wasser umfasst, und
der Router so angeordnet ist, dass er beim Gebrauch parfümiertes Wasser auf die Operationsstelle lenkt, um so die Operationsstelle zu kühlen, während die Operationsstelle von der Quelle der elektromagnetischen Energie geschnitten wird.

128. Apparatur zur Implementierung eines medizinischen Verfahrens gemäß Anspruch 123, wobei die Quelle der elektromagnetischen Energie beim Gebrauch so angeordnet ist, dass sie die elektromagnetische Energie auf die Operationsstelle fokussiert,
wobei das parfümierte Medium einen parfümierten Nebel umfasst, und
der Router so angeordnet ist, dass er beim Gebrauch den parfümierten Nebel auf die Operationsstelle lenkt, um so die Operationsstelle zu kühlen, während die Operationsstelle von der Quelle der elektromagnetischen Energie geschnitten wird.

129. Apparatur zur Implementierung eines medizinischen Verfahrens gemäß Anspruch 122, wobei der Router in einem ersten Modus betrieben werden kann, bei dem das gelenkte Medium parfümiert ist, und in einem zweiten Modus, bei dem das gelenkte Medium nicht parfümiert ist.

130. Apparatur zur Implementierung eines medizinischen Verfahrens gemäß Anspruch 123, wobei der Router einen Zerstäuber für das Zerstäuben des parfümierten Mediums, ehe das parfümierte Medium in Richtung der Operationsstelle gelenkt wird, umfasst.

131. Apparatur zur Implementierung eines medizinischen Verfahrens gemäß Anspruch 127, wobei der Zerstäuber so angeordnet ist, das er beim Gebrauch das zerstäubte parfümierte Medium in ein Luftvolumen über der Operationsstelle lenkt, und
wobei die Quelle der elektromagnetischen Energie so angeordnet ist, dass sie beim Gebrauch die elektromagnetische Energie in das Luftvolumen lenkt, wobei die elektromagnetische Energie eine Wellenlänge hat, die im wesentlichen von Teilen des zerstäubten parfümierten Mediums im Luftvolumen absorbiert wird, wobei die Absorption der elektromagnetischen Energie durch die Teile des zerstäubten parfümierten Mediums dazu führt, dass die Teile des zerstäubten parfümierten Mediums explodieren und mechanische Kräfte auf die Operationsstelle ausüben.

132. Apparatur zur Implementierung eines medizinischen Verfahrens gemäß Anspruch 120, wobei der Router in einem ersten Modus betrieben werden kann, bei dem die gelenkte Fluid parfümiert ist, und in einem zweiten Modus, bei dem die gelenkte Fluid nicht parfümiert ist.

133. Apparatur zur Implementierung eines medizinischen Verfahrens, die umfasst
ein Schneidegerät gemäß einem beliebigen der Ansprüche 1 bis 27 für die Durchführung einer medizinischen Behandlung an einer Operationsstelle, die im Inneren eines menschlichen Körpers lekalisiert ist oder mit diesem in Verbindung steht, und
einen Fluidrouter für das Lenken einer ionisierten Lösung beim Gebrauch in Richtung der Operationsstelle.

134. Apparatur zur Implementierung eines medizinischen Verfahrens gemäß Anspruch 133, wobei die ionisierte Lösung eine biokompatible Salinelösung umfasst.

135. Apparatur zur Implementierung eines medizinischen Verfahrens gemäß Anspruch 133, wobei das medizinische Instrument eines aus der Gruppe umfasst, die besteht aus einem Elektrokauter, einer Quelle für elektromagnetische Energie, einem Laser, einem mechanischen Bohrer, einer mechanischen Säge, einem Kanalfinder, einer Spritze und einem Absauger.

136. Apparatur zur Implementierung eines medizinischen Verfahrens gemäß Anspruch 135, wobei die Quelle der elektromagnetischen Energie die elektromagnetische Energie auf die Operationsstelle fokussiert, und
wobei der Fluidrouter das ionisierte Fluid auf die Operationsstelle lenkt, um so die Operationsstelle zu kühlen, während die Operationsstelle geschnitten wird.

137. Apparatur zur Implementierung eines medizinischen Verfahrens gemäß Anspruch 135, wobei der Fluidrouter einen Zerstäuber für das Zerstäuben des ionisierten Fluids zu zerstäubten Fluidteilchen, ehe die zerstäubten Fluidteilchen in Richtung der Operationsstelle gelenkt werden, umfasst.

138. Apparatur zur Implementierung eines medizinischen Verfahrens gemäß Anspruch 137, wobei der Zerstäuber die zerstäubten Teilchen in ein Luftvolumen über der Operationsstelle lenkt, und
wobei die Quelle der elektromagnetischen Energie die elektromagnetische Energie in das Luftvolumen fokussiert, wobei die elektromagnetische Energie eine Wellenlänge hat, die im wesentlichen von den zerstäubten Teilchen im Luftvolumen absorbiert wird, wobei die Absorption der elektromagnetischen Energie durch die zerstäubten Teilchen dazu führt, dass die zerstäubten Teilchen explodieren und mechanische Kräfte auf die Operationsstelle ausüben.

139. Apparatur zur Implementierung eines medizinischen Verfahrens gemäß Anspruch 137, wobei der Fluidrouter in einem ersten Modus betrieben werden kann, bei dem das von ihm gelenkte Fluid ionisiert ist, und in einem zweiten Modus, bei dem das von ihm gelenkte Fluid nicht ionisiert ist.

140. Apparatur zur Implementierung eines medizinischen Verfahrens gemäß Anspruch 139, wobei der Fluidrouter in einer Vielzahl von Modi zwischen dem ersten Modus und dem zweiten Modus betrieben werden kann, um dadurch die Schneidekraft der Quelle der elektromagnetischen Energie kontinuierlich zu steuern.

141. Apparatur zur Implementierung eines medizinischen Verfahrens gemäß Anspruch 140, wobei die Schneidekraft der Quelle der elektromagnetischen Energie abnimmt, wenn das Fluid aus dem Fluidrouter stärker ionisiert wird.

142. Apparatur zur Implementierung eines medizinischen Verfahrens gemäß Anspruch 141, wobei die Stärke der Quelle der elektromagnetischen Energie eingestellt werden kann, um die Schneidekraft der Quelle der elektromagnetischen Energie zu steuern.

143. Apparatur zur Implementierung eines medizinischen Verfahrens gemäß Anspruch 142, wobei die Stärke der Quelle der elektromagnetischen Energie erhöht werden kann, wenn das Fluid aus dem Fluidrouter stärker ionisiert wird, um dadurch eine konstante Schneidekraft der Quelle der elektromagnetischen Energie aufrecht zu halten, und
wobei die Stärke der Quelle der elektromagnetischen Energie abgesenkt werden kann, wenn das Fluid aus dem Fluidrouter schwächer ionisiert wird, um dadurch eine konstante Schneidekraft der Quelle der elektromagnetischen Energie aufrecht zu halten.

144. Apparatur gemäß einem beliebigen der Ansprüche 1 bis 27, wobei der Zerstäuber so angeordnet ist, dass er beim Gebrauch pigmentierte zerstäubte Fluidteilchen in das vorher festgelegte Volumen abgibt.

145. Apparatur gemäß einem beliebigen der Ansprüche 1 bis 27, die ferner umfasst
eine Fluidquelle zur Abgabe, beim Gebrauch, eines pigmentierten Fluids in die ungefähre Nachbarschaft der Zieloberfläche, wobei das pigmentierte Fluid eine Farbe hat, die im wesentlichen von der elektromagnetischen Energie nicht absorbiert wird.

146. Apparatur gemäß einem beliebigen der Ansprüche 1 bis 27, die ferner umfasst eine Fluidquelle zur Abgabe von Fluid in die ungefähre Nachbarschaft der Zieloberfläche, wobei das Fluid eine Farbe hat, die im wesentlichen von der elektromagnetischen Energie nicht absorbiert wird.

147. Apparatur gemäß einem beliebigen der Ansprüche 1 bis 27, die ferner umfasst eine mit dem Zerstäuber verbundene Zuführeinrichtung für das selektive Zuführen einer Pigmentierung zu den zerstäubten Fluidteilchen.

148. Apparatur gemäß Anspruch 147, wobei die Pigmentierung selektiv den zerstäubten Fluidteilchen gemäß einer Benutzereingabe zugeführt wird.

149. Apparatur gemäß Anspruch 147, wobei die elektromagnetische Energie eine Wellenlänge hat, die im wesentlichen von den zerstäubten Fluidteilchen absorbiert wird, wenn die Pigmentierung den zerstäubten Fluidteilchen zugeführt wird.

150. Apparatur gemäß Anspruch 147, wobei die elektromagnetische Energie eine Wellenlänge hat, die im wesentlichen von den zerstäubten Fluidteilchen absorbiert wird, wenn die Pigmentierung den zerstäubten Fluidteilchen nicht zugeführt wird.

151. Apparatur gemäß Anspruch 147, wobei der Zerstäuber eine Wasserleitung und eine Luftquellenleitung umfasst, und
wobei die Pigmentierung selektiv den zerstäubten Fluidteilchen entweder über die Wasserquellenleitung oder über Luftquellenleitung zugeführt wird.

152. Apparatur zur Implementierung eines medizinischen Verfahrens, die umfasst
eine Apparatur gemäß einem beliebigen der Ansprüche 1 bis 27 für die Durchführung einer medizinischen Behandlung an einer Operationsstelle, die im Inneren eines menschlichen Körpers lokalisiert ist oder mit diesem in Verbindung steht, und
einen Fluidrouter für das Lenken eines Fluids, das einen messbaren Fluidparameter aufweist, der sich von einem entsprechenden messbaren Fluidparameter von Wasser unterscheidet, in Richtung der Operationsstelle.

153. Apparatur gemäß Anspruch 152, wobei der messbare Fluidparameter einer aus der Gruppe ist, die besteht aus einer Dichte, einer volumenbezogenen Masse, einem pH-Wert, einer Viskosität und einer Temperatur.

154. Apparatur zur Implementierung eines medizinischen Verfahrens, die umfasst
ein Schneidegerät gemäß einem beliebigen der Ansprüche 1 bis 27 für die Durchführung einer medizinischen Behandlung an einer Operationsstelle, die im Inneren eines menschlichen Körpers lokalisiert ist oder mit diesem in Verbindung steht, und einen Router für das Lenken eines Medikaments in Richtung der Operationsstelle.

155. Apparatur zur Implementierung eines medizinischen Verfahrens gemäß Anspruch 154, wobei, beim Gebrauch, das Medikamentenmedium ein Antibiotikum, Iod, Steroid, Anästhetikum, einen Entzündungshemmer, ein Desinfektionsmittel, Adrenalin, Epinephrin, ein Antiseptikum oder Adstringens umfassen kann.

156. Apparatur zur Implementierung eines medizinischen Verfahrens gemäß Anspruch 154, wobei, beim Gebrauch, das Medikamentenmedium Vitamine, Kräuter oder Mineralien umfassen kann.

157. Apparatur zur Implementierung eines medizinischen Verfahrens gemäß Anspruch 154, wobei das medizinische Instrument eines aus der Gruppe umfasst, die besteht aus einem Elektrokauter, einer Quelle für elektromagnetische Energie, einem Laser, einem mechanischen Bohrer, einer mechanischen Säge, einem Kanalfinder, einer Spritze und einem Absauger.

158. Apparatur zur Implementierung eines medizinischen Verfahrens gemäß Anspruch 157, wobei die Quelle der elektromagnetischen Energie so angeordnet ist, dass sie beim Gebrauch die elektromagnetische Energie auf die Operationsstelle fokussiert, und
wobei der Router beim Gebrauch das Medium mit dem Medikament auf die Öperationsstelle lenkt, um so die Operationsstelle zu kühlen und medikamentös zu versorgen, während die Operationsstelle geschnitten wird.

159. Apparatur zur Implementierung eines medizinischen Verfahrens gemäß Anspruch 158, wobei der Router in einem ersten Modus betrieben werden kann, bei dem das gelenkte Fluid mit einem Medikament versehen ist, und in einem zweiten Modus, bei dem das gelenkte Fluid nicht mit einem Medikament versehen ist.

160. Apparatur zur Implementierung eines medizinischen Verfahrens gemäß Anspruch 159, wobei der Router einen Zerstäuberfür das Zerstäuben des Mediums mit dem Medikament zu zerstäubten Teilchen, ehe das Medium mit dem Medikament in Richtung der Operationsstelle gelenkt wird, umfasst.

161. Apparatur zur Implementierung eines medizinischen Verfahrens gemäß Anspruch 160, wobei der Zerstäuber beim Gebrauch so angeordnet ist, das er die zerstäubten Teilchen in ein Luftvolumen über der Operationsstelle lenkt, und
wobei die Quelle der elektromagnetischen Energie beim Gebrauch so angeordnet ist, dass sie die elektromagnetische Energie in das Luftvolumen fokussiert, wobei die elektromagnetische Energie eine Wellenlänge hat, die im wesentlichen von den zerstäubten Teilchen im Luftvolumen absorbiert wird, wobei die Absorption der elektromagnetischen Energie durch die zerstäubten Teilchen dazu führt, dass die zerstäubten Teilchen explodieren und mechanische Kräfte auf die Operationsstelle ausüben.

162. Apparatur zur Implementierung eines medizinischen Verfahrens gemäß Anspruch 160, wobei der Fluidrouter in einem ersten Modus betrieben werden kann, bei dem das durch ihn gelenkte Fluid mit einem Medikament versehen ist, und in einem zweiten Modus, bei dem das gelenkte Fluid nicht mit einem Medikament versehen ist.

163. Apparatur zum Abtöten von Bakterien in oder auf sowohl dem Mund eines Patienten als auch einem zahnärztlichen Instrument in der Nachbarschaft des Mundes des Patienten, umfassend
ein chirurgisches Werkzeug, das eine Apparatur gemäß Anspruch 1 umfasst, für das Schneiden oder Bohren von Material im Munde eines Patienten, und
einen Fluidrouter für das Lenken eines Desinfektionsmittels in den Mund des Patienten, wobei ein Teil des gelenkten Desinfektionsmittels in die Luft übertritt und sich auf dem zahnärztlichen Instrument absetzt, wodurch das zahnärztliche Instrument desinfiziert wird.

164. Apparatur zum Bohren und Konditionieren, die umfasst
eine Apparatur gemäß einem beliebigen der Beispiele 1 bis 27 für die Durchführung eines Bohrvorgangs an einem Zahn im Munde eines Patienten, und
einen Fluidrouter für das Lenken eines konditionierten Fluids in Richtung des Zahnes, an dem operiert wird, wobei das konditionierte Fluid eine messbare Eigenschaft aufweist, die sich von einer entsprechenden messbaren Eigenschaft von Wasser unterscheidet.

165. Apparatur zum Bohren und Konditionieren gemäß Anspruch 164, wobei die messbare Eigenschaft wenigstens eine aus der Gruppe umfasst, die besteht aus einem mittels der Geschmacksknospen eines Patienten wahrnehmbaren Aroma, einem mittels des Geruchsinnes des Patienten wahrnehmbaren Duft, einer Salzigkeit, einem Pigment, einem Medikament und einem Desinfektionsmittel.

166. Apparatur zum Bohren und Konditionieren gemäß Anspruch 165, wobei das Medikament wenigstens eines aus der Gruppe umfasst, die besteht aus einem Antibiotikum, einem Steroid, einem Anästhetikum, einem Entzündungshemmer, einem Desinfektionsmittel, Adrenalin, Epinephrin, einem Antiseptikum und einem Adstringens.

167. Apparatur zum Bohren und Konditionieren gemäß Anspruch 165, wobei das Medikament entweder Vitamine, Kräuter oder Mineralien umfasst.

168. Apparatur zur Implementierung eines medizinischen Verfahrens, die umfasst
eine Apparatur gemäß einem beliebigen der Ansprüche 1 bis 27 für die Durchführung einer medizinischen Behandlung an einer Operationsstelle, die im Inneren eines menschlichen Körpers lokalisiert ist oder mit diesem in Verbindung steht, und
einen Fluidrouter für das selektive Lenken eines pigmentierten Fluids in Richtung der Operationsstelle.

169. Apparatur zur Implementierung eines medizinischen Verfahrens gemäß Anspruch 168, wobei das medizinische Instrument eines aus der Gruppe umfasst, die besteht aus einem Elektrokauter, einer Quelle für elektromagnetische Energie, einem Laser, einem mechanischen Bohrer, einer mechanischen Säge, einem Kanalfinder, einer Spritze und einem Absauger.

170. Apparatur zur Implementierung eines medizinischen Verfahrens gemäß Anspruch 169, wobei der Fluidrouter in einem ersten Modus betrieben werden kann, bei dem das gelenkte Fluid pigmentiert ist, und in einem zweiten Modus, bei dem das gelenkte Fluid nicht pigmentiert ist.

171. Apparatur zur Implementierung eines medizinischen Verfahrens gemäß Anspruch 170, wobei eine Wellenlänge der Quelle der elektromagnetischen Energie im wesentlichen von der pigmentierten Flüssigkeit absorbiert wird.

## Revendications

1. Appareil comprenant :
une sortie de fluide comprenant un fluide en utilisation et étant construit pour diriger le fluide vers une première proximité par rapport à la sortie de fluide ; et
une source d'énergie électromagnétique pour fournir de l'énergie électromagnétique à une seconde proximité ;
la première proximité et la seconde proximité étant disposées en des emplacements respectifs par rapport à la sortie de fluide ;
**caractérisé en ce que** ladite première proximité et ladite seconde proximité se coupent dans un volume (59) par rapport à la sortie de fluide, ladite sortie de fluide comprenant en outre un atomiseur (63, 65, 71) pour générer une combinaison de particules de fluide atomisées, et pour placer la combinaison de particules de fluide atomisées dans ledit volume ; ladite source d'énergie électromagnétique comprenant une source d'énergie électromagnétique configurée spécialement (51) qui est configurée, lorsque ledit appareil est en utilisation, pour fournir de l'énergie électromagnétique à une longueur d'onde qui est sensiblement absorbée par ladite combinaison de particules de fluide atomisées et pour diriger une concentration crête de ladite énergie électromagnétique dans ledit volume ; ladite combinaison de particules de fluide atomisées étant dimensionnée et distribuée de telle manière que, lorsqu'elle est placée dans ledit volume et irradiée avec ladite énergie électromagnétique focalisée, ladite énergie électromagnétique est sensiblement absorbée par au moins une partie de ladite combinaison de particules de fluide atomisées, tandis que des efforts mécaniques disruptifs sont appliqués à une surface cible (107).

2. Appareil selon la revendication 1 dans lequel ladite combinaison de particules de fluide comprend une combinaison de particules de fluide qui sont dimensionnées et distribuées de telle manière que lorsqu'elles sont placées dans ledit volume et irradiées par ladite source d'énergie électromagnétique, lesdites particules de fluide augmentent de volume.

3. Appareil selon la revendication 1 ou la revendication 2 dans lequel ladite sortie comprend un atomiseur, et ladite combinaison de particules de fluide comprend une combinaison de particules de fluide atomisées ayant des diamètres étroitement répartis autour d'une valeur moyenne.

4. Appareil selon l'une quelconque des revendications précédentes dans lequel lorsque ledit appareil est positionné en utilisation de telle manière que le volume est positionné au-dessus de la surface cible, la partie des particules de fluide augmente de volume et applique des efforts mécaniques disruptifs à ladite surface cible.

5. Appareil selon l'une quelconque des revendications précédentes dans lequel l'énergie électromagnétique est hautement absorbée par la partie de particules de fluide.

6. Appareil selon l'une quelconque des revendications précédentes dans lequel lorsque ledit appareil est positionné, en service, de telle manière que ledit volume est positionné au-dessus d'une surface cible, ledit appareil applique des efforts mécaniques disruptifs de coupe et d'ablation à la surface cible.

7. Appareil selon l'une quelconque des revendications précédentes dans lequel la sortie est alimentée par une ligne d'entrée d'air et une ligne d'entrée d'eau.

8. Appareil selon l'une quelconque des revendications précédentes, dans lequel l'énergie est délivrée à travers une fibre optique (23) ; et
dans lequel les particules de fluide entrent en contact avec la fibre optique pour de ce fait refroidir et nettoyer la. fibre optique.

9. Appareil selon la revendication 8, dans lequel les particules de fluide entrent en contact avec la fibre optique pour de ce fait éliminer les débris particulaires de la fibre optique.

10. Appareil selon la revendication 8 ou la revendication 9 dans lequel la fibre optique comprend du saphir.

11. Appareil selon la revendication 1 comprenant en outre :
une commande utilisateur pour entrer une combinaison de particules de fluide atomisées spécifiée par l'utilisateur, la combinaison de particules de fluide atomisées correspondant à une taille moyenne, une répartition spatiale et une vitesse des particules de fluide atomisées spécifiées par l'utilisateur ; dans lequel l'atomiseur réagit à la commande utilisateur.

12. Appareil selon l'une quelconque des revendications précédentes, dans lequel l'absorption de l'énergie électromagnétique par la partie de particules de fluide dans ledit volume amène ladite partie de particules de fluide à appliquer des efforts mécaniques disruptifs à ladite surface cible.

13. Appareil selon la revendication 12, dans lequel :
les efforts mécaniques disruptifs comprennent des efforts mécaniques de coupe et d'ablation ; et
lorsque ledit appareil est positionné en utilisation de telle manière que ledit volume est positionné au-dessus de la surface cible, ladite partie de particules de fluide est amenée à appliquer les efforts mécaniques de coupe et d'ablation sur la surface cible.

14. Appareil selon la revendication 11 ou la revendication 12, dans lequel ladite commande utilisateur comprend un dispositif d'entrée utilisateur (77) pour spécifier soit une coupe à haute résolution soit une coupe à basse résolution, et pour spécifier soit une coupe à pénétration profonde soit une coupe à pénétration peu profonde ; et
ledit atomiseur réagit au dispositif d'entrée utilisateur pour générer :
(1) une combinaison de particules de fluide atomisées comprenant des particules de fluide relativement petites, en réponse à une entrée utilisateur spécifiant une coupe à haute résolution ;
(2) une combinaison de particules de fluide atomisées comprenant des particules de fluide relativement grosses, en réponse à une entrée utilisateur spécifiant une coupe à basse résolution ;
(3) une combinaison de particules de fluide atomisées comprenant une répartition de particules de fluide à relativement basse densité, en réponse à une entrée utilisateur spécifiant une coupe à pénétration profonde ; et
(4) une combinaison de particules de fluide atomisées comprenant une répartition de particules de fluide à relativement haute densité, en réponse à une entrée utilisateur spécifiant une coupe à pénétration peu profonde.

15. Appareil selon la revendication 14, dans lequel le dispositif d'entrée utilisateur comprend une entrée unique pour commander l'efficacité de coupe.

16. Appareil selon la revendication 15, dans lequel le dispositif d'entrée utilisateur génère une répartition à relativement basse densité de particules de fluide relativement petites lorsque l'entrée unique spécifie une efficacité de coupe élevée et une répartition à relativement haute densité de particules de fluide relativement grosses lorsque l'entrée unique spécifie une efficacité de coupe faible.

17. Appareil selon la revendication 16, dans lequel chacune des particules de fluide relativement petites a un diamètre de particule de fluide, et
dans lequel un diamètre de particule de fluide moyen des diamètres des particules de fluide relativement petites est inférieur à la longueur d'onde de l'énergie électromagnétique focalisée dans le volume d'air adjacent à la surface cible.

18. Appareil selon la revendication 16, dans lequel chacune des particules de fluide relativement grosses a un diamètre de particule de fluide, et
dans lequel un diamètre de particule de fluide moyen des diamètres des particules de fluide relativement grosses est supérieur à la longueur d'onde de l'énergie électromagnétique focalisée dans le volume d'air adjacent à la surface cible.

19. Appareil selon l'une quelconque des revendications précédentes, dans lequel la source d'énergie électromagnétique (51) est un laser à grenat de gallium, de scandium et d'yttrium, dopé au chrome et à l'erbium (Er, Cr:YSGG) à l'état solide, qui génère de l'énergie électromagnétique ayant une longueur d'onde d'environ 2,78 microns.

20. Appareil selon l'une quelconque des revendications 1 à 19, dans lequel le fluide comprend de l'eau, et
dans lequel la source d'énergie électromagnétique (51) est un laser à grenat de gallium, de scandium et d'yttrium, dopé au chrome et à l'erbium (Er, Cr:YSGG) à l'état solide, qui génère de la lumière ayant une longueur d'onde dans une plage allant d'environ 2,70 à environ 2,80 microns.

21. Appareil selon l'une quelconque des revendications 1 à 19, dans lequel le fluide comprend de l'eau, et
dans lequel la source d'énergie électromagnétique (51) comprend un des lasers suivants :
(a) laser à grenat de gallium, de scandium et d'yttrium, dopé à l'erbium (Er:YSGG) à l'état solide, qui génère de l'énergie électromagnétique ayant une longueur d'onde dans une plage allant d'environ 2,70 à environ 2,80 microns ;
(b) laser à grenat d'aluminium et d'yttrium, dopé à l'erbium (Er:YAG) à l'état solide, qui génère de l'énergie électromagnétique ayant une longueur d'onde d'environ 2,94 microns ;
(c) laser à grenat d'aluminium et d'yttrium, dopé à l'erbium, au thulium et au chrome (CTE: YAG) à l'état solide, qui génère de l'énergie électromagnétique ayant une longueur d'onde d'environ 2,69 microns ;
(d) laser à orthoaluminate d'yttrium, dopé à l'erbium (Er:YAL03) à l'état solide, qui génère de l'énergie électromagnétique ayant une longueur d'onde dans la plage allant d'environ 2,71 à environ 2,86 microns ;
(e) laser à grenat d'aluminium et d'yttrium, dopé à l'holmium (Ho:YAG) à l'état solide, qui génère de l'énergie électromagnétique ayant une longueur d'onde d'environ 2,10 microns ;
(f) laser à grenat d'aluminium et d'yttrium, dopé au néodyme quadruplé, (Nd quadruplé:YAG) à l'état solide, qui génère de l'énergie électromagnétique ayant une longueur d'onde d'environ 266 nanomètres ;
(g) laser à excimère de fluorure d'argon (ArF), qui génère de l'énergie électromagnétique ayant une longueur d'onde d'environ 193 nanomètres ;
(h) laser à excimère de chlorure de xénon (XeCl), qui génère de l'énergie électromagnétique ayant une longueur d'onde d'environ 308 nanomètres ;
(i) laser à excimère de fluorure de krypton (KrF), qui génère de l'énergie électromagnétique ayant une longueur d'onde d'environ 248 nanomètres ; et
(j) laser à bioxyde de carbone (CO2) qui génère de l'énergie électromagnétique ayant une longueur d'onde dans une plage allant d'environ 9,0 à environ 10,6 microns.

22. Appareil selon la revendication 21, dans lequel le laser à grenat d'aluminium et d'yttrium, dopé au néodyme quadruplé à l'état solide a une fréquence de répétition supérieure à environ 1 Hz, une plage de largeur d'impulsion comprise entre environ 1 picoseconde et environ 1000 microsecondes, et une énergie supérieure à environ 1 millijoule par impulsion.

23. Appareil selon la revendication 21, dans lequel le laser à grenat de gallium, de scandium et d'yttrium, dopé au chrome et à l'erbium à l'état solide a une fréquence de répétition d'environ 20 Hz, une largeur d'impulsion d'environ 140 microsecondes, et une énergie comprise entre environ 1 et environ 300 millijoules par impulsion.

24. Appareil selon l'une quelconque des revendications 1 à 23, adapté à une utilisation avec une surface cible (107) qui comprend un tissu dur.

25. Appareil selon la revendication 24, ledit tissu dur comprenant l'un des éléments parmi de la dent, de l'émail de dent, de la dentine, du cément, de l'os et du cartilage.

26. Appareil selon l'une quelconque des revendications 1 à 25, adapté à une utilisation avec une surface cible (107) qui comprend un tissu mou.

27. Appareil selon la revendication 26, ledit tissu mou comprenant l'un des éléments parmi de la peau, de la muqueuse, de la gencive, du muscle, du coeur, du foie, du rein, du cerveau, de l'oeil et des vaisseaux.

28. Appareil selon l'une quelconque des revendications 1 à 27, adapté pour une utilisation avec une surface cible (107), qui comprend l'un des éléments parmi le verre, un matériau cristallin, et la surface d'une puce semi-conductrice.

29. Appareil selon la revendication 28, dans lequel la fraise mécanique induite de façon électromagnétique est configurée de telle manière que, en utilisation, elle effectue une légère ablation de la surface du verre ou du matériau cristallin, ce qui la rend adaptée au collage d'argent ou d'un autre matériau diélectrique sur la surface cible en verre ou en matériau cristallin pour former un miroir.

30. Appareil selon la revendication 29, dans lequel la fraise mécanique induite de façon électromagnétique est configurée de telle manière que, en utilisation, elle nettoie et dégraisse la surface du verre ou du matériau cristallin, ce qui la rend adaptée au collage d'argent ou d'un autre matériau diélectrique sur la surface cible en verre ou en matériau cristallin pour former le miroir.

31. Appareil selon la revendication 28, ladite surface de puce semi-conductrice comprenant une couche d'oxyde, qui est éliminée de façon sélective par la fraise mécanique induite de façon électromagnétique pour de ce fait former des fenêtres dans la couche d'oxyde pour des implantations de dopant dans la surface de la puce semi-conductrice.

32. Appareil selon la revendication 31, ladite couche d'oxyde comprenant du dioxyde de silicium.

33. Appareil selon la revendication 22, dans lequel la fraise mécanique induite de façon électromagnétique est adaptable, en utilisation, pour éliminer des parties de la couche de dioxyde de silicium sans nécessiter de réserve, de masques photographiques, de lumière ultraviolette, de solvants, de composés chimiques ou aqueux, et d'acides.

34. Appareil selon la revendication 28, ladite surface cible étant une surface de puce semi-conductrice ayant une couche de réserve et la fraise mécanique induite de façon électromagnétique étant adaptée pour, en utilisation, éliminer la couche de réserve de la surface de la puce semi-conductrice.

35. Appareil selon la revendication 34, dans lequel la fraise mécanique induite de façon électromagnétique est adaptée pour effectuer une légère ablation de la surface de puce semi-conductrice pour de ce fait éliminer la couche de réserve et conditionner la surface de puce semi-conductrice pour le collage d'un conducteur sur la surface de la puce semi-conductrice.

36. Appareil selon la revendication 11 ou la revendication 12, dans lequel ladite commande utilisateur (77) comprend une entrée de spécification pour spécifier au moins l'un parmi une résolution de coupe et un niveau de pénétration pour l'efficacité de coupe ; et
ledit atomiseur comprend des moyens pour sélectionner l'une d'une pluralité de buses de pulvérisation de fluide (71, 72) en réponse à une spécification de l'utilisateur de la résolution de coupe, et des moyens pour sélectionner une pression de fluide amont pour la buse de pulvérisation de fluide sélectionnée en réponse à une spécification de l'utilisateur du niveau de pénétration ;
ledit atomiseur étant configuré pour appliquer la pression de fluide amont à une buse de pulvérisation de fluide, pour de ce fait générer la combinaison de particules de fluide atomisées spécifiée par l'utilisateur

37. Appareil selon la revendication 36, dans lequel l'entrée de spécification comprend :
une première entrée utilisateur pour spécifier un niveau de résolution pour l'efficacité de coupe, le niveau de résolution comprenant soit une coupe à haute résolution soit une coupe à basse résolution ; et
une seconde entrée utilisateur pour spécifier une niveau de pénétration pour l'efficacité de coupe, le niveau de pénétration comprenant soit une coupe profonde, soit une coupe peu profonde.

38. Appareil selon la revendication 37, dans lequel l'atomiseur génère une combinaison de particules de fluide atomisées comprenant des particules de fluide relativement petites, en réponse à la première entrée utilisateur spécifiant une coupe à haute résolution,
dans lequel l'atomiseur génère une combinaison de particules de fluide atomisées comprenant des particules de fluide relativement grosses, en réponse à la première entrée utilisateur spécifiant une coupe à basse résolution,
dans lequel l'atomiseur génère une combinaison de particules de fluide atomisées comprenant une distribution à densité relativement faible de particules de fluide, en réponse à la seconde entrée utilisateur spécifiant une coupe profonde, et
dans lequel l'atomiseur génère une combinaison de particules de fluide atomisées comprenant une distribution à densité relativement élevée de particules de fluide, en réponse à la seconde entrée utilisateur spécifiant une coupe peu profonde.

39. Procédé non thérapeutique comprenant les étapes suivantes consistant à :
fournir du fluide à une première proximité de la surface cible ; et
fournir de l'énergie électromagnétique à une seconde proximité de la surface cible ;
la première proximité et la seconde proximité se coupant dans un volume adjacent à la surface cible ;
ladite étape consistant à fournir du fluide comprenant la génération d'une combinaison de particules de fluide atomisées et le placement de ladite combinaison de particules de fluide atomisées dans ledit volume adjacent à la surface cible ;
ladite étape consistant à fournir de l'énergie électromagnétique comprenant l'envoi d'une concentration crête d'énergie électromagnétique, qui a une longueur d'onde qui est sensiblement absorbée par ledit fluide, dans ledit volume de manière à être absorbée par au moins une partie desdites particules de fluide, pour amener la partie des particules de fluide atomisées à augmenter de volume tandis que des efforts mécaniques disruptifs sont appliqués sur ladite surface cible.

40. Procédé selon la revendication 39, dans lequel l'étape de placement des particules de fluide atomisées dans la zone d'interaction comprend une sous-étape de placement de particules d'eau atomisées dans la zone d'interaction.

41. Procédé selon la revendication 40, dans lequel l'étape de focalisation de l'énergie électromagnétique sur les particules de fluide atomisées dans la zone d'interaction comprend une sous-étape de focalisation d'énergie électromagnétique à partir d'un laser à grenat d'erbium, de chrome, d'yttrium, de scandium et de gallium (Er, Cr:YSGG) à l'état solide, qui génère de l'énergie électromagnétique ayant une longueur d'onde d'environ 2,78 microns, sur les particules d'eau atomisées dans la zone d'interaction.

42. Procédé selon la revendication 40, comprenant l'étape consistant à fournir des efforts mécaniques de coupe induits de façon électromagnétique sur une surface cible pour éliminer de ce fait des parties de la surface cible, et
comprenant en outre l'étape consistant à entrer via une commande utilisateur une combinaison de particules de fluide atomisées spécifiées par l'utilisateur, la combinaison de particules de fluide atomisées spécifiées par l'utilisateur correspondant à une taille moyenne, une répartition spatiale, et une vitesse des particules de fluide spécifiées par l'utilisateur, et dans lequel
dans ladite étape consistant à générer une combinaison de particules, ladite combinaison de particules de fluide atomisées spécifiées par l'utilisateur est générée en réponse à l'entrée issue de la commande utilisateur ; et
ladite partie desdites particules de fluide atomisées est une partie de la combinaison de particules de fluide atomisées spécifiées par l'utilisateur.

43. Procédé selon l'une quelconque des revendications 39 à 42, dans lequel la surface cible comprend au moins l'un des éléments parmi le cartilage, l'os ou la dent.

44. Procédé selon l'une quelconque des revendications 29 à 42, dans lequel la surface cible comprend l'un des éléments parmi le verre, un matériau cristallin, et une surface de puce semi-conductrice.

45. Procédé selon la revendication 44 comprenant une élimination mécanique de parties d'une surface cible, dans lequel la surface du verre ou du matériau cristallin est soumise à une légère ablation avant que de l'argent ou un autre matériau diélectrique ne soit collé sur la surface cible en verre ou en matériau cristallin pour former un miroir.

46. Procédé selon la revendication 44 comprenant une élimination mécanique de parties d'une surface cible, dans lequel la surface d'une puce semi-conductrice comprend une couche d'oxyde, qui est éliminée de façon sélective pour de ce fait former des fenêtres dans la couche d'oxyde pour des implantations de dopants dans la surface de puce semi-conductrice.

47. Procédé selon la revendication 44 comprenant une élimination mécanique de parties d'une surface cible, dans lequel une couche de réserve est éliminée de la surface de puce semi-conductrice.

48. Procédé selon la revendication 41 dans lequel ladite étape consistant à entrer comprend la spécification d'au moins l'un parmi une résolution de coupe et un niveau de pénétration pour l'efficacité de coupe ; et
ladite étape consistant à générer comprend la sélection de l'une d'une pluralité de buses de pulvérisation de fluide, en réponse à une spécification de la résolution de coupe ;
la sélection d'une pression de fluide amont pour la buse de pulvérisation de fluide sélectionnée, en réponse à une spécification du niveau de pénétration ; et
l'application de la pression de fluide amont à la buse de pulvérisation de fluide, pour de ce fait générer la combinaison de particules de fluide atomisées spécifiées par l'utilisateur.

49. Procédé selon la revendication 48, l'étape consistant à spécifier au moins l'un parmi une résolution de coupe et un niveau de pénétration pour l'efficacité de coupe comprenant les étapes suivantes :
spécification, via une entrée utilisateur, de soit une coupe à haute résolution, soit une coupe à basse résolution ; et
spécification, via une entrée utilisateur, de soit une coupe à pénétration profonde, soit une coupe pénétration peu profonde.

50. Procédé selon la revendication 49 comprenant l'étape consistant à commander une efficacité de coupe d'une fraise mécanique induite de façon électromagnétique, dans lequel l'étape consistant à appliquer la pression de fluide amont à la buse de pulvérisation de fluide comprend les sous-étapes suivantes :
génération d'une combinaison de particules de fluide atomisées comprenant les particules de fluide relativement petites, en réponse à une entrée utilisateur spécifiant une coupe à haute résolution ;
génération d'une combinaison de particules de fluide atomisées comprenant des particules de fluide relativement grosses, en réponse à une entrée utilisateur spécifiant une coupe à basse résolution ;
génération d'une combinaison de particules de fluide atomisées qui comprend une répartition des particules de fluide à relativement faible densité, en réponse à une entrée utilisateur spécifiant une coupe à pénétration profonde ; et
génération d'une combinaison de particules de fluide atomisées qui comprend une répartition des particules de fluide à relativement haute densité, en réponse à une entrée utilisateur spécifiant une coupe à pénétration peu profonde.

51. Procédé selon la revendication 50 comprenant l'étape consistant à commander l'efficacité de coupe d'une fraise mécanique induite de façon électromagnétique, dans lequel l'étape consistant à appliquer la pression de fluide amont à la buse de pulvérisation de fluide comprend en outre les sous-étapes suivantes :
génération de particules de fluide atomisées à énergie cinétique relativement élevée, en réponse à au moins l'une d'une spécification utilisateur pour une coupe à pénétration profonde et d'une spécification utilisateur pour une coupe à haute résolution ; et
génération de particules de fluide atomisées à énergie cinétique relativement faible, en réponse à au moins l'une d'une spécification utilisateur pour une coupe à pénétration peu profonde et d'une spécification utilisateur pour une coupe à basse résolution.

52. Fraise optique pour utilisation dentaire, comprenant un appareil selon l'une quelconque des revendications 1 à 14, et
un logement ayant une partie inférieure, une partie supérieure et une partie d'interfaçage ;
la source d'énergie électromagnétique comprenant :
un premier tube de fibre optique pour transporter l'énergie laser à travers le logement vers la partie supérieure du logement :
un premier embout monté autour du premier tube de fibre optique au niveau de la partie supérieure du logement ;
un second tube de fibre optique possédant une extrémité proximale et une extrémité distale ;
un second embout monté autour du second tube de fibre optique au niveau de l'extrémité proximale et en contact avec la partie d'interfaçage du logement ; et
une optique de focalisation positionnée entre le premier embout et le second embout, l'optique de focalisation focalisant l'énergie laser à mesure que l'énergie laser passe de la première fibre optique à la seconde fibre optique pour de ce fait réduire la dissipation de l'énergie laser entre la première fibre optique et la seconde fibre optique.

53. Fraise optique pour utilisation dentaire selon la revendication 52, comprenant en outre un bouchon possédant une partie d'entrée et une partie de sortie, le bouchon se montant sur la partie d'interfaçage du logement.

54. Fraise optique pour utilisation dentaire selon la revendication 52, dans lequel le deuxième tube de fibre optique comprend du saphir.

55. Pièce à main médicale, comprenant un appareil selon l'une quelconque des revendications 1 à 14, et
un logement comprenant une extrémité de logement proximale et une extrémité de fourniture de rayonnement ;
ladite source d'énergie électromagnétique comprenant un premier guide de fibre possédant une extrémité de sortie et étant disposée à l'intérieur du logement pour conduire le rayonnement laser d'une source laser extérieure vers l'extrémité de fourniture de rayonnement du logement ;
une première virole comprenant un matériau céramique ou cristallin et étant adaptée pour immobiliser le premier guide de fibre sur le logement de sorte que l'extrémité de sortie du premier guide de fibre fasse face à l'extrémité de fourniture de rayonnement du logement ;
un second guide de fibre possédant une extrémité réceptrice adaptée pour recevoir le rayonnement laser issu de l'extrémité de sortie du premier guide de fibre ; et
une seconde virole comprenant un matériau céramique ou cristallin et étant adaptée pour immobiliser le second guide de fibre sur le logement de sorte que l'extrémité réceptrice du second guide de fibre fasse face à l'extrémité de sortie du premier guide de fibre.

56. Pièce à main médicale selon la revendication 55, la première virole immobilisant de façon démontable le premier guide de fibre sur le logement de sorte que l'extrémité de sortie du premier guide de fibre fasse face à l'extrémité de fourniture de rayonnement du logement.

57. Pièce à main médicale selon la revendication 56, la première virole et la seconde virole formant une voie d'écoulement de gaz selon une direction allant du premier guide de fibre vers l'extrémité réceptrice du second guide de fibre.

58. Pièce à main médicale selon la revendication 57, la voie d'écoulement de gaz s'étendant à travers l'extrémité réceptrice du second guide de fibre et à travers la seconde virole.

59. Appareil selon l'une quelconque des revendications 1 à 14, comprenant en outre un appareil conducteur d'énergie électromagnétique, comprenant :
un logement ;
un guide d'énergie électromagnétique interne disposé à l'intérieur du logement, le guide d'énergie électromagnétique interne possédant une extrémité proximale, une extrémité distale, et un axe s'étendant entre l'extrémité proximale et l'extrémité distale ; et
un guide d'énergie électromagnétique externe entourant le guide d'énergie électromagnétique interne et étant en contact avec celui-ci, le guide d'énergie électromagnétique externe possédant un axe qui est sensiblement colinéaire avec l'axe du guide d'énergie électromagnétique interne.

60. Appareil selon la revendication 59, le guide d'énergie électromagnétique externe comprenant une virole.

61. Appareil selon la revendication 60, le guide d'énergie électromagnétique interne et le guide d'énergie électromagnétique externe comprenant tous les deux un matériau cristallin.

62. Appareil selon la revendication 59, comprenant en outre au moins un autre guide d'énergie électromagnétique entourant le guide d'énergie électromagnétique externe et étant en contact avec celui-ci, l'autre guide d'énergie électromagnétique possédant un axe qui est sensiblement colinéaire avec l'axe du guide d'énergie électromagnétique externe.

63. Appareil selon la revendication 59, comprenant en outre un guide d'énergie électromagnétique source pour entrer l'énergie électromagnétique dans le guide d'énergie électromagnétique interne et dans le guide d'énergie électromagnétique externe.

64. Appareil selon la revendication 60, un diamètre du guide d'énergie électromagnétique source étant plus grand qu'un diamètre du guide d'énergie électromagnétique interne.

65. Appareil selon la revendication 59, le guide d'énergie électromagnétique interne et le guide d'énergie électromagnétique externe étant concentriques.

66. Appareil selon la revendication 59, le guide d'énergie électromagnétique interne et le guide d'énergie électromagnétique externe partageant le même axe.

67. Appareil selon la revendication 66, au moins l'un parmi le guide d'énergie électromagnétique interne et le guide d'énergie électromagnétique externe comprenant du saphir.

68. Appareil selon l'une quelconque des revendications 1 à 14, comprenant en outre un appareil conducteur d'énergie optique, comprenant une fibre cristalline courbe adaptée pour y conduire de l'énergie optique, la fibre cristalline courbe possédant une extrémité proximale, une extrémité distale, et un axe s'étendant entre l'extrémité proximale et l'extrémité distale, une première partie de l'axe près de l'extrémité proximale de la fibre cristalline courbe n'étant pas parallèle à une seconde partie de l'axe près de l'extrémité distale de la fibre cristalline courbe.

69. Appareil selon la revendication 68, la fibre cristalline courbe s'étendant entre une source laser et une extrémité distale d'un dispositif de fourniture laser.

70. Appareil selon la revendication 68, un angle formé entre la première partie de l'axe et la seconde partie de l'axe étant d'environ 90°.

71. Appareil selon la revendication 68, comprenant en outre un logement, la fibre cristalline courbe étant disposée dans le logement.

72. Appareil selon la revendication 71, le logement étant connecté à une pièce à main médicale, et la fibre cristalline courbe étant refroidie par une voie d'écoulement de gaz dans la pièce à main médicale.

73. Appareil selon la revendication 68, l'énergie optique comprenant une source de lumière cohérente.

74. Appareil selon la revendication 73, la lumière cohérente ayant une longueur d'onde d'un ordre d'environ 2,5 à 3,0 microns.

75. Appareil selon la revendication 74, la lumière cohérente ayant une longueur d'onde dans une plage allant d'environ 2,78 à environ 2,94 microns.

76. Appareil selon la revendication 75, la fibre cristalline courbe comprenant du saphir.

77. Appareil selon l'une quelconque des revendications 1 à 14, comprenant un appareil conducteur d'énergie optique, comprenant :
un logement ;
une fibre cristalline disposée à l'intérieur du logement, la fibre cristalline étant adaptée pour y conduire de l'énergie optique, la fibre cristalline possédant une extrémité proximale, une extrémité distale, et un axe s'étendant de l'extrémité proximale à l'extrémité distale ; et
une voie d'écoulement de gaz à l'intérieur du logement, la voie d'écoulement de gaz enveloppant la fibre cristalline et s'étendant de l'extrémité proximale de la fibre cristalline à l'extrémité distale de la fibre cristalline.

78. Appareil selon la revendication 77, la fibre cristalline étant courbe.

79. Pièce à main médicale, comprenant :
un logement comprenant une extrémité proximale de logement et une extrémité distale de logement ;
un appareil selon l'une quelconque des revendications 1 à 27, disposé à l'extrémité distale de logement et adapté pour couper des tissus ; et
une ligne de médication possédant une extrémité proximale de ligne et une extrémité distale de ligne, la ligne de médication étant adaptée pour recevoir la médication dans l'extrémité proximale de la ligne et faire sortir la médication par l'extrémité distale de la ligne, qui est disposée près de l'extrémité distale de logement.

80. Pièce à main médicale selon la revendication 79, la fraise à tissus comprenant soit un laser soit une fraise.

81. Pièce à main médicale selon la revendication 80, la fraise à tissus étant en outre adaptée à l'ablation de tissus.

82. Système d'alimentation pour pièce à main médicale, comprenant l'appareil selon l'une quelconque des revendications 1 à 14, et
un logement comprenant une extrémité proximale de logement et une extrémité de fourniture de rayonnement ;
un guide de fibre possédant une extrémité de sortie et étant disposé à l'intérieur du logement pour conduire le rayonnement laser d'une source laser externe vers l'extrémité de fourniture de rayonnement du logement ;
une virole adaptée pour immobiliser le guide de fibre sur le logement de sorte que l'extrémité de sortie du guide de fibre fasse face à l'extrémité de fourniture de rayonnement du logement ;
un tube protecteur interne disposé sur le guide de fibre ; et
un tube protecteur externe disposé sur le tube protecteur interne.

83. Système d'alimentation pour pièce à main médicale selon la revendication 82, le guide de fibre comprenant une pluralité de gaines tubulaires entourant le guide de fibre et fixées à celui-ci.

84. Système d'alimentation pour pièce à main médicale selon la revendication 82, la virole comprenant une extrémité proximale et une extrémité distale, le tube protecteur interne comprenant une extrémité proximale et une extrémité distale, le tube protecteur externe comprenant une extrémité proximale et une extrémité distale, et au moins l'une parmi l'extrémité distale du tube protecteur interne et l'extrémité distale du tube protecteur externe étant en contact avec l'extrémité proximale de la virole.

85. Système d'alimentation pour pièce à main médicale selon la revendication 84, la virole comprenant une ouverture pour recevoir le guide de fibre, un diamètre de l'ouverture étant à peu près égal à un diamètre extérieur du guide de fibre.

86. Système d'alimentation pour pièce à main médicale selon la revendication 84, l'extrémité proximale du tube protecteur interne et l'extrémité proximale du tube protecteur externe étant disposées près d'un connecteur SMA, qui entoure l'extrémité proximale du guide de fibre.

87. Système d'alimentation pour pièce à main médicale selon la revendication 86, le connecteur SMA possédant une extrémité proximale et une extrémité distale, et le guide de fibre couvrant une distance entre l'extrémité proximale de la virole et l'extrémité distale du connecteur SMA.
une longueur d'au moins l'un parmi le tube protecteur interne et le tube protecteur externe étant inférieure à la distance couverte par le guide de fibre entre l'extrémité proximale de la virole et l'extrémité distale du connecteur SMA.

88. Système d'alimentation pour pièce à main médicale selon la revendication 87, une longueur d'au moins l'un parmi le tube protecteur interne et le tube protecteur externe étant au moins 6,35 mm (un quart de pouce) plus courte que la distance couverte par le guide de fibre entre l'extrémité proximale de la virole et l'extrémité distale du connecteur SMA.

89. Système d'alimentation pour pièce à main médicale selon la revendication 88, la longueur la plus courte de l'au moins un parmi le tube protecteur interne et le tube protecteur externe facilitant le coulissement de l'au moins un parmi le tube protecteur interne et le tube protecteur externe sur le guide de fibre.

90. Système d'alimentation pour pièce à main médicale selon la revendication 86, le connecteur SMA étant adapté pour fixer le guide de fibre sur une source d'énergie électromagnétique ;
le tube protecteur interne et le tube protecteur externe comprenant l'un parmi un matériau plastique souple et un matériau métallique souple ; et
le système d'alimentation de la pièce à main médicale comprenant en outre un tube métallique disposé autour du tube protecteur externe près de l'extrémité proximale du tube protecteur externe.

91. Système d'alimentation pour pièce à main médicale selon la revendication 90, la source d'énergie électromagnétique comprenant un laser.

92. Système d'alimentation pour pièce à main médicale selon la revendication 90, le système d'alimentation de la pièce à main médicale comprenant en outre un tube en matière plastique entourant et le tube métallique et étant en contact avec celui-ci.

93. Système d'alimentation pour pièce à main médicale selon la revendication 92, le tube protecteur interne et le tube protecteur externe réduisant tous les deux le risque d'endommagement du guide de fibre.

94. Système d'alimentation pour pièce à main médicale selon la revendication 93, le tube protecteur interne et le tube protecteur externe réduisant tous les deux la probabilité que la flexion du guide de fibre entraîne la rupture du guide de fibre.

95. Système d'alimentation pour pièce à main médicale selon la revendication 92, le tube protecteur interne et le tube protecteur externe réduisant tous les deux la probabilité que le guide de fibre se rompe près de la virole.

96. Système d'alimentation pour pièce à main médicale selon la revendication 95, le tube protecteur interne et le tube protecteur externe comprenant une matière plastique souple.

97. Système d'alimentation pour pièce à main médicale selon la revendication 82, le tube protecteur interne étant disposé de façon coulissante sur le guide de fibre.

98. Système d'alimentation pour pièce à main médicale selon la revendication 82, le tube protecteur externe étant disposé de façon coulissante sur le tube protecteur interne.

99. Système d'alimentation pour pièce à main médicale selon la revendication 98, comprenant en outre au moins un autre tube protecteur disposé de façon coulissante sur le tube protecteur externe.

100. Système d'alimentation pour pièce à main médicale selon la revendication 99, le tube protecteur interne étant disposé de façon coulissante sur le guide de fibre.

101. Système d'alimentation pour pièce à main médicale selon la revendication 82 comprenant en outre:
un second guide de fibre possédant une extrémité réceptrice adaptée pour recevoir le rayonnement laser issu de l'extrémité de sortie du guide de fibre ;
une deuxième virole adaptée pour immobiliser le second guide de fibre sur le logement de sorte que l'extrémité réceptrice du second guide de fibre fasse face à l'extrémité de sortie du guide de fibre ; et
une entretoise disposée entre la virole et la deuxième virole.

102. Système d'alimentation pour pièce à main médicale selon la revendication 101, au moins l'une parmi la virole, la deuxième virole et l'entretoise comprenant au moins l'un parmi un matériau céramique et un matériau cristallin.

103. Système d'alimentation pour pièce à main médicale selon la revendication 102, le matériau cristallin comprenant du saphir.

104. Pièce à main médicale comprenant l'appareil selon les revendications 1, 11 ou 12,
un logement fourreau adapté pour être tenu à la main par un utilisateur et comprenant une extrémité proximale de logement fourreau, une partie intermédiaire de logement fourreau, une extrémité de fourniture de rayonnement, et une ouverture allongée s'étendant à l'intérieur du logement fourreau de l'extrémité proximale du logement fourreau à la partie intermédiaire du logement fourreau, le logement fourreau comprenant un guide de fibre de fourniture s'étendant entre la partie intermédiaire du logement fourreau et l'extrémité de fourniture de rayonnement, le guide de fibre de fourniture possédant une extrémité proximale et une extrémité distale ; et
un arbre équipé adapté pour être disposé de façon amovible à l'intérieur de l'ouverture allongée, l'arbre équipé comprenant un guide de fibre source adapté pour fournir de l'énergie électromagnétique à la partie intermédiaire du logement fourreau, le guide de fibre source possédant une extrémité proximale et une extrémité distale.

105. Pièce à main médicale selon la revendication 104, comprenant en outre un collier pour montage autour de l'extrémité proximale du logement fourreau lorsque l'arbre équipé est disposé à l'intérieur du logement fourreau, le collier maintenant l'arbre équipé à l'intérieur du logement fourreau.

106. Pièce à main médicale selon la revendication 105, l'extrémité proximale du logement fourreau comprenant des filets pour recevoir des filets du collier.

107. Pièce à main médicale selon la revendication 104, l'extrémité distale du guide de fibre source étant entourée par une première virole, et
l'extrémité proximale du guide de fibre de fourniture étant entourée par une deuxième virole.

108. Pièce à main médicale selon la revendication 107, comprenant en outre une troisième virole entourant une partie du guide de fibre de fourniture près de l'extrémité de fourniture de rayonnement du logement fourreau.

109. Pièce à main médicale selon la revendication 107, l'arbre équipé comprenant une ligne d'alimentation en air disposée autour du guide de fibre source.

110. Pièce à main médicale selon la revendication 107, la fibre comprenant une pluralité de tubes protecteurs disposés autour du guide de fibre source et une ligne d'alimentation en air disposée autour de la pluralité de tubes protecteurs.

111. Pièce à main médicale selon la revendication 109, le logement fourreau comprenant une ligne d'air s'étendant de l'extrémité proximale du logement fourreau à l'extrémité de fourniture de rayonnement ; et
une ligne d'eau s'étendant de l'extrémité proximale du logement fourreau à l'extrémité de fourniture de rayonnement.

112. Pièce à main médicale selon la revendication 110, le logement fourreau comprenant en outre une ligne de médication s'étendant généralement de l'extrémité proximale du logement fourreau à l'extrémité de fourniture de rayonnement.

113. Appareil pour mettre en oeuvre une procédure médicale comprenant :
un appareil selon l'une quelconque des revendications 1 à 27 pour effectuer une fonction de traitement médical sur un site opératoire situé à l'intérieur d'un corps humain ou connecté à celui-ci ; et
un dispositif d'acheminement de fluide pour acheminer un fluide aromatisé dans la direction du site opératoire.

114. Appareil pour mettre en oeuvre une procédure médicale selon la revendication 113, dans lequel un fluide aromatisé est sélectionné en utilisation pour être aromatisé pour flatter les papilles gustatives du patient subissant l'intervention chirurgicale.

115. Appareil pour mettre en oeuvre une procédure médicale selon la revendication 114, dans lequel le fluide aromatisé est sélectionné en utilisation pour être un fluide qui comprend un arôme de fruit ou un arôme de menthe.

116. Appareil pour mettre en oeuvre une procédure médicale selon la revendication 113, dans lequel l'instrument médical comprend soit un électrocautériseur, soit une source d'énergie électromagnétique, soit un laser, soit une fraise mécanique, soit une scie mécanique, soit un canal finder, soit une seringue, soit un évacuateur.

117. Appareil pour mettre en oeuvre une procédure médicale selon la revendication 116, dans lequel la source d'énergie électromagnétique focalise l'énergie électromagnétique sur le site opératoire ; et
dans lequel le dispositif d'acheminement de fluide achemine le fluide aromatisé sur le site opératoire, pour de ce fait refroidir le site opératoire à mesure que le site opératoire est coupé.

118. Appareil pour mettre en oeuvre une procédure médicale selon la revendication 116, dans lequel le dispositif d'acheminement de fluide est utilisable entre un premier mode selon lequel le fluide acheminé est aromatisé, et un second mode selon lequel le fluide acheminé n'est pas aromatisé.

119. Appareil pour mettre en oeuvre une procédure médicale selon la revendication 115, dans lequel le dispositif d'acheminement de fluide comprend un atomiseur pour atomiser le fluide aromatisé en particules de fluide aromatisé atomisées avant d'acheminer le fluide aromatisé dans la direction du site opératoire.

120. Appareil pour mettre en oeuvre une procédure médicale selon la revendication 119, dans lequel l'atomiseur est configuré pour acheminer les particules de fluide aromatisé atomisées dans un volume d'air situé au-dessus du site opératoire ; et
dans lequel la source d'énergie électromagnétique est configurée pour focaliser l'énergie électromagnétique dans le volume d'air, l'énergie électromagnétique ayant une longueur d'onde qui est sensiblement absorbée par les particules de fluide aromatisé atomisées dans le volume d'air, l'absorption de l'énergie électromagnétique par les particules de fluide aromatisé atomisées faisant exploser les particules de fluide aromatisé atomisées et appliquer des efforts mécaniques sur le site opératoire.

121. Appareil pour mettre en oeuvre une procédure médicale selon la revendication 120 dans lequel le dispositif d'acheminement de fluide est utilisable entre un premier mode selon lequel le fluide acheminé est aromatisé, et un second mode selon lequel le fluide acheminé n'est pas aromatisé.

122. Appareil pour mettre en oeuvre une procédure médicale comprenant :
un appareil selon l'une quelconque des revendications 1 à 27, pour effectuer une fonction de traitement médical sur un site opératoire situé à l'intérieur d'un corps humain ou connecté à celui-ci ; et
un dispositif d'acheminement pour acheminer un milieu parfumé dans la direction du site opératoire.

123. Appareil pour mettre en oeuvre une procédure médicale selon la revendication 122, dans lequel le milieu parfumé est sélectionné en utilisation pour être un milieu qui est parfumé pour flatter le sens de l'odorat du patient subissant l'intervention chirurgicale.

124. Appareil pour mettre en oeuvre une procédure médicale selon la revendication 123, dans lequel le milieu parfumé est sélectionné en utilisation pour comprendre soit un parfum de fruit, soit un parfum de menthe, soit un désodorisant.

125. Appareil pour mettre en oeuvre une procédure médicale selon la revendication 122, dans lequel l'instrument médical comprend soit un électrocautériseur, soit une source d'énergie électromagnétique, soit un laser, soit une fraise mécanique, soit une scie mécanique, soit un canal finder, soit une seringue, soit un évacuateur.

126. Appareil pour mettre en oeuvre une procédure médicale selon la revendication 123, ledit milieu parfumé comprenant de l'air parfumé.

127. Appareil pour mettre en oeuvre une procédure médicale selon la revendication 122, dans lequel la source d'énergie électromagnétique est configureé, en utilisation, pour focaliser l'énergie électromagnétique sur le site opératoire,
ledit milieu parfumé comprenant de l'eau parfumée, et
ledit dispositif d'acheminement étant configuré pour acheminer, en utilisation, l'eau parfumée sur le site opératoire, pour de ce fait refroidir le site opératoire lorsque le site opératoire est coupé par la source d'énergie électromagnétique.

128. Appareil pour mettre en oeuvre une procédure médicale selon la revendication 123, dans lequel la source d'énergie électromagnétique est configurée, en utilisation, pour focaliser l'énergie électromagnétique sur le site opératoire, et
ledit milieu parfumé comprenant un brouillard parfumé, et
ledit dispositif d'acheminement étant configuré pour acheminer, en utilisation, le brouillard parfumé sur le site opératoire, pour de ce fait refroidir le site opératoire à mesure le site opératoire est coupé par la source d'énergie électromagnétique.

129. Appareil pour mettre en oeuvre une procédure médicale selon la revendication 122, dans lequel le dispositif d'acheminement est utilisable entre un premier mode selon lequel le milieu acheminé est parfumé, et un second mode selon lequel le milieu acheminé n'est pas parfumé.

130. Appareil pour mettre en oeuvre une procédure médicale selon la revendication 123, dans lequel le dispositif d'acheminement comprend un atomiseur pour atomiser le milieu parfumé avant d'acheminer le milieu parfumé dans la direction du site opératoire.

131. Appareil pour mettre en oeuvre une procédure médicale selon la revendication 127, dans lequel l'atomiseur est configuré pour acheminer, en utilisation, le milieu parfumé atomisé dans un volume d'air situé au-dessus du site opératoire ; et
dans lequel la source d'énergie électromagnétique est configurée pour focaliser, en utilisation, l'énergie électromagnétique dans le volume d'air, l'énergie électromagnétique ayant une longueur d'onde qui est sensiblement absorbée par des parties du milieu parfumé atomisé dans le volume d'air, l'absorption de l'énergie électromagnétique par les parties du milieu parfumé atomisé faisant exploser les parties du milieu parfumé atomisé et appliquer des efforts mécaniques sur le site opératoire.

132. Appareil pour mettre en oeuvre une procédure médicale selon la revendication 130, dans lequel le dispositif d'acheminement est utilisable entre un premier mode selon lequel le milieu acheminé est parfumé, et un second mode selon lequel le milieu acheminé n'est pas parfumé.

133. Appareil pour mettre en oeuvre une procédure médicale, comprenant :
une fraise selon l'une quelconque des revendications 1 à 27, pour effectuer une fonction de traitement médical sur un site opératoire situé à l'intérieur d'un corps humain, ou connecté à celui-ci ; et
un dispositif d'acheminement de fluide pour acheminer, en utilisation, une solution ionisée dans la direction du site opératoire.

134. Appareil pour mettre en oeuvre une procédure médicale selon la revendication 133, ladite solution ionisée comprenant une solution saline biocompatible.

135. Appareil pour mettre en oeuvre une procédure médicale selon la revendication 133, dans lequel l'instrument médical comprend soit un électrocautériseur, soit une source d'énergie électromagnétique, soit un laser, soit une fraise mécanique, soit une scie mécanique, soit un canal finder, soit une seringue, soit un évacuateur.

136. Appareil pour mettre en oeuvre une procédure médicale selon la revendication 135, dans lequel la source d'énergie électromagnétique focalise l'énergie électromagnétique sur le site opératoire ; et
dans lequel le dispositif d'acheminement de fluide achemine la solution ionisée sur le site opératoire, pour de ce fait refroidir le site opératoire à mesure que le site opératoire est coupé.

137. Appareil pour mettre en oeuvre une procédure médicale selon la revendication 135, dans lequel le dispositif d'acheminement de fluide comprend un atomiseur pour atomiser la solution ionisée en particules atomisées avant d'acheminer les particules atomisées dans la direction du site opératoire.

138. Appareil pour mettre en oeuvre une procédure médicale selon la revendication 137, dans lequel l'atomiseur achemine les particules atomisées dans un volume d'air situé au-dessus du site opératoire ; et
dans lequel la source d'énergie électromagnétique focalise l'énergie électromagnétique dans le volume d'air, l'énergie électromagnétique ayant une longueur d'onde qui est sensiblement absorbée par les particules atomisées dans le volume d'air, l'absorption de l'énergie électromagnétique par les particules atomisées faisant exploser les particules atomisées et appliquer des efforts mécaniques sur le site opératoire.

139. Appareil pour mettre en oeuvre une procédure médicale selon la revendication 137, dans lequel le dispositif d'acheminement est utilisable entre un premier mode selon lequel la solution qui en est acheminée est ionisée, et un second mode selon lequel la solution qui en est acheminée n'est pas ionisée.

140. Appareil pour mettre en oeuvre une procédure médicale selon la revendication 139, dans lequel le dispositif d'acheminement de fluide est utilisable dans une pluralité de modes entre le premier mode et le second mode pour de ce fait commander en continu une puissance de coupe de la source d'énergie électromagnétique.

141. Appareil pour mettre en oeuvre une procédure médicale selon la revendication 140, dans lequel la puissance de coupe de la source d'énergie électromagnétique diminue à mesure que le fluide issu du dispositif d'acheminement de fluide devient plus ionisé.

142. Appareil pour mettre en oeuvre une procédure médicale selon la revendication 141, dans lequel une puissance de la source d'énergie électromagnétique est ajustable pour commander la puissance de coupe de la source d'énergie électromagnétique.

143. Appareil pour mettre en oeuvre une procédure médicale selon la revendication 142, dans lequel la puissance de la source d'énergie électromagnétique peut être augmentée lorsque le fluide issu du dispositif d'acheminement de fluide devient plus ionisé, pour de ce fait maintenir une puissance de coupe constante de la source d'énergie électromagnétique ; et
dans lequel la puissance de la source d'énergie électromagnétique peut être diminuée lorsque le fluide issu du dispositif d'acheminement de fluide devient moins ionisé, pour de ce fait maintenir une puissance de coupe constante de la source d'énergie électromagnétique.

144. Appareil selon l'une quelconque des revendications 1 à 27, dans lequel
ledit atomiseur est configuré pour placer, en utilisation, des particules de fluide pigmenté atomisées dans ledit volume prédéfini.

145. Appareil selon l'une quelconque des revendications 1 à 27, comprenant en outre :
une source de fluide pour fournir, en utilisation, du fluide pigmenté à une proximité approchée de la surface cible, le fluide pigmenté ayant une couleur qui est sensiblement non absorbée par l'énergie électromagnétique.

146. Appareil selon l'une quelconque des revendications 1 à 27, comprenant en outre une source de fluide pour fournir du fluide à une proximité approchée de la surface cible, le fluide ayant une couleur qui est sensiblement non absorbée par l'énergie électromagnétique.

147. Appareil selon l'une quelconque des revendications 1 à 27, comprenant en outre des moyens de fourniture connectés à l'atomiseur, pour fournir de façon sélective une pigmentation aux particules de fluide atomisées.

148. Appareil selon la revendication 147, dans lequel la pigmentation est fournie de façon sélective aux particules de fluide atomisées en fonction d'une entrée utilisateur.

149. Appareil selon la revendication 147, dans lequel l'énergie électromagnétique a une longueur d'onde qui est sensiblement absorbée par les particules de fluide atomisées lorsque la pigmentation est fournie aux particules de fluide atomisées.

150. Appareil selon la revendication 147, dans lequel l'énergie électromagnétique a une longueur d'onde qui est sensiblement absorbée par les particules de fluide atomisées lorsque la pigmentation n'est pas fournie aux particules de fluide atomisées.

151. Appareil selon la revendication 147, dans lequel l'atomiseur comprend une ligne de source d'eau et une ligne de source d'air ; et
dans lequel la pigmentation est fournie de façon sélective aux particules de fluide atomisées via l'une parmi la ligne de source d'air et la ligne de source d'eau.

152. Appareil pour mettre en oeuvre une procédure médicale, comprenant : un appareil selon l'une quelconque des revendications 1 à 27 pour effectuer une fonction de traitement médical sur un site opératoire situé à l'intérieur d'un corps humain ou connecté à celui-ci ; et
un dispositif d'acheminement de fluide pour acheminer un fluide, qui a un paramètre de fluide mesurable différent d'un paramètre de fluide mesurable correspondant de l'eau, dans la direction du site opératoire.

153. Appareil selon la revendication 152, dans lequel un paramètre de fluide mesurable comprend l'un parmi une densité, une gravité spécifique, un niveau pH, une viscosité et une température.

154. Appareil pour mettre en oeuvre une procédure médicale comprenant :
une fraise selon l'une quelconque des revendications 1 à 27, pour effectuer une fonction de traitement médical sur un site opératoire situé à l'intérieur d'un corps humain ou connecté à celui-ci ; et un dispositif d'acheminement pour acheminer un milieu de médication dans la direction du site opératoire.

155. Appareil pour mettre en oeuvre une procédure médicale selon la revendication 154, dans lequel, en utilisation, le milieu de médication peut comprendre l'un parmi un antibiotique, de l'iode, un stéroïde, un anesthésique, un anti-inflammatoire, un désinfectant, de l'adrénaline, de l'epinéphrine, un antiseptique, et un stringent.

156. Appareil pour mettre en oeuvre une procédure médicale selon la revendication 154, dans lequel, en utilisation, le milieu de médication peut comprendre l'un parmi des vitamines, des herbes, et des minéraux.

157. Appareil pour mettre en oeuvre une procédure médicale selon la revendication 154, dans lequel l'instrument médical est soit un électrocautériseur, soit une source d'énergie électromagnétique, soit un laser, soit une fraise mécanique, soit une scie mécanique, soit un canal finder, soit une seringue, soit un évacuateur.

158. Appareil pour mettre en oeuvre une procédure médicale selon la revendication 157 dans lequel la source d'énergie électromagnétique est configurée pour focaliser, en utilisation, l'énergie électromagnétique sur le site opératoire ; et
dans lequel le dispositif d'acheminement, en utilisation, achemine le milieu de médication sur le site opératoire pour de ce fait refroidir et médicamenter le site opératoire à mesure que le site opératoire est coupé.

159. Appareil pour mettre en oeuvre une procédure médicale selon la revendication 158, dans lequel le dispositif d'acheminement est utilisable entre un premier mode selon lequel le milieu acheminé est médicamenté, et un second mode selon lequel le milieu acheminé n'est pas médicamenté.

160. Appareil pour mettre en oeuvre une procédure médicale selon la revendication 159, dans lequel le dispositif d'acheminement comprend un atomiseur pour atomiser le milieu de médication en particules atomisées avant d'acheminer le milieu de médication dans la direction du site opératoire.

161. Appareil pour mettre en oeuvre une procédure médicale selon la revendication 160, dans lequel l'atomiseur est configuré, en utilisation, pour acheminer, en utilisation, les particules atomisées dans un volume d'air situé au-dessus du site opératoire ; et
dans lequel la source d'énergie électromagnétique est configurée, en utilisation, pour focaliser l'énergie électromagnétique dans le volume d'air, l'énergie électromagnétique ayant une longueur d'onde qui est sensiblement absorbée par les particules atomisées dans le volume d'air, l'absorption de l'énergie électromagnétique par les particules atomisées faisant exploser les particules atomisées et appliquer des efforts mécaniques sur le site opératoire.

162. Appareil pour mettre en oeuvre une procédure médicale selon la revendication 160, dans lequel le dispositif d'acheminement de fluide peut être utilisable entre un premier mode selon lequel le fluide qui en est acheminé est médicamenté, et un second mode selon lequel le fluide qui en est acheminé n'est pas médicamenté.

163. Appareil pour tuer les bactéries dans ou sur la bouche d'un patient et un instrument dentaire dans une proximité de la bouche du patient, comprenant :
un outil chirurgical comprenant un appareil selon la revendication 1 pour couper ou percer un matériau dans la bouche d'un patient ; et
un dispositif d'acheminement de fluide pour acheminer du désinfectant dans la bouche du patient, une partie du désinfectant acheminé étant mis en suspension dans l'air et se déposant sur l'instrument dentaire pour de ce fait désinfecter l'instrument dentaire.

164. Appareil de perçage et de conditionnement comprenant :
un appareil selon l'une quelconque des revendications 1 à 27 pour effectuer une opération de perçage sur une dent dans la bouche d'un patient ; et
un dispositif d'acheminement de fluide pour acheminer du fluide conditionné dans la direction de la dent objet de l'intervention, le fluide conditionné ayant une propriété mesurable qui est différente d'une propriété mesurable correspondante de l'eau.

165. Appareil de perçage et de conditionnement selon la revendication 164, ladite propriété mesurable comprenant au moins l'un parmi un arôme détectable par les papilles gustatives du patient, un parfum détectable par le sens de l'odorat du patient, une salinité, un pigment, une médication et un désinfectant.

166. Appareil de perçage et de conditionnement selon la revendication 165, ladite médication comprenant au moins l'un parmi un antibiotique, de l'iode, un stéroïde, un anesthésique, un anti-inflammatoire, un désinfectant, de l'adrénaline, de l'épinéphrine, un antiseptique, et un stringent.

167. Appareil de perçage et de conditionnement selon la revendication 165, dans lequel la médication comprend l'un parmi des vitamines, des herbes, et des minéraux.

168. Appareil pour mettre en oeuvre une procédure médicale comprenant :
un appareil selon l'une quelconque des revendications 1 à 27 pour effectuer une fonction de traitement médical sur un site opératoire situé à l'intérieur d'un corps humain ou connecté à celui-ci ; et
un dispositif d'acheminement de fluide pour acheminer de façon sélective un fluide pigmenté dans la direction du tissu.

169. Appareil pour mettre en oeuvre une procédure médicale selon la revendication 168, dans lequel l'instrument médical comprend soit un électrocautériseur, soit une source d'énergie électromagnétique, soit un laser, soit une fraise mécanique, soit une scie mécanique, soit un canal finder, soit une seringue, soit un évacuateur.

170. Appareil pour mettre en oeuvre une procédure médicale selon la revendication 169, dans lequel le dispositif d'acheminement de fluide est utilisable entre un premier mode selon lequel le fluide acheminé est pigmenté, et un second mode selon lequel le fluide acheminé n'est pas pigmenté.

171. Appareil pour mettre en oeuvre une procédure médicale selon la revendication 170, dans lequel une longueur d'onde de la source d'énergie électromagnétique est sensiblement absorbée par le fluide pigmenté.
